# EUROPEAN PATENT APPLICATION

(11) **EP 1 806 149 A1**
(43) Date of publication of application: **11.07.2007**
(21) Application number: 05799275.2
(22) Date of filing: 20.10.2005
(51) Int. Cl.: A61K 47/02, A61K 9/14, A61K 9/20, A61K 31/225, A61K 45/00, A61K 47/04, A61K 47/30, A61K 47/32, A61K 47/36, A61K 47/38, A61K 47/42, A61P 3/04, A61P 3/06, A61P 3/10, A61P 9/10, A61P 9/12, A61P 43/00, C07C 237/30

(54) **SOLID MEDICINAL PREPARATION IMPROVED IN SOLUBILITY AND STABILITY AND PROCESS FOR PRODUCING THE SAME**

(30) Priority: 25.10.2004 JP 2004310254; 30.11.2004 US 631894 P
(71) Applicant: Japan Tobacco, Inc., Tokyo 105-8422 (JP)
(72) Inventor: YAMANE, Shogo, Japan Tobacco Inc., Takatsuki-shi, Osaka 569-1125 (JP); YAMANE, Hirofumi, Japan Tobacco Inc., Takatsuki-shi, Osaka 569-1125 (JP); SUNAMI, Masaki, Japan Tobacco Inc., Takatsuki-shi, Osaka 569-1125 (JP)
(74) Representative: Vossius & Partner
(86) International application number: PCT/JP2005/019744
(87) International publication number: WO 2006/046623

(57) **Abstract**

The present invention relates to a solid formulation with improved solubility and maintained stability including a chemical substance poorly soluble in water as a pharmaceutically active ingredient formulated in an improved manner.

More specifically, the present invention relates to a solid formulation having improved water-solubility which comprises a chemical substance poorly soluble in water as a pharmaceutically active ingredient, a water-soluble polymer substance and an inorganic porous substance, **characterized in that**
(1) the chemical substance poorly soluble in water forms a solid dispersion together with the water-soluble polymer substance,
(2) the water-soluble polymer substance forming the solid dispersion is in the form of fine pieces, and
(3) the water-soluble polymer substance is in the form of fine pieces is maintained to be adsorbed on and/or adhered to the surface of the inorganic porous substance.

The solid formulation can be produced by stirring and mixing a solution obtained by dissolving a chemical substance poorly soluble in water as a pharmaceutically active ingredient and a water-soluble polymer substance in an organic solvent with an inorganic porous substance and then granulating and drying the mixture.

## Description

### Technical Field

The present invention relates to a solid formulation with improved solubility and stability and a method for producing said formulation.

More specifically the present invention relates to a solid formulation having improved water-solubility which comprises a chemical substance poorly soluble in water as a pharmaceutically active ingredient, a water-soluble polymer substance and an inorganic porous substance characterized in that
(1) the chemical substance poorly soluble in water forms a solid dispersion together with the water-soluble polymer substance,
(2) the water-soluble polymer substance forming the solid dispersion is in the form of fine pieces, and
(3) the water-soluble polymer substance is in the form of fine pieces is maintained to be adsorbed on and/or adhered to the surface of the inorganic porous substance, and a method for producing said formulation.

The solid formulation of the present invention is excellent in solubility and high in bioavailability and capable of fully sufficiently exhibiting original activity as a pharmaceutically active ingredient with an extremely little dosage. Therefore, the solid formulation of the present invention, when administered to a patient, imposes little burden to the patient and can also reduce apprehension of side effects. Furthermore, the solid formulation has excellent stability and can be produced extremely efficiently and easily by the process of the present invention.

The solid formulation of the present invention can be readily and completely absorbed by the body, and accordingly, when this technique is applied to microsomal triglyceride transfer protein (MTP) inhibiting substances, etc. , it does not simplygive rise to effects of reducing dosage and improving bioavailability but it can be used as an excellent therapeutic or prophylactic agent for hyperlipemia, arterial sclerosis, coronary artery disease, adiposis, diabetes mellitus or hypertension in which side effects such as fatty liver are reduced by selectively inhibiting the target MTP of intestinum tenue.

### Background Art

Hereinbelow, background art on formulation techniques for improving water-solubility of a chemical substance which is poorly soluble in water and serves as a pharmaceutically active ingredient as well as background art on MTP inhibiting substances which are considered to be one of the examples of pharmaceutical products to which the solid pharmaceutical formulation of the present invention is most suitably applied are described.

### 1. Concerning formulation techniques

### 1-1. Solubility and stability of drug

In designing a solid pharmaceutical formulation, in particular an oral formulation, it is considered to be important in aspect of efficacy of pharmaceutical products to design sufficiently high bioavailability.

Many factors affect the bioavailability in oral administration. Although these factors include water solubility, drug absorbency in the whole alimentary canal, dosage intensity and first-pass effect, it is said that the solubility in water is the most important and dominant factor in the development of pharmaceutical products since the dissolution rate is the rate-determining step of absorption particularly in pharmaceutical agents poorly soluble in water.

Unfortunately, however, many chemical substances for pharmaceutical products are poorly soluble in water and only very little of the dosage is transferred and absorbed in the blood and thus they have a defect that the bioavailability is extremely low.

There is a report that 338 pharmaceutical compounds marketed for oral administration except for protein pharmaceutical products were subjected to solubility test of the Japanese Pharmacopeia and summated by classifying in.to categories from "extremely soluble" to "hardly soluble" and as a result 150 analytes, which was 40% of the total samples, were classified as the "hardly soluble" compounds having solubility not more than 0.1 mg/ml.

Although a drug should have high bioavailability, in other words, high solubility so as to exhibit the efficacy thereof as mentioned above, the stability thereof is also an extremely important matter on the other hand since the drugs are stored for a long period of time.

Generally crystallized chemical substances are superior in stability but inferior in solubility to the amorphous substances thereof. In this way, solution of solubility and solution of stability are in an incompatible relationship with each other, and solving the problems of stability and solubility at the same time is an extremely difficult task.

### 1-2. Concerning improvement in solubility of poorly soluble drugs

Various pharmaceutical devices such as conversion into salt compounds, formation of solvates, crystal polymorphism, pulverization of drug into fine powders, mixing and milling with an excipient have been made in order to improve the solubility of these poorly soluble drugs.

However, formulation techniques which maintain stability while improving solubility, particularly formulation techniques which can be generally applicable to any drugs have not been found.

Solid dispersion has been widely used as means for improving solubility of poorly soluble drugs, and a number of poorly soluble drugs have been put into practice as solid dispersion pharmaceutical products. The solid dispersion is defined as "one in which one or more active ingredients are dispersed in a solid-state inert carrier or the matrix thereof prepared by fusion method, solvent method or fusion-solvent method" (see Non-Patent Document 1).

The solid dispersion is a solid mixture obtained by procedures of dissolving a drug in an organic solvent together with a carrier consisting of an inorganic porous substance or a polymer substance to form a uniform liquid phase followed by drying or procedures of fusing both of a polymer substance and a drug followed by solidifying the same, and the drug is uniformly dispersed in the carrier matrix or on the surface thereof as microcrystals or in molecular size, that is, in single-molecule state. Generally, an amorphous substance is in a higher energy state and accordingly has an improved dissolution rate and increases drug level in an aqueous solution as compared with a crystal and, as a result, has an increased absorption and enables the blood concentration to be raised (for example, see Patent Documents 1 and 2).

### 1-3. Concerning production method of solid dispersion

Several kinds of methods have been suggested as methods of producing solid dispersion and particularly practical methods include solvent method, fusion method, spray drying method, mixing and milling method. Of these, fusion method and solvent method are described below.

### (A) Fusion method

Fusion method is a method of heating and melting a drug which is an active ingredient together with a carrier consisting of an inorganic porous substance and a polymer substance and, and thereby obtaining a solid dispersion. For example, there has been reported a production method of solid dispersion consisting of itraconazole and an inorganic porous substance which is characterized by mixing itraconazole, an antifungal agent of triazole type, and an inorganic porous substance selected from the group consisting of calcium silicate, light anhydrous silicic acid, magnesium silicate, magnesium aluminosilicate, magnesium aluminometasilicate, magnesium aluminum silicate, synthetic aluminum silicate and hydrated silicon dioxide followed by heating and melting the mixture and cooling the obtained fused body (for example, see Patent Document 3). Aside from this, there has been reported a method of melt-extruding a mixture containing itraconazole and a water-soluble polymer, and then triturating the melt-extruded mixture (for example, see Patent Document 4).

### (B) Solvent method

Solvent method is a method of dissolving a drug and a carrier which is a water-soluble polymer in a solvent such as an organic solvent and then evaporating the solvent to produce a solid dispersion. It is considered that solubility and bioavailability can be improved because a drug poorly soluble in water is dissolved in a solvent and thereby the drug becomes amorphous and dispersed in the carrier as it is (for example, see Patent Document 5).

As a specific example, there is known a so-called spray drying method in which a liquid having dissolved nifedipine, which is a drug poorly soluble in water, and a polymer matrix such as polyvinylpyrrolidone, hydroxypropylmethylcellulose, methylcellulose in an organic solvent is sprayed and dried on fine powders of lactose and the like granulated with a water-soluble polymer such as hydroxypropylcellulose and thereby a solid dispersion is obtained (for example, see Patent Documents 6 and 7).

There is also reported that a drug poorly soluble in water such as cycloheptadines and a water-soluble polymer and the like such as polyvinylpyrrolidone, hydroxypropylmethylcellulose and hydroxypropylcellulose are dissolved in a water/alcohol system and then sprayed and granuled on lactose and thereby a solid dispersion is prepared (for example, see Patent Document 5).

There is also reported "As for a specific drug poorly soluble in water, some techniques are known in which a drug is dissolved in an organic solvent and mixed with an inorganic porous substance or a polymer substance, and after that the solvent is evaporated to thereby allow the drug to be adsorbed on inorganic porous substance or to be dispersed in the polymer substance. Such methods, however, cannot be applied on all the poorly soluble drugs and, in addition, physical properties of the poorly soluble drug had to be examined in detail so as to determine the most suitable substances by trial and error." (for example, see Patent Document 8).

A pharmaceutical composition with improved solubility and absorbency and the production method thereof by allowing 3-bis(4-methoxyphenyl)methylene-2-indolinone to be amorphous in the presence of a polymer substance and/or an inorganic porous substance are also known and in addition, as the specific production methods, a method for obtaining amorphous product by heat-fusion method or solvent method using TAS-301 as the inorganic porous substance and a method for obtaining amorphous product by solvent method using a polymer substance are also known. However, technical significance of using both a polymer substance and an inorganic porous substance at the same time has not been specifically known at all (for example, see Patent Document 8).

### (C) Carrier for solid dispersion

There are two types of carrier for solid dispersion, i.e., polymer substance and inorganic porous substance.

As a polymer substance, those which can dissolve in water or a weakly basic or strongly basic buffer and are pharmaceutically accepted can be used, but the type thereof is not particularly limited. For example, hydroxypropylmethylcellulose (Shin-Etsu Chemical tradename TC-5), hydroxypropylcellulose, hydroxypropylmethylcellulose phthalate, hydroxypropylmethylcellulose acetate succinate, methylcellulose, carboxymethylethylcellulose, polyvinylpyrrolidone (BASF AG: trade name, Colidone), polyethylene glycol, ethyl acrylate-methyl methacrylate copolymer or methacrylic acid copolymer or a mixture thereof can be used (for example, see Patent Document 8).

On the other hand, an inorganic porous substance is a fine particle or powder having a number of fine pores and it is particularly not limited as long as it is stable at a heating melting temperature of the active ingredient, and, for example, calcium silicate, light anhydrous silicic acid, magnesium silicate, magnesium aluminosilicate, magnesium aluminometasilicate, magnesium aluminum silicate, synthetic aluminum silicate, hydrated silicon dioxide, etc. are used. The particle size is from 0.01 µ to 100 µ, and averagely 0.1 to 10 µ.

The amount to be used of the polymer substance and/or inorganic porous- substance is not particularly limited, and varies depending on the type of drug and carrier to be used but it is said that the substance is suitably used in an amount of 0.5 to 5 times the drug on a weight basis in many cases (for example, see Patent Document 8).

Separately from this, formulations having blended therein a hydrophobic excipient, a stabilizing agent and a disintegrating agent are known.

For example, granulated particles characterized by containing (a) a solid solution of an amorphous and pharmaceutically active drug which is crystalline in normal conditions and slightly water-soluble under ambient pressure and at ambient temperature and a pharmaceutically acceptable hydrophobic excipient, (b) a stabilizing agent including polyethylene glycol, sugar, sorbitol, mannitol, polyvinylpyrrolidone or one or more cellulose ethers and (c) a disintegrating agent including croscarmellose sodium, sodium starch glycollate, crosspovidone or a cross-linked polyacrylate are known (for example, see Patent Documents 9 and 10).

### (D) Concerning blending ratio of drug and carrier in solid dispersion

As for the ratio of drug and matrix (carrier) in the solid dispersion, there is a report, for example, in the case of the nifedipine which is one of calcium antagonists generally used in the prevention and therapy of stenocardia and hypertension, that the ratio of the polymer substance (hydroxypropylmethylcellulose) to the drug (nifedipine) is conveniently 1.5 to 3 weight parts. The reason for adopting such a blending ratio is that if the blending amount of the matrix is not more than 1 weight part for 1 weight part of nifedipine, a composition in the form of a solid dispersion cannot be obtained and in the case of 7 weight parts or more, volume of the composition increases and a formulation produced by using this is unpreferably large in the dimension (for example, see Patent Document 11).

The above-mentioned technique of solid dispersion is an excellent technique but even this solid dispersion technique is not an absolute one and does not always provides an amorphous substance, and as a result, may not increase the dissolution rate of the drug. For example, some parameters such as interaction between the polymer and the active ingredient, the ratios thereof and adopted manufacturing technique affect chemical and physical characteristics of the obtained solid dispersion. On this account, there is a report that it is necessary to select both the polymer and the operation conditions for the specific active ingredient respectively in order to produce a dispersion which brings about conversion to amorphous form and retaining the amorphous form of the product (for example, see Patent Document 12).

In addition, when either of the above-mentioned fusion method and the solvent method is adopted, the solid dispersion obtained in this way should be milled, screened and mixed with other substances as required and formed into a formulation such as granule and tablet (for example, see Patent Document 13). In particular, the solvent method may result in a film-like dispersed product depending on the production conditions and in this case it was necessary to cut the film into pieces.

### 4-4. Concerning Stability

Stability of a formulation is one of the important factors to secure effectiveness and quality of a pharmaceutical product.

Upon the approval application of a pharmaceutical product, stability tests such as a long-term preservation test, an acceleration test and a severe test of the drug substance and the formulation thereof are required in order to obtain the information necessary for determining the preservation method and validated period of the pharmaceutical product.

Generally, a chemical substance is more stable in a crystal state than in an amorphous state. From a point of view of solubility, however, a problem is involved that a drug which forms a more stable crystal is more difficult to dissolve.

### 2. Concerning MTP

A lipid absorption inhibitor, in particular MTP inhibiting substance is exemplified and described below as an example of a drug poorly soluble in water.

MTP is expressed in both organs of liver and intestinum tenue and is a transfer protein catalyzing the migration of triglyceride, cholesteryl ester and phosphatidylcholine between small monolayer cytoplasmic membranes and generates lipoproteins.

In this way, MTP can not only lipidize apo B-100 newly synthesized in the liver but also lipidize apo B-48 newly synthesized in the intestine.

Therefore, each will generate lipoparticles rich in triglyceride such.as VLDL (super very low density lipoprotein) and chylomicron. Thus, it can be expected that an MTP inhibiting substance and/or an Apo B secretion inhibiting substance will reduce the plasma level of LDL-c and triglyceride and have an effect of decreasing lipid absorption in the intestine.

Accordingly, an MTP inhibiting substance and/or an Apo B secretion inhibiting substance can be used for the treatment of non-insulin dependence diabetes mellitus, arteria coronaria heart failure, pancreatitis, cholesteremia, hypertriglyceridaemia, hyperlipidemia, mixed dyslipidemia, hyperlipidemia after eating, atherosclerosis and obesity.

### 2-1. MTP and Apo B and lipid in blood

It has been said from before that hyperlipemia, diabetes mellitus, hypertension and the like are one of the risk factors in arterial sclerosis , Hyperlipemia refers to a state where lipids such as cholesterol has abnormally increased in blood, and the causes thereof include primary hyperlipemia resulted from genetic abnormalities of enzyme and protein participating in the metabolism of low density lipoprotein (LDL), lipoprotein receptor, etc., secondary hyperlipemia derived from various kinds of diseases and drug administration, and acquired hyperlipemia based on overnutrition.

Lipid taken from meal is absorbed in intestinum tenue by the work of bile acid, and secreted to the blood via lymph duct as chylomicron. As for the secreted chylomicron, the triglyceride (TG) moiety is decomposed to free fatty acids by the work of lipoprotein lipase (LPL) present in the capillary vessel, and it is converted to chylomicron remnant having a high content of cholesteryl ester (CE), and taken up in the liver through hepatic chylomicron remnant receptor. Furthermore, the chylomicron remnant taken up in the liver is converted into CE and TG and associated with apolipoprotein B synthesized on granular endoplasmic reticulum and forms VLDL. This VLDL is transferred to Golgi apparatus, secreted outside the cell after modification, and converted into intermediate density lipoprotein (IDL) by the work of LPL, then to LDL by hepatic triglyceride lipase (HTGL), and the lipid is distributed among the peripheral tissues.

Presence of a protein having transfer activity of TG and CE into intestinum tenue and hepatic microsomal fraction was pointed out from the past at the time of chylomicron formation in the intestinum tenue or VLDL formation in the liver, and the protein from the microsomal fraction of bovine liver, i.e., MTP was isolated and purified from the liver and intestine by Wetterau et al. in 1985 (see Non-Patent Document 2). However, this MTP did not come into the limelight in the field of clinical medicine until a report appeared in 1993 that the cause of a-β-lipoproteinemia was deficiency of MTP. That is, this disease is characterized in that although there is no aberration in gene relating to apolipoprotein B, apolipoprotein B is scarcely detected in serum, serum cholesterol is not higher than 50 mg/dL, serum triglyceride shows an extremely low value, and besides, there is no lipoprotein containing apolipoprotein B such as chylomicron, VLDL and LDL in blood at all. This showed that MTP is a protein indispensable for the association of apolipoprotein B with TG and CE, and the formation of VLDL and chylomicron, and that it plays an essential role in the secretion thereof.

Because lipids are inherently insoluble in water, lipids in the blood associate with hydrophilic protein called apolipoprotein, and exists as so-called lipoproteins. Each of VLDL, IDL, LDL or chylomicron participating hyperlipemia is a lipoprotein. MTP is present in hepatic cells and microsomal fraction of intestinum tenue epidermic cell and in charge of transfer of TG and CE in the cell. In the liver and the intestinum tenue, as the apolipoprotein B (apolipoprotein B100 in the liver and apolipoprotein B48 in the intestinum tenue) is synthesized, TG and CE associate with respective apolipoprotein B through the transfer action of MTP, and VLDL or chylomicron is formed. As a result, these lipoprotein are extracellularly secreted as VLDL in the liver and as chylomicron in the intestinum tenue.

MTP can be said to be indispensable in constructing this lipoprotein. That is, transfer of lipids such as TG or CE to apolipoprotein is inhibited by inhibiting the activity of MTP and the formation of lipoprotein is inhibited.

In the meantime, it has been clarified that LDL closely participates in the development of arterial sclerosis in general and, and LDL goes vascular endothelium and deposits on the intercellular matrix of vascular wall, where it is oxidated and denatured, and peroxidated lipid and denatured protein cause a series of inflammatory reaction, resulting invasion of macrophage, deposition and foaming of lipids, migration and proliferation of smooth muscle cells and increase of the intercellular matrix, etc. and an arterial sclerosis nest in the vascular wall is formed. Therefore, it is considered that prevention or therapy of arterial sclerosis, coronary artery disease and hypertension can be performed by decreasing LDL.

In this way, MTP can not only lipidize apo B-100 newly synthesized in the liver but also lipidize apo B-48 newly synthesized in the intestine.

Therefore, it can be expected that an MTP inhibiting substance and/or an Apo B secretion inhibiting substance will inhibit the formation of lipoproteins such as chylomicron, VLDL, LDL, reduce the plasma level of LDL-c and triglyceride and besides decreases lipid absorption in the intestine, and thus it is expected as a new type prophylactic or therapeutic agent of hyperlipemia, arterial sclerosis, coronary artery disease, diabetes mellitus, adiposis or hypertension and further as a prophylactic or therapeutic agent of pancreatitis, cholesteremia, hypertriglyceridaemia.

In the meantime, as the development of MTP inhibitor proceeds, it was pointed out that although the MTP inhibiting substance exhibited an effect of decreasing lipids in the blood, it had a possibility to bring about adiposity (fatty liver) in the liver, and hepatotoxicity came to be concerned about. Therefore, if MTP in the intestinum tenue alone can be selectively and immediately inhibited before it reaches the liver, triglyceride and cholesterol in the blood can be significantly decreased without being accompanied with side effects such as fatty liver.

Under these circumstances, newMTP inhibitor without side effects such as fatty liver and the improvement formulation thereof are strongly desired.

### 2-2. MTP inhibiting substance

As the MTP inhibiting substance and/or Apo B secretion inhibitor as mentioned above, particularly as the MTP inhibiting substance, the following compounds are already known.
(A) The following 3-piperidyl-4-oxoquinazoline derivatives are described in Japanese Patent Laid-Open No. 11-228569 (see Patent Document 14). 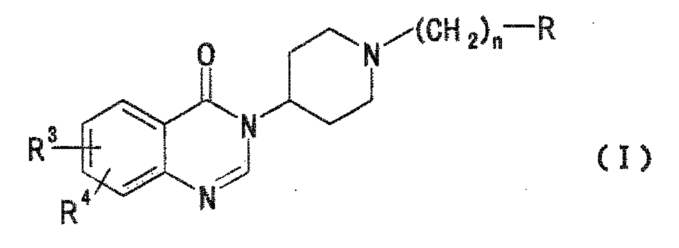 In the formula, R is a formula (i): 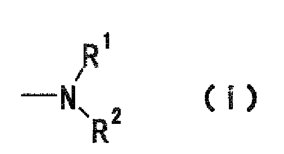 wherein R1 and R2 maybe the same or different and each represents a hydrogen atom; a lower alkyl group having 1 to 4 carbon atoms; an aralkyl group; a benzoyl group; a cycloalkyl group having 3 to 7 carbon atoms; an aryl group; or a heteroaryl group containing 1 to 3 heteroatoms selected from a nitrogen atom, a sulfur atom and an oxygen atom, wherein the cycloalkyl group, aryl group or heteroaryl group may be substituted with 1 to 3 substituents selected from a halogen atom; a lower alkyl group having 1 to 4 carbon atoms; a lower haloalkyl group having 1 to 4 carbon atoms; a nitro group; a lower alkoxy group having 1 to 4 carbon atoms; an amino group; an amino group substituted with a lower alkyl group having 1 to 4 carbon atoms; a hydroxyl group; a phenoxy group; and a sulfo group, and at least one of R1 or R2 is not a hydrogen atom; or a formula (ii): 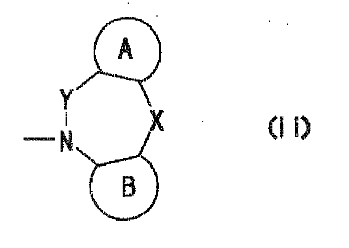 wherein A and B may be the same or different and each represents an aromatic hydrocarbon ring; an aromatic heterocycle containing 1 to 3 heteroatoms selected from a nitrogen atom, a sulfur atom and an oxygen atom; a cycloalkane ring having 3 to 7 carbon atoms; or a cycloalkene ring having 5 to 7 carbon atoms, wherein the aromatic hydrocarbon ring, aromatic heterocycle, cycloalkane ring or cycloalkene ring may be substituted with 1 to 3 substituents selected from a halogen atom; a lower alkyl group having 1 to 4 carbon atoms; a lower haloalkyl group having 1 to 4 carbon atoms; a hydroxyl group; a nitro group; a lower alkoxy group having 1 to 4 carbon atoms; an amino group; an amino group substituted with a lower alkyl group having 1 to 4 carbon atoms; and a sulfo group) and X is a single bond; a linear or branched lower alkyl group having 1 to 4 carbon atoms; a linear or branched lower alkenylene group having 2 to 4 carbon atoms, wherein the lower alkyl group or lower alkenylene group may be substituted with a group selected from a halogen atom; a hydroxyl group; a nitro group; a lower alkoxy group having 1 to 4 carbon atoms; an amino group; and a sulfo group; an oxygen atom; a sulfur atom; an imino group which may be substituted with a lower alkyl group having 1 to 4 carbon atoms; a carbonyl group; -O-Z-; -Z-O-; -S-Z-; -Z-S-; -NH-Z-; -NR5-Z-; -Z-NH-; or -Z-NR5-, wherein Z is a lower alkyl group having 1 to 4 carbon atoms; or a carbonyl group, and R5 is a lower alkyl group having 1 to 4 carbon atoms, and Y which is a single bond; a linear or branched lower alkyl group having 1 to 4 carbon atoms; or a carbonyl group, n is an integer selected from 1 to 4, R3 and R4 may be the same or different and each represents a hydrogen atom; a lower alkyl group having 1 to 4 carbon atoms; a halogen atom; a lower haloalkyl group having 1 to 4 carbon atoms; a hydroxyl group; an amino group; or a nitro group.
(B) An MTP inhibiting substance consisting of a benzamide compound having a piperidine ring as follows is described in National Publication of International Patent Application No. 2004-514676 (WO 2002/042291) (see Patent Document 15). 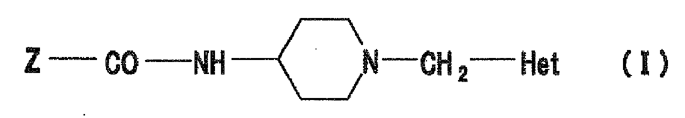
   In the formula, Z represents biphenyl which may be optionally substituted with one or more substituent groups selected from trihalomethyl and trihalomethoxy at 2'-position, 3'-position, 4'-position, 5'-position and 6'-position, and Het represents quinolyl or quinoxalyl or pyridyl which may be optionally substituted with one or more substituent groups selected from halo, cyano, nitro, (C₁ to C₆) alkyl, (C₆ to C₁₂) aryl, (C₁ to C₆) alkoxy, hydroxyl, (C₁ to C₆) thioalkoxy, carboxyl and (C₁ to C₆) alkoxycarbonyl.
(C) An MTP inhibiting substance consisting of a benzamide derivative represented by the following formula is described in National Publication of International Patent Application No. 2003-535900 (WO 01/097810) (see Patent Document 16). 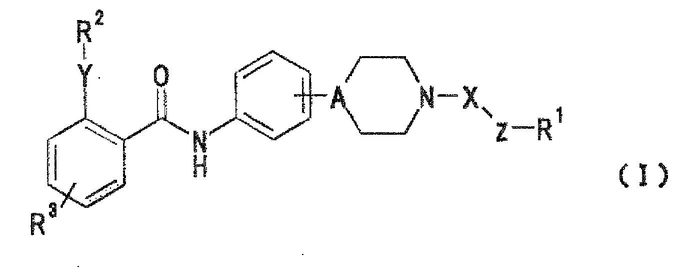
   In the formula,
   A represents N or CH;
   X is selected from the following groups: (i) -C₁₋₆ alkylene-, optionally containing one or two double bonds and optionally substituted with one or more hydroxy, C₁₋₆ alkyl, C₁₋₆ alkoxy, C₁₋₆ acyl or C₁₋₆ acyloxy groups, (ii) oxo, sulfonyl, thioxo, (iii) -C₁₋₆ alkylenecarbonyl-, -C₁₋₆ alkylenesulfonyl-, -C₁₋₆ alkylenethioxo-, (iv) -C₂₋₆ alkyleneoxy-, -C₂₋₆alkylenethio-, -C₂₋₆ alkylene (N-H or N-C₁₋₆ alkyl)amino-, (v) -C₁₋₆ alkylenecarboxy-, -C₁₋₆ alkylenethioamido-, -C₁₋₆ alkylene (N-H or N-C₁₋₆ alkyl)carboxamido-, and (vi) -C₂₋₆ alkyleneoxycarbonyl-, -C₂₋₆ alkylenethiocarbonyl-, -C₂₋₆ alkylene (N-H or N-C₁₋₆ alkyl)aminocarbonyl-;
   Z represents a direct link or-C₁₋₆ alkylene-, optionally containing one double bond and optionally substituted with one or more hydroxy, C₁₋₆ alkyl, C₁₋₆ alkoxy, C₁₋₆ acyl or C₁₋₆ acyloxy groups;
   R¹ is selected from the following groups: (i) hydrogen, C₁₋₃ perfluoroalkyl, (ii) C₆₋₁₀ aryl, C₃₋₈ cycloalkyl and fused benz derivatives thereof, C₇₋₁₀ polycycloalkyl, C₄₋₈ cycloalkenyl, C₇₋₁₀ polycycloalkenyl, (iii) a heterocyclyl selected from the group consisting of monocyclic radicals and fused polycyclic radicals, wherein said radicals contain a total of from 5 to 14 ring atoms, wherein said radicals contain a total of from 1-4 ring heteroatoms independently selected from oxygen, nitrogen and sulfur, and wherein individual rings of said radicals may be independently saturated, partially unsaturated, or aromatic, and (iv) where either X is C₁₋₆ alkylene and Z is a direct link, or Z is C₁₋₆ alkylene, R¹ additionally may represent a halogen, cyano, nitro or C₁₋₆ acyl group,
      wherein, when R¹ contains one or more rings, said rings may each independently bear 0 to 4 substituents independently selected from (i) halogen, hydroxy, cyano, nitro, formyl, C₁₋₆ alkylsulfonylamino, (ii) C₁₋₆ alkyl, C₃₋₈ cycloalkyl, C₁₋₃ perfuoroalky, (iii) C₁₋₆ alkoxy, methylenedioxy, C₁₋₃ perfuoroalkoxy C₁₋₆ alkylthio, (iv) amino, C₁₋₆ alkylamino, di-C₁₋₆ alkylamino, (v) phenyl, phenoxy, phenylthio, halophenylthio, benzyl, benzyloxy, (vi) hydroxycarbonyl, C₁₋₆ alkoxycarbonyl, (vii) aminocarbonyl, C₁₋₆ alkylaminocarbonyl, di-C₁₋₆ alkylaminocarbonyl, di-₁₋₆ alkylaminocarbonyl C₁₋₆ alkoxy, C₁₋₃perfluoroalkylaminocarbonyl, (viii) C₁₋₆ acyl, C₁₋₆ acyloxy, C₁₋₆ acyloxy C₁₋₆ alkyl, C₁₋₆ acylamino, and (ix) an aromatic heterocyclyl consisting of monocyclic radicals,
      wherein said radicals contain 5 to 6 ring atoms, wherein said radicals contain a total of from 1 to 4 ring heteroatoms independently selected from oxygen, nitrogen and sulfur, and where each of the said heterocyclyl groups is optionally substituted with one or more groups independently selected from halogen, C₁₋₄ alkyl, C₁₋₄ alkoxy, C₁₋₃ perfluoroalkyl and C₁₋₃ perfluoroalkoxy;
   Y represents a direct or oxylink, -C₁₋₆ alkylene-, -oxy C₁₋₆ alkylene- or a heterocyclyl consisting of monocyclic radicals, wherein said radicals contain 5 ring atoms, and wherein said radicals contain a total of from 1 to 4 ring heteroatoms independently selected from oxygen, nitrogen and sulfur and wherein the ring may be independently saturated, partially unsaturated, or aromatic;
   R² represents phenyl, C₃₋₈ cycloalkyl, or a heterocyclyl consisting of monocyclic radicals, wherein said radicals contain a total of from 5 to 6 ring atoms, wherein said radicals contain a total of from 1 to 4 ring heteroatoms independently selected from oxygen, nitrogen and sulfur, wherein the ring may be independently saturated, partially unsaturated, or aromatic, and where each R² is optionally substituted with one or more groups independently selected from halogen, C₁₋₄ alkyl, C₁₋₄ alkoxy, C₃₋₈ cycloalkyl, C₁₋₃ perfluoroalkyl, C₁₋₃ perfluoroalkoxy, hydroxycarbonyl, C₁₋₆alkoxycarbonyl, cyano, nitro, C₁₋₄ alkylaminosulfonyl; and
   R³ represents hydrogen or one or more groups independently selected from halogen, C₁₋₄ alkyl, C₁₋₄ alkoxy, C₁₋₃ perfluoroalkyl or C₁₋₃ perfluoroalkoxy.
(D) An MTP inhibiting substance consisting of a substituted piperazine derivative represented by the following formula is described in National Publication of International Patent Application No. 2003-521484 (WO 01/047898) (see Patent Document 17). 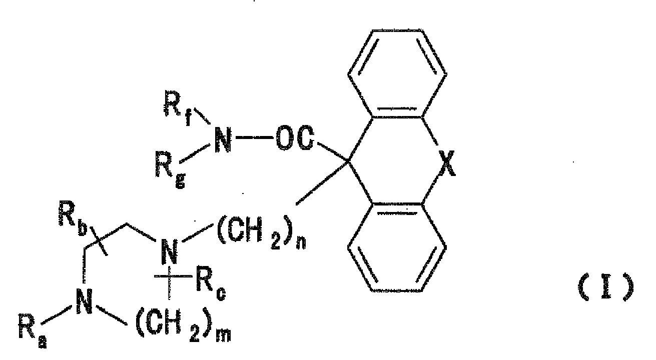
   In the formula, m represents a numeral of 2 or 3, n represents a numeral of 1, 2, 3, 4 or 5, X represents a carbon-carbon bond, an oxygen atom, a methylene, ethylene, imino or N-(C₁₋₃-alkyl)-imino group, Rₐ represents a bi- or tri-nuclear aromatic hydrocarbon wherein an angular methyne group may be substituted with a nitrogen atom, a bi- or tri-nuclear heteroa.romatic hydrocarbon linked to the piperazino group via a carbon atom, consisting of a 5-membered heteroaryl ring containing one or two nitrogen atoms and a cyclopentadienyl ring condensed via a vinylene group, wherein additionally a methyne group may be substituted with a nitrogen atom and/or an angular methyne group may be substituted with a nitrogen atom, a 5-membered heteroaryl ring which contains an imino group optionally substituted with a C₁₋₃-alkyl, phenyl-C₁₋₃-alkyl, phenyl, pyridinyl, pyridazinyl, pyrimidinyl or pyrazinyl group, or an oxygen or sulphur atom, and a phenyl or 6-membered heteroaryl ring condensed via one or two of the available vinylene groups, containing one, two or three nitrogen atoms, wherein the condensed rings may be the same or different, or a naphthyl ring condensed via one of the two available vinylene groups, wherein additionally in a bicyclic or tricyclic group thus formed an angular carbon atom may be substituted with a nitrogen atom, or a 5-membered heteroaryl ring which contains an imino group optionally substituted with a C₁₋₃-alkyl, phenyl-C₁₋₃-alkyl, phenyl, pyridinyl, pyridazinyl, pyrimidinyl or pyrazinyl group, or an oxygen or sulphur atom, and one or two nitrogen atoms, and a phenyl, naphthyl, pyridine, pyridazine, pyrimidine or pyrazine ring condensed via the available vinylene group, wherein an angular carbon atom may be substituted with a nitrogen atom, a naphthyl or 6-membered heteroaryl ring containing one, two or three nitrogen atoms, and a pyridinyl, pyridazinyl, pyrimidinyl or pyrazinyl ring condensed via a vinylene group, wherein an angular carbon atom may be substituted with a nitrogen atom, or a phenyl ring and a 6-membered heteroaryl ring condensed via one or two of the available vinylene groups, containing one, two or three nitrogen atoms, wherein the condensed rings may be the same or different and additionally in a bicyclic or tricyclic group thus formed an angular carbon atom may be substituted with a nitrogen atom, a pyridine, pyrazine or pyridazine ring and a phenyl or 6-membered heteroaryl ring condensed via the two available vinylene groups, containing one, two or three nitrogen atoms, wherein the condensed rings may be the same or different and additionally in a tricyclic group thus formed an angular carbon atom may be substituted with a nitrogen atom, wherein the bi- and tricyclic groups mentioned above under Rₐ may additionally be mono- or disubstituted in the carbon skeleton by a fluorine, chlorine, bromine or iodine atom, by a C₁₋₃-alky, hydroxy, C₁₋₃-alkoxy, carboxy, C₁₋₃-alkoxycarbonyl, aminocarbonyl, C₁₋₃-alkylaminocarbonyl or N,N-di-(C₁₋₃-alkyl)-aminocarbonyl group, wherein the substituents may be the same or different and additionally the hydrogen atoms in the above-mentioned alkyl and alkoxy moieties may be wholly or partly substituted with fluorine atoms, R_{b} and R_{c} independently of one another in each case represent a hydrogen atom or a C₁₋₃-alkyl group, R_{f} and R_{g}, which may be the same or different, represent hydrogen atoms or C₁₋₆-alkyl groups wherein the hydrogen atoms may be wholly or partly substituted with fluorine atoms, C₃₋₇-cycloalkyl, phenyl, C₁₋₃-alkoxy-carbonyl-C₁₋₂-alkyl, carboxy-C₁₋₂-alkyl, methoxy-C₂-₃-alkyl, heteroaryl, phenyl-C₁₋₃-alkyl or heteroaryl-C₁₋₃-alkyl groups, wherein the above-mentioned phenyl and heteroaryl groups may be mono-, di- or tri-substituted in the carbon skeleton by fluorine, chlorine or bromine atoms, by C₁₋₃-alkyl or C₁₋₃-alkoxy groups wherein the hydrogen atoms may be wholly or partially substituted with fluorine atoms, by hydroxy, carboxy, C₁₋₃-alkoxycarbonyl, aminocarbonyl, C₁₋₃-alkyl-aminocarbonyl, N,N-di- (C₁₋₃-alkyl)-aminocarbonyl, N,N-di- (C₁₋₃-alkyl)-amino, nitro or amino groups, wherein the substituents may be the same or different, and/or a hydrogen atom bound to a nitrogen atom of the above-mentioned heteroaryl groups may be substituted with a C₁₋₃-alkyl group wherein the hydrogen atoms may be wholly or partially substituted with fluorine atoms, by a C₁₋₃-alkyl-carbonyl or C₁₋₄-alkoxy-carbonyl group, or R_{f} and R_{g} together with the nitrogen atom between them represent a 3- to 7-membered cycloalkyleneimino group, wherein the methylene group in the 4 position in a 6- or 7-membered cycloalkyleneimino group may additionally be substituted with an oxygen or sulphur atom, by a sulphinyl, sulphonyl, imino or N- (C₁₋₃-alkyl) -imino group, wherein the tricyclic group in the above-mentioned general formula I may be mono- or disubstituted with fluorine or chlorine atoms, by methyl or methoxy groups and the substituents may be the same or different.
(E) An MTP inhibiting substance consisting of a benzamide derivative represented by the following formula is described in National Publication of International Patent Application No. 2003-520270 (WO 01/053260) (see Patent Document 18). 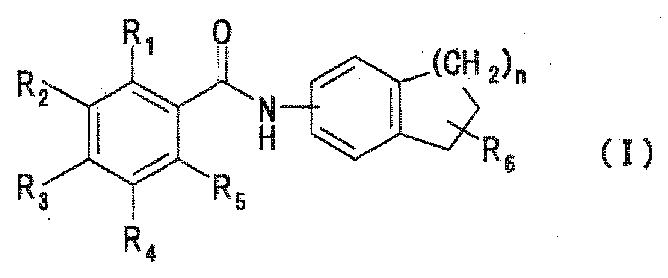
   In the formula, R₂-C, R₃ - C, R₄ - C or R₅-C may be substituted with N; and
   n is 1, 2 or 3;
   R₁ is aryl, heteroaryl or (aryl or heteroaryl)-lower alkoxy; R₂, R₃, R₄ and R₅ are independently hydrogen, lower alkyl, lower alkoxy, halo, trifluoromethyl or cyano;
   R₆ is 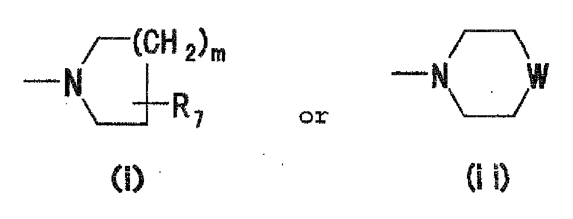
   m is 1, 2 or 3;
   R₇ is hydrogen, lower alkyl, (aryl or heteroaryl)-lower alkyl, lower alkoxy, (aryl or heteroaryl)-lower alkoxy, hydroxy, oxo, lower alkylenedioxy or lower alkanoyloxy;
   W is O, S or NR₈;
   R₈ is -CORa, 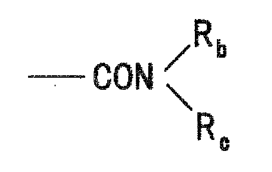
   -COOR_{d}, -SO₂Rₑ, hydrogen, optionally substituted lower alkyl, aryl, heteroaryl or (aryl or heteroaryl)-lower alkyl;
   Rₐ, R_{d} and Rₑ are independently optionally substituted lower alkyl, cycloalkyl, adamantyl, aryl, heteroaryl or (aryl or heteroaryl)-lower alkyl; and
   R_{b} and R_{c} are independently hydrogen, cycloalkyl, optionally substituted lower alkyl, aryl, heteroaryl or (aryl or heteroaryl)-lower alkyl ; or R_{b} and R_{c} together represent lower alkylene.
(F) An MTP inhibiting substance consisting of a substituted piperazine derivative represented by the following formula is described in National Publication of International Patent Application No. 2003-519131 (WO 01/047899) (see Patent Document 19). 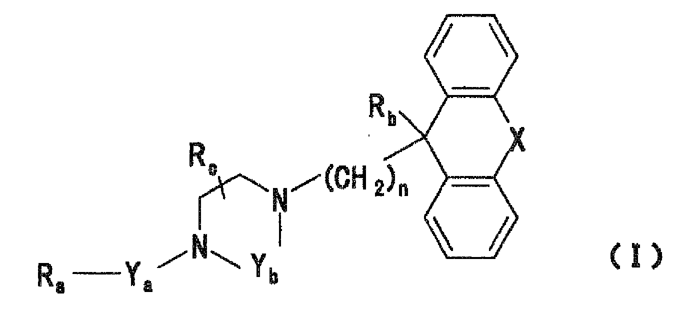
   In the formula, n represents a number 2, 3, 4 or 5, X represents a carbon-carbon bond, an oxygen atom, a methylene, ethylene, imino or N- (C₁₋₃-alkyl) -imino group, Yₐ represents a carbonyl or sulphonyl group, Y_{b} represents the group - (CH₂)ₘ-, wherein m represents a number 2 or 3 and wherein a hydrogen atom may be substituted with a C₁₋₃-alkyl group or a methylene group linked to a nitrogen atom may be substituted with a carbonyl group, Rₐ represents a C₁₋₆-alkoxy, phenyl-C₁₋₃-alkoxy or amino group, wherein the amino group may be mono- or disubstituted with C₁₋₃-alkyl, phenyl-C₁₋₄-alkyl or phenyl groups and the substituents may be the same or different a phenyl, naphthyl, tetrahydronaphthyl, phenoxy or heteroaryl group, a C₁₋₉-alkyl group optionally substituted with a hydroxy, C₁₋₃-alkoxy, C₁₋₄ alkoxycarbonyl or C₁₋₄-alkyl-carbonyloxy group, which may be substituted in the alkyl moiety with a C₁₋₃-alkyl group, by one or two phenyl groups, by a naphthyl, fluorenyl, phenoxy, heteroaryl or C₃₋₇-cycloalkyl group, or a C₃₋₇-cycloalkyl group substituted with a phenyl group, a phenylcarbonyl, naphthylcarbonyl, tetrahydronaphthylcarbonyl, phenoxycarbonyl or heteroarylcarbonyl group, a C₁₋₉-alkylcarbonyl group, which may be substituted in the alkyl moiety by one or two phenyl groups, by a naphthyl, fluorenyl, phenoxy, heteroaryl or C₃₋₇-cycloalkyl group, or a C₃₋₇-cycloalkylcarbonyl group substituted with a phenyl group, wherein all the phenyl, naphthyl and heteroaryl moieties mentioned under Rₐ hereinbefore may be substituted with the groups R₁ and R₂, wherein R₁ represents a hydrogen, fluorine, chlorine or bromine atom, a cyano, C₁₋₃-alkyl, C₂₋₄-alkenyl, phenyl, hydroxy, C₁₋₄-alkoxy, phenyl-C₁₋₃-alkoxy, carboxy, C₁₋₃-alkoxycarbonyl, aminocarbonyl, C₁₋₃-alkylaminocarbonyl, N,N-di-(C₁₋₃-alkyl)-aminocarbonyl, nitro, amino, C₁₋₃-alkylamino, di-(C₁₋₃-alkyl) -amino, phenyl-C₁₋₃-alkylamino, N-(C₁-₃-alkyl)-phenyl-C₁₋₃-alkylamino, C₁₋₃-alkylcarbonylamino, N-(C₁₋₃-alkyl)-C₁₋₃-alkylcarbonylamino, C₁₋₃-alkylsulphonylamino or N-(C₁₋₃-alkyl)-C₁₋₃-alkylsulphonylamino group and R₂ represents a hydrogen, fluorine, chlorine or bromine atom, a C₁₋₃-alkyl, hydroxy or C₁₋₄-alkoxy group, wherein in the above-mentioned alkyl and alkoxy moieties of the groups R₁ and R₂ the hydrogen atoms maybe wholly or partially substituted with fluorine atoms, or R₁ and R₂ together represent a methylenedioxy group, or wherein all the phenyl moieties mentioned above under Rₐ may be substituted with three chlorine or bromine atoms or by three to five fluorine atoms, R_{b} represents a carboxy, C₁₋₆-alkoxycarbonyl, C₁₋₆-alkoxycarbonyl-C₁₋₃-alkylcarbonyl, C₃₋₇-cycloalkoxycarbonyl or phenyl-C₁₋₃-alkoxycarbonyl group or a R₃NR₄-CO group wherein R₃ and R₄, which may be the same or different, represent hydrogen atoms, C₁₋₆-alkyl groups wherein the hydrogen atoms may be wholly or partly substituted with fluorine atoms and the C₁₋₃-alkyl moiety of a C₁₋₃-alkylamino group may be substituted with a carboxy or C₁₋₃-alkoxycarbonyl group or in the 2 or 3 position may also be substituted with an amino, C₁₋₃-alkylamino or di-(C₁₋₃-alkyl)-amino group, C₁₋₃-cycloalkyl, pyridyl, pyridinyl-C₁₋₃-alkyl, phenyl, naphthyl or phenyl-C₁₋₃-alkyl groups, wherein the above-mentioned phenyl groups may be substituted in each case by a fluorine, chlorine or bromine atom, by a C₁₋₃alkyl group wherein the hydrogen atoms may be wholly or partly substituted with fluorine atoms, byahydroxy, C₁₋₃-alkoxy, carboxy, C₁₋₃-alkoxycarbonyl, aminocarbonyl, C₁₋₃-alkylaminocarbonyl, N,N-di-(C₁₋₃-alkyl)-aminocarbonyl or N,N-di- (C₁₋₃-alkyl) -amino group, or R₃ and R₄ together with the nitrogen atom between them represent a 3- to 7-membered cycloalkyleneimino group, wherein the methylene group in the 4 position of a 6 or 7-membered cycloalkyleneimino group may additionally be substituted with an oxygen or sulphur atom, by a sulphinyl, sulphonyl, imino or N-(C₁₋₃-alkyl)-iminogroup, and R_{c} represents a hydrogen atom or a C₁₋₃-alkyl group, wherein the tricyclic group in the above-mentioned general formula I may additionally be mono- or disubstituted with fluorine or chlorine atoms, by methyl or methoxy groups and the substituents may be the same or different, by the above-mentioned heteroaryl groups is meant a 6-membered heteroaryl group, containing one, two or three nitrogen atoms, or a 5-membered heteroaryl group, containing an imino group optionally substituted with a C₁₋₃-alkyl group, an oxygen or sulphur atom or an imino group optionally substituted with a C₁₋₃-alkyl group and one or two nitrogen atoms or an oxygen or sulphur atom and a nitrogen atom, wherein a phenyl ring may be fused to the above-mentioned heteroaryl groups via a vinylene group, and wherein the carboxy group mentioned in the definition of the above-mentioned groups maybe substituted with a group which can be converted into a carboxy group in vivo or by a group which is negatively charged under physiological conditions, and all the above-mentioned saturated alkyl and alkoxy moieties which contain more than 2 carbon atoms maybe straight-chain or branched, unless stated otherwise.
(G) An MTP inhibiting substance consisting of a substituted piperazine derivative represented by the following formula is described in National Publication of International Patent Application No. 2003-509505 (WO 01/021604) (see Patent Document 20). 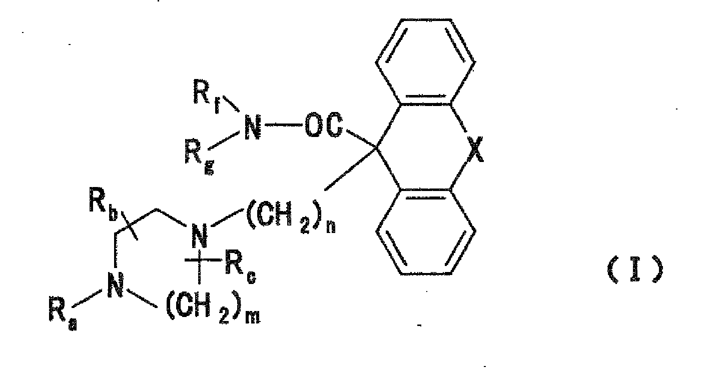
   In the formula, n represents the number 1, 2, 3, 4 or 5, m represents the number 2 or 3, X represents a carbon-carbon bond, an oxygen atom, a methylene, ethylene, imino or N-(C₁₋₃-alkyl)-imino group, Rₐ represents a phenyl group or heteroaryl group substituted with the groups R₁ and R₂, wherein R₁ represents a hydrogen, fluorine, chlorine or bromine atom, a C₁₋₃-alkyl group wherein the hydrogen atoms may be wholly or partly substituted with fluorine atoms, a hydroxy group, a C₁₋₄-alkoxy group wherein the hydrogen atoms may be wholly or partly substituted with fluorine atoms, a phenoxy, heteroaryloxy, phenyl-C₁₋₃-alkoxy, carboxy, C₁₋₃-alkoxycarbonyl, aminocarbonyl, C₁₋₃-alkylaminocarbonyl, N,N-di-(C₁₋₃-alkyl)-aminocarbonyl, nitro, amino, C₁₋₃-alkylamino, di-(C₁₋₃-alkyl) -amino, phenyl-C₁₋₃-alkylamino, N-(C₁₋₃-alkyl)-phenyl-C₁₋₃-alkylamino, C₁₋₃-alkycarbonylamino, N-(C₁₋₃-alkyl)-C₁₋₃-alkylcarbonylamino, C₁₋₃-alkylsulphonylamino or N-(C₁₋₃-alkyl)-C₁₋₃-alkylsulphonylamino group, while the above-mentioned phenyl or heteroaryl moieties of the group R₁ may be substituted with 1 to 5 fluorine, chlorine or bromine atoms, a C₁₋₃-alkyl group wherein the hydrogen atoms may be wholly or partly substituted with fluorine atoms, a hydroxy group, or a C₁₋₄-alkoxy group wherein the hydrogen atoms may be wholly or partly substituted with fluorine atoms, and R₂ represents a hydrogen, fluorine, chlorine or bromine atom, a C₁₋₃-alkyl group wherein the hydrogen atom.s may be wholly or partly substituted with fluorine atoms, or a C₁₋₄-alkoxy group wherein the hydrogen atoms may be wholly or partly substituted with fluorine atoms, or R₁ and R₂ together represent a methylenedioxy group, or Rₐ represents a monocyclic heteroaryl or phenyl group which is substituted in each case by a phenyl or monocyclic heteroaryl group, while the above-mentioned phenyl groups and heteroaryl groups may in each case be substituted with a fluorine, chlorine or bromine atom, a C₁₋₃-alkyl group wherein the hydrogen atoms may be wholly or partly substituted with fluorine atoms, by a hydroxy, C₁₋₃-alkoxy, carboxy, C₁₋₃-alkoxycarbonyl, amino-carbonyl, C₁₋₃-alkylaminocarbonyl or N,N-di-(C₁₋₃-alkyl)-aminocarbonyl group, R_{b} and R_{c} independently of one another represent a hydrogen atom or a C₁₋₃-alkyl group and R_{f} and R_{g}, which may be identical or different, represent hydrogen atoms, C₁₋₆-alkyl groups wherein the hydrogen atoms may be wholly or partly substituted with fluorine atoms, C₃₋₇-cycloalkyl groups, phenyl, heteroaryl, phenyl-C₁₋₃-alkyl orheteroaryl-C₁₋₃-alkyl groups, while the above-mentionedphenyl groups and heteroaryl groups may in each case be substituted with one to three fluorine, chlorine or bromine atoms, by one to three C₁₋₃-alkyl groups wherein the hydrogen atoms may be wholly or partly substituted with fluorine atoms, by one to three hydroxy groups, one to three C₁₋₃-alkoxy groups wherein the hydrogen atoms may be wholly or partly substituted with fluorine atoms, or by a carboxy, C₁₋₃-alkoxycarbonyl, aminocarbonyl, C₁₋₃-alkylaminocarbonyl, N,N-di-(C₁₋₃-alkyl)-aminocarbonyl, N,N-di- (C₁₋₃-alkyl) -amino, nitro or amino group, or R_{f} and R_{g} together with the nitrogen atom between them represent a 3-to 7-membered cycloalkyleneimino group, while the methylene group at the 4-position of a 6- or 7-membered cycloalkyleneimino group may additionally be substituted with an oxygen or sulphur atom, by a sulphinyl, sulphonyl, imino or N-(C₁₋₃-alkyl)-imino group, while the tricyclic group in the above-mentioned general formula I may be mono- or disubstituted with fluorine or chlorine atoms, by methyl or methoxy groups and the substituents may be identical or different and the abovementioned heteroaryl groups means 6-membered heteroaryl groups containing one, two or three nitrogen atoms, or 5-mewbered heteroaryl groups which may contain one to four heteroatoms such as, for example, nitrogen, oxygen and sulphur, while hydrogen atoms bound to nitrogen may optionally be substituted with C₁₋₃-alkyl groups.
(H) An MTP inhibiting substance consisting of a benzamide derivative (biphenylamide derivative) represented by the following formula is described in National Publication of International Patent Application No. 2003-505373 (WO 01/005762) (see Patent Document 21). 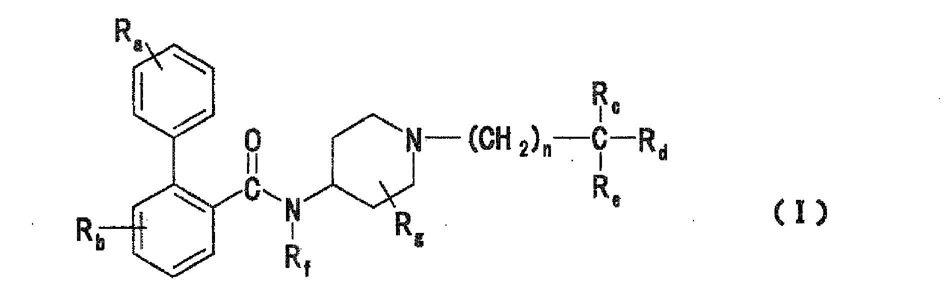
   In the formula, n represents a number 1, 2, 3, 4 or 5, Rₐ and R_{b}, which may be the same or different, each represent a hydrogen, fluorine, chlorine or bromine atom, a C₁₋₃-alkyl group wherein the hydrogen atoms may be wholly or partially substituted with fluorine atoms, a hydroxy, C₁₋₃-alkoxy, amino, C₁₋₃-alkylamino or di-(C₁₋₃-alkyl)amino group, R_{c} represents a hydrogen atom, a C₁₋₁₀-alkyl, C₃₋₇-cycloalkyl or C₃₋₇-cycloalkyl-C₁₋₃-alkyl group, wherein in each case the hydrogen atoms may be wholly or partially substituted with fluorine atoms, a phenyl, naphthyl or monocyclic 5 or 6-membered heteroaryl group optionally substituted with a fluorine, chlorine or bromine atom, by a C₁₋₃-alkyl group wherein the hydrogen atoms may be wholly or partially substituted with fluorine atoms, by a hydroxy, C₁₋₃-alkoxy, carboxy, C₁₋₃-alkoxycarbonyl, aminocarbonyl, C₁₋₃-alkylaminocarbonyl or N,N-di-(C₁₋₃-alkyl)-aminocarbonyl group, by a 3- to 7 -membered cycloalkyleneimino group, wherein the methylene group in the 4 position in a 6- or 7-membered cycloalkyleneimino group may additionally be substituted with an oxygen or sulphur atom, by a sulphinyl, sulphonyl, imino or N-(C₁₋₃-alkyl)-imino group, by a nitro, amino, C₁₋₃-alkylamino, di-(C₁₋₃-alkyl)-amino, C₁₋₃-alkylcarbonylamino, N-(C₁₋₃-alkyl)-C₁₋₃-alkylcarbonylamino, C₁₋₃-alkylsulphonylamino or N-(C₁₋₃-alkyl)-C₁₋₃-alkylsulphonylamino group, wherein the 6-membered heteroaryl group contains one, two or three nitrogen atoms and the 5-membered heteroaryl group contains an imino group optionally substituted with a C₁₋₃-alkyl group, an oxygen or sulphur atom, or an imino group optionally substituted with a C₁₋₃-alkyl group and an oxygen or sulphur atom or one or two nitrogen atoms, and optionally a phenyl ring may be fused to the above-mentioned monocyclic heterocyclic groups via two adjacent carbon atoms, Rₐ represents a phenyl, naphthyl or monocyclic 5 or 6-membered heteroaryl group optionally substituted with a fluorine, chlorine or bromine atom, by a C₁₋₃-alkyl group wherein the hydrogen atoms may be wholly or partially substituted with fluorine atoms, by a hydroxy, C₁₋₃-alkoxy, carboxy, C₁₋₃-alkoxycarbonyl, aminocarbonyl, C₁₋₃-alkylaminocarbonyl or N,N-di-(C₁₋₃-alkyl)-aminocarbonyl group, by a 3- to 7-membered cycloalkyleneimino group, wherein the methylene group may additionally be substituted at the 4-position in a 6- or 7-membered cycloalkyleneimino group with an oxygen or sulphur atom, with a sulphinyl, sulphonyl, imino or N-(C₁₋₃-alkyl)-imino group, by a nitro, amino, C₁₋₃-alkylamino, di-(C₁₋₃-alkyl)-amino, C₁₋₃-alkylcarbonylamino, N-(C₁₋₃-alkyl)-C₁₋₃-alkylcarbonylamino, C₁₋₃-alkylsulphonylamino or N-(C₁₋₃-alkyl)-C₁₋₃-alkylsulphonylamino group, wherein the 6 -memberedheteroaryl group contains one, two or three nitrogen atoms and the 5-membered heteroaryl group contains an imino group optionally substituted with a C₁₋₃-alkyl group, an oxygen or sulphur atom, or an imino group optionally substituted with a C₁₋₃-alkyl group and an oxygen or sulphur atom or one or two nitrogen atoms, and optionally a phenyl ring may be fused to the above-mentioned monocyclic heterocyclic groups via two adjacent carbon atoms, Rₑ represents a carboxy group, a C₁₋₆-alkoxycarbonyl or C₃₋₇-cycloalkoxycarbonyl group wherein the alkyl or cycloalkyl moiety may be substituted in each case from the 2 position, relative to the oxygen atom, by a C₁₋₃-alkoxy, amino, C₁₋₃-alkylamino or di-(C₁₋₃-alkyl)-amino group, a phenyl-C₁₋₃-alkoxycarbonyl or heteroaryl-C₁₋₃-alkoxycarbonyl group, and the heteroaryl moiety is the same as defined above R_{f} represents a hydrogen atom, a C₁₋₃-alkyl or phenyl-C₁₋₃-alkyl group and. R_{g} represents a hydrogen atom or a C₁₋₃-alkyl group.
(I) An MTP inhibiting substance containing as an active ingredient a benzamide derivative represented by the following formula, specifically 4'-trifluoromethyl-biphenyl-3-carboxylic acid (1,2,3,4-tetrahydro-isoquinolin-6-yl)-amide is described in National Publication of International Patent Application No. 2003-510483 (WO 98/47875) (see Patent Document 22). 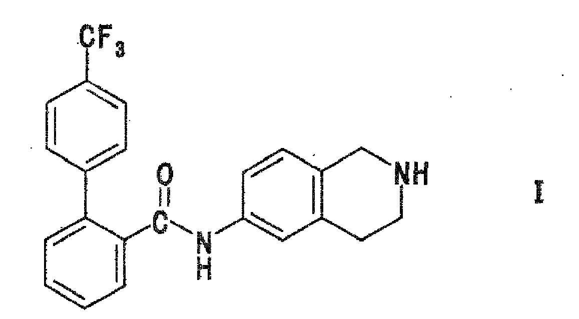
(J) MTP inhibiting substances consisting of benzamide derivatives (biphenylamide derivatives) represented by the following (i), (ii), (iii), (iv) are described in Japanese Patent Laid-Open No. 2001-172180 (see Patent Document 23).
   (i) Formula (1) 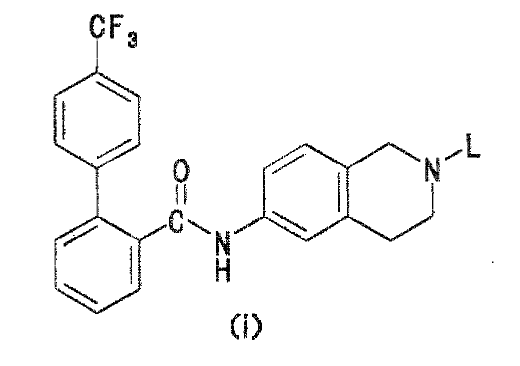
      In the formula, L is (A) X - Y - Z, and X is a moiety selected from the group consisting of CH₂, CO, CS or SO₂; Y is a moiety selected from the group consisting of a direct link; an aliphatic hydrocarbylene having 20 or less carbon atoms which may be monosubstituted with hydroxyl, (C₁-C₁₀)alkoxy, (C₁-C₁₀)acyl, (C₁-C₁₀)acyloxy or (C₆-C₁₀)aryl; NH; and O provided that Y is a direct link when X is CH₂; and Z is a moiety selected from the group consisting of (1) hydrogen, halogen, cyano, (2) hydroxyl, (C₁-C₁₀)alkoxy, (C₁-C₁₀)alkylthio, (C₁-C₁₀)acyl, thiophenylcarbonyl, (C₁-C₁₀)alkoxycarbonyl, (3) (C₁-C₁₀)alkylamino, di(C₁-C₁₀)alkylamino, (C₆-C₁₀)aryl (C₁-C₁₀)alkylamino provided that Y is not O or NH; (4) unsubstituted vinyl, (C₆-C₁₀)aryl, (C₃-C₈)cycloalkyl and condensed derivatives thereof, (C₇-C₁₀)polycycloalkyl, (C₄-C₈)cycloalkenyl, (C₇-C₁₀) polycycloalkenyl, (5) (C₆-C₁₀)aryloxy, (C₆-C₁₀)arylthio, (C₆-C₁₀)aryl (C₁-C₁₀)alkoxy, (C₆-C₁₀)aryl (C₁-C₁₀)alkylthio, (C₃-C₈)cycloalkyloxy, (C₄-C₈)cycloalkenyloxy, and (6) a heterocyclic group selected from the group consisting of a monocyclic group and a condensed polycyclic group wherein the group contains 5 to 14 ring-member atoms in total and 1 to 4 ring-member heteroatoms in total independently selected from oxygen, nitrogen and sulphur, and each ring of the group may be independently saturated, partly unsaturated or aromatic provided that when X is CH₂, Z is H or selected from the groups (4) and (6), and when Z contains one or more rings, each of the rings may independently contain 0 to 4 substituents selected from halo, hydroxy, cyano, nitro, oxo, thioxo, aminosulfonyl, phenyl, phenoxy, phenylthio, halophenylthio, benzyl, benzyloxy, (C₁-C₁₀)alkyl, (C₁-C₁₀)alkoxy, (C₁-C₁₀)alkoxycarbonyl, (C₁-C₁₀)alkylthio, (C₃-C₁₀)alkylamino, (C₁-C₁₀)alkylaminocarbonyl, di(C₁-C₁₀)alkylamino, di(C₁-C₁₀)alkylaminocarbonyl, di (C₁-C₁₀)alkylamino (C₁-C₁₀)alkoxy, (C₁-C₃)perfluoroalkyl, (C₁- C₃)perfluoroalkoxy, (C₁-C₁₀)acyl, (C₁-C₁₀)acyloxy, (C₁-C₁₀)acyloxy(C₁-C₁₀)alkyl and pyrrolidinyl; or (B) G, wherein G is selected from the group consisting of the following groups: (a) phenyl or a heterocyclic ring containing 3 to 14 ring-member atoms wherein each of the heterocyclic rings may be independently saturated, partly unsaturated or aromatic and each of the phenyl and heterocyclic rings may contain 1 to 4 substituent groups independently selected from halogen, hydroxy, cyano, nitro, oxo, thioxo, aminosulfonyl, phenyl, phenoxy, phenylthio, benzyl, benzoyl, benzyloxy, (C₁-C₁₀) alkyl, (C₁-C₄) perfluoroalkyl, (C₁-C₁₀) alkoxy, (C₁-C₄) perfluoroalkoxy, (C₁-C₁₀) alkoxycarbonyl, (C₁-C₁₀) alkylthio, (C₁-C₁₀) alkylamino, di(C₁-C₁₀) alkylamino, (C₁-C₁₀) alkylaminocarbonyl, di(C₁-C₁₀) alkylaminocarbonyl, (C₁-C₁₀) acyl, (C₁-C₄) perfluoroacyl, (C₁-C₁₀) acyloxy, (C₁-C₆) acylamino and (C₁-C₆) perfluoroacylamino; (b) -CH₂CN, (c) 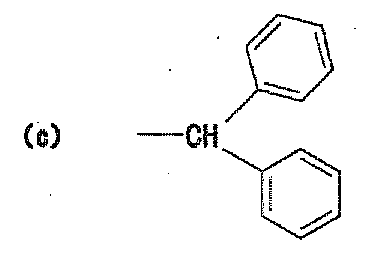 (d) (C₂-C₁₂)alkyl or (C₂-C₁₂) perfluoroalkyl which may be substituted with 1 to 3 substituent groups independently selected from the following substituent groups: (1) phenyl, halogen, nitro, cyano, hydroxyl, -NR¹R², -OCOR³, (C₁-C₄) alkoxy, (C₁-C₄)perfluoroalkoxy, (C₁-C₄)thioalkoxy or (C₁-C₄)perfluorothioalkoxy, wherein each of R¹ and R² are independently selected fromhydrogen, formyl, phenyl, benzyl, benzoyl, (C₃-C₈)cycloalkyl, (C₃-C₈)cycloalkenyl, (C₁-C₄) alkyl, (C₁-C₄)perfluoroalkyl, (C₁-C₁₀)alkoxycarbonyl, (C₁-C₆) acyl, (C₁-C₆) perfluoroacyl, aminocarbonyl, (C₁-C₁₀) alkylaminocarbonyl, di (C₁-C₁₀) alkylaminocarbonyl, aminosulfonyl, (C₁-C₄)alkylaminosulfonyl, di (C₁-C₄) alkylaminosulfonyl, (C₁-C₄)perfluoroalkylaminosulfonyl, di(C₁-C₄)perfluoroalkylaminosulfonyl, (C₁-C₄)alkylsulfonyl, and (C₁-C₄)perfluoroalkylsulfonyl, or R¹ and R² together with the nitrogen atom to which they are bonded, form a saturated, partially-saturated or aromatic heterocyclic ring, wherein said heterocyclic ring contains a total of from 3 to 14 ring atoms and incorporates optionally an additional 1 to 4 ring heteroatoms selected independently from oxygen, nitrogen and sulfur, wherein said heterocyclic ring may have optionally from 1 to 4 substituents selected independently from halogen, hydroxy, cyano, nitro, oxo, thioxo, aminosulfonyl, phenyl, phenoxy, phenylthio, benzyl, benzoyl, benzyloxy, (C₁-C₁₀) alkyl, (C₁-C₄) perfluoroalkyl, (C₁-C₁₀) alkoxy, (C₁-C₄) perfluoroalkoxy, (C₁-C₁₀) alkoxycarbonyl, (C₁-C₁₀) alkylthio, (C₁-C₁₀) alkylamino, di (C₁-C₁₀) alkylamino, (C₁-C₁₀) alkylaminocarbonyl, di (C₁-C₁₀) alkylaminocarbonyl, (C₁-C₁₀) acyl, (C₁-C₁₀) perfluoroacyl, (C₁-C₁₀) acylamino and (C₁-C₁₀) acyloxy, and R³ of -OCOR³ is selected from -NR¹R², phenyl, (C₁-C₁₀)alkyl, (C₁-C₄) perfluoroalkyl, (C₁-C₆) alkoxy and (C₁-C₆)perfluoroalkoxy, (2) (C₃-C₈)cycloalkyl or (C₃-C₈) cycloalkenyl wherein each of said (C₃-C₈) cycloalkyl and (C₃-C₈)cycloalkenyl may have optionally from 1 to 4 substituents selected independently from halogen, hydroxy, cyano, nitro, oxo, thioxo, aminosulfonyl, phenyl, phenoxy, phenylthio, benzyl, benzoyl, benzyloxy, (C₁-C₁₀) alkyl, (C₁-C₄) perfluoroalkyl, (C₁-C₁₀) alkoxy, (C₁-C₄) perfluoroalkoxy, (C₁-C₁₀) alkoxycarbonyl, (C₁-C₁₀) alkylthio, (C₁-C₁₀) alkylamino, di (C₁-C₁₀) alkylamino, (C₁-C₁₀) alkylaminocarbonyl, di(C₁-C₁₀) alkylaminocarbonyl, (C₁-C₁₀) acyl, (C₁-C₁₀) perfluoroacyl, (C₁-C₁₀) acylamino, (C₁-C₁₀) perfluoroacylamino and (C₁-C₁₀) acyloxy, (3) a saturated, partially-saturated or aromatic heterocyclic ring containing a total of from 3 to 14 ring atoms, wherein said heterocyclic ring incorporates a total of from 1 to 4 ring heteroatoms selected independently from oxygen, nitrogen and sulfur, wherein said heterocyclic ring may have optionally from 1 to 4 substituents selected independentlyfromhalogen, hydroxy, cyano, nitro, oxo, thioxo, aminosulfonyl, phenyl, phenoxy, phenylthio, benzyl, benzoyl, benzyloxy, (C₁-C₁₀) alkyl, (C₁-C₄) perfluoroalkyl, (C₁-C₁₀) alkoxy, (C₁-C₄) perfluoroalkoxy, (C₁-C₁₀) alkoxycarbonyl, (C₁-C₁₀) alkylthio, (C₁-C₁₀) alkylamino, di (C₁-C₁₀) alkylamino, (C₁-C₁₀) alkylaminocarbonyl, di (C₁-C₁₀) alkylaminocarbonyl, (C₁-C₁₀) acyl, (C₁-C₁₀) perfluoroacyl, (C₁-C₁₀) acylamino, (C₁-C₁₀) perfluoroacylamino and (C₁-C₁₀) acyloxy, (e) (C₃-C₈) cycloalkyl or (C₃-C₈) cycloalkenyl wherein each of said (C₃-C₈) cycloalkyl and (C₃-C₈) cycloalkenyl may have optionally from 1 to 4 substituents selected independently from halogen, hydroxy, cyano, nitro, oxo, thioxo, aminosulfonyl, phenyl, phenoxy, phenylthio, benzyl, benzoyl, benzyloxy, (C₁-C₁₀) alkyl, (C₁-C₄) perfluoroalkyl, (C₁-C₁₀) alkoxy, (C₁-C₄) perfluoroalkoxy, (C₁-C₁₀) alkoxycarbonyl, (C₁-C₁₀) alkylthio, (C₁-C₁₀) alkylamino, di(C₁-C₁₀) alkylamino, (C₁-C₁₀) alkylaminocarbonyl, di (C₁-C₁₀) alkylaminocarbonyl, (C₁-C₁₀) acyl, (C₁-C₁₀) perfluoroacyl, (C₁-C₁₀) acylamino, (C₁-C₁₀) perfluoroacylamino and (C₁-C₁₀) acyloxy, (f) -(CH₂)ₙCOR⁴, wherein R⁴ of -(CH₂)ₙCOR⁴ is selected from hydroxy, phenyl, -NR¹R² (C₁-C₄) alkyl, (C₁-C₄) perfluoroalkyl, (C₁-C₄)alkoxy, (C₁-C₄)perfluoroalkoxy, (C₃-C₈)cycloalkyl and (C₃-C₈)cycloalkenyl, and n is an integer from 1 to 4.
   (ii) BMS-197.636
      (9-[4-[4-(2,3-dihydro-1-oxo-1H-isoindol-2-yl)-1-piperidin yl]butyl]-N-propyl-9H-fluorene-9-carboxamide) and the pharmaceutically acceptable salts thereof.
   (iii) the compound BMS-200150
      (2-[1-(3,3-diphenylpropyl)-4-piperidinyl]-2, 3-dihydro-1H-isoindol-1-one) and the pharmaceutically acceptable salts thereof.
   (iv) the compound BMS-201038
      (9-[4-(4-[2-(4-trifluoromethylphenyl)benzoylamino]piperid in-1-yl)butyl]-N-2,2,2-trifluoroethyl)-9H-fluorene-9-carb oxamide) and the pharmaceutically acceptable salts thereof.

   In addition, the following compounds are described in Patent Document 23 as specific examples. · 4'-trifluoromethyl-biphenyl-2-carboxylic acid-(2-butyl-1,2,3,4-tetrahydroisoquinolin-6-yl)-amide, · 4'-trifluoromethyl-biphenyl-2-carboxylic acid-[2-(2-acetylaminoethyl)-1,2,3,4-tetrahydroisoquinoli n-6-yl)-amide,
   · 9-[4-[4-(2,3-dihydro-1-oxo-1H-isoindol-2-yl)-1-piperidiny l]butyl]-N-propyl-9H-fluorene-9-carboxamide and
   · 2-[1-(3,3-diphenylpropyl)-4-piperidinyl]-2,3-dihydro-1H-i soindol-1-one.
(K) An MTP inhibiting substance consisting of a benzamide derivative as shown blow is described in Japanese Patent Laid-Open No. 2003-169395 (see Patent Document 24). · 4'-trifluoromethyl-biphenyl-2-carboxylic acid-[2-(1H-[1,2,4]triazol-3-ylmethyl)-1,2,3,4-tetrahydro isoquinolin-6-yl]-amide, · 4'-trifluoromethyl-biphenyl-2-carboxylic acid-[2-(2-acetylaminoethyl)-1,2,3,4-tetrahydroisoquinoli n-6-yl]-amide,
   · 9-(4-[4-[4'-trifluoromethyl-biphenyl-2-carbonyl)amino]-pi peridin-1-yl}butyl)-9H-fluorene-9-carboxylic acid-(2,2,2-trifluoroethyl)-amide,
   · 9-[4-[4-(2-benzothiasol-2-yl-benzoylamino)piperidin-1-yl]butyl]-9H-fluorene-9-carboxylic acid-(2,2,2-trifluoroethyl)amide,
   · [11a-R]-8-[(4-cyanophenyl)methoxy]-2-cyclopentyl-7-(prop-2-enyl)-2,3,11,11a-tetrahydro-6H-pyrazino[1,2b]isoquinoli ne-1,4-dione,
   · [11a-R]-2-cyclopentyl-7-(prop-2-enyl)-8-[(pyridin-2-yl)me thoxy]-2,3,11,11a-tetrahydro-6H-pyrazino[1,2b]isoquinolin e-1,4-dione, · 2-cyclopentyl-2-[4-(2,4-dimethylpyrido[2,3-b]indol-9-ylme thyl)phenyl]-N-(2-hydroxy-1-phenylethyl)acetamide and
   · 2-cyclopentyl-N-(2-hydroxy-1-phenylethyl)-2-[4-(quinolin-2-ylmethoxy)-phenyl]acetamide.

### 2-3. Problems unsolved in MTP inhibiting substances

In the meantime, while various MTP inhibiting substances have come to be known as above, as the development of MTP inhibitor proceeds, it was pointed out that although the MTP inhibiting substance exhibited an effect of decreasing lipids in the blood, it had a possibility to bring about adiposity (fatty liver) in the liver, and hepatotoxicity came to be concerned about (see Patent Document 25).

Under these circumstances, new MTP inhibitor without side effects such as fatty liver and having sufficient prophylactic and therapeutic effects is strongly desired.
(L) Researches on compounds selectively inhibiting MTP of intestinum tenue have been extensively performed recently, and compounds for intestinum tenue selective MTP inhibition represented by the following general formula (1) has been reported in Japanese Patent Laid-Open No. 2003-321424 (see Patent Document 26).
   An ester compound represented by the general formula (1); 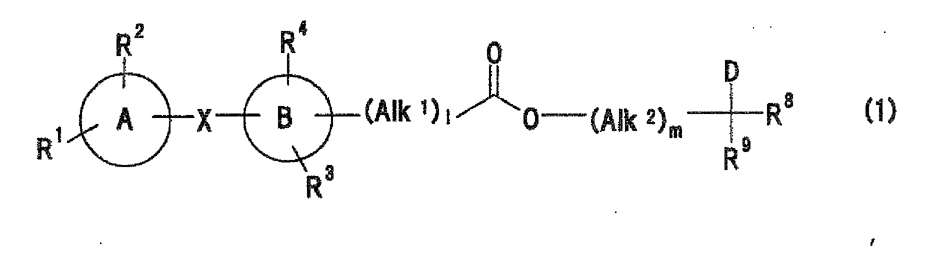 wherein R¹ and R² are a hydrogen atom, a C₁₋₆ alkyl group, a C₃₋₇ cycloalkyl group, a C₁₋₆ alkoxygroup, a halo C₁₋₆alkyl group, a halo C₁₋₆ alkyloxy group, a C₆₋₁₄ aryl group which may be substituted, a C₇₋₁₆ aralkyl group which may be substituted, a C₆₋₁₄ aryloxy group which may be substituted, a C₇₋₁₆ aralkyloxy group which may be substituted, a C₇₋₁₅ arylcarbonyl group which may be substituted, a hetero ring group which may be substituted, a C₂₋₇ alkoxycarbonyl group, a halogen atom, a C₂₋₆ alkenyl group or -N (R⁴⁰) (R⁴¹), wherein R⁴⁰ and R⁴¹ maybe the same or different, and are a hydrogen atom or a C₆₋₁₄ aryl group which may be substituted; ring A is a C₆₋₁₄ aryl group, a heterocyclic group or 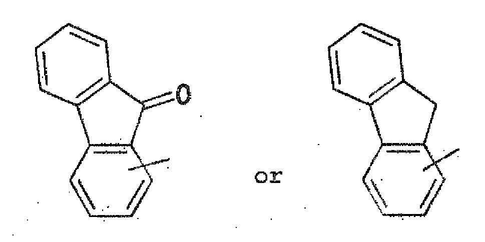
   X is -COO- (CH₂)ₙ- , -CON (R¹⁰)-(CH₂)ₙ or -N-(R¹⁰)-CO-(CH₂)ₙ-, wherein R¹⁰ is a hydrogen atom, a C₁₋₆ alkyl group or a C₃₋₇ cycloalkyl group, and n is 0 or an integer of 1 to 3; R³ and
   R⁴ may be the same or different, and are a hydrogen atom, a hydroxyl group, a halogen atom, a C₁₋₆ alkyl group which may be substituted, a C₁₋₆ alkoxy group, a halo C₁₋₆ alkyl group, a C₇₋₁₆ aralkyloxy group, a C₁₋₆ acyl group, a heterocyclic group which may be substituted, -CON(R¹¹) (R¹²), wherein R¹¹ and R¹² may be the same or different, and are a hydrogen atom, a C₁₋₆ alkyl group, a C₆₋₁₄ aryl group which may be substituted or a C₇₋₁₆ aralkyl group which may be substituted, a C₁₋₆ alkoxy group, or may form, together with the nitrogen atom to which they are bonded, 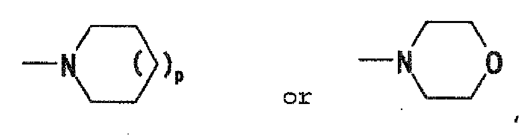 wherein p is 0 or an integer of 1 to 2, -(CH₂)_{q}-N(R¹³) (R¹⁴), wherein R¹³ and R¹⁴ may be the same or different, and are a hydrogen atom, a C₁₋₆ alkyl group, a C₂₋₇ alkoxycarbonyl group or a C₁₋₆ acyl group, or may form, together with the nitrogen atom to which they are bonded, 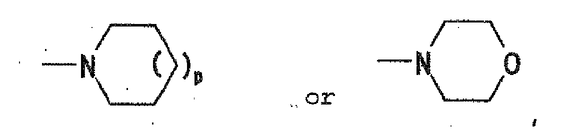 wherein p is the same meaning as above, and q is 0 or an integer of 1 to 3, or -CO(R¹⁵), wherein R¹⁵ is a hydroxyl group, a C₁₋₆ alkoxy group, a C₆₋₁₄ aryloxy group which may be substituted, a C₇₋₁₆ aralkyloxy group which may be substituted or a C₁₋₆ alkyl group; ring B is 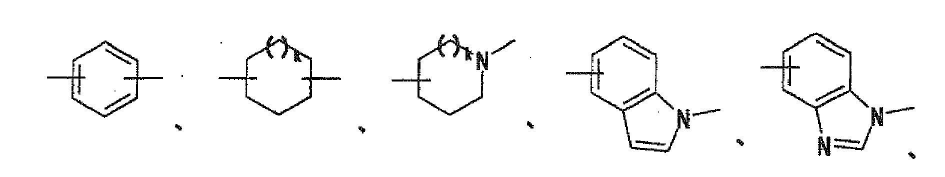 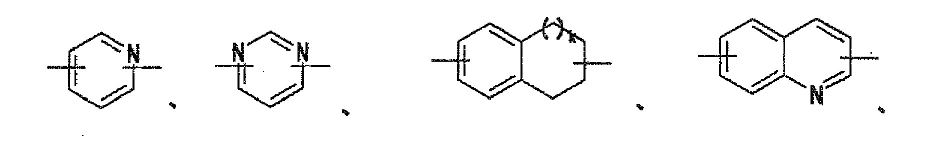 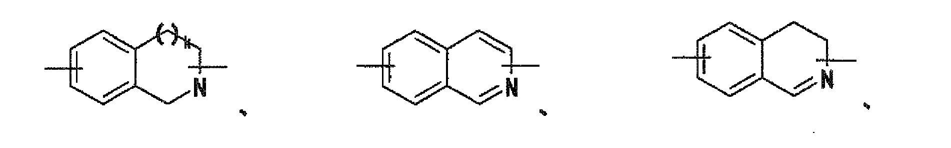 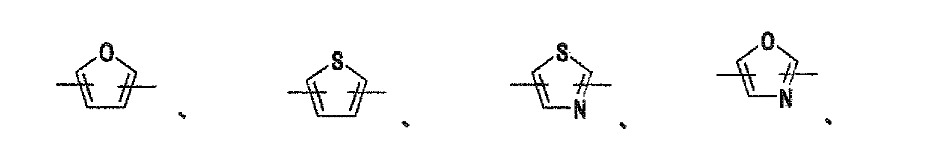 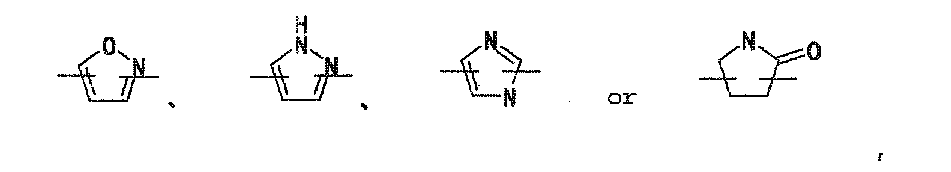 wherein K is 0 or an integer of 1 to 2, or ring B may form, together with R³, R¹⁰ and the nitrogen atom to which R¹⁰ is bonded, 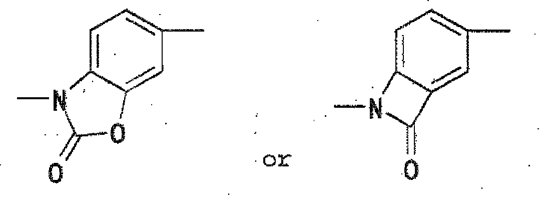
   Alk1¹ is alkandiyl or alkendiyl; Alkl² is alkandiyl or alkendiyl; 1 is 0 or an integer of 1 to 3; m is 0 or an integer of 1 to 3; D is a C₁₋₆ alkyl group, a C₂₋₆ alkenyl group, a C₂₋₇ alkoxycarbonyl group, -N(R⁴²)-CO(R⁴³), wherein R⁴² is a hydrogen atom or a C₁₋₆ alkyl group, and R⁴³ is a C₆₋₁₄ aryl group or a C₇₋₁₆ aralkyl group, or the following formula: 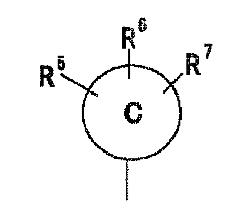 wherein R⁵, R⁶ and R⁷ may be the same or different, and are a hydrogen atom, a C₁₋₆ alkyl group, a C₁₋₆ alkoxy group, a C₂₋₇ alkoxycarbonyl group, a carboxyl group, a halogen atom, a cyano group, a nitro group, a halo C₁₋₆ alkyl group, a C₁₋₆ acyl group, a hydroxyl group, an amino group, a C₆₋₁₄ aryl group which may be substituted or -(CH₂)ᵣ-CON(R¹⁶) (R¹⁷), wherein R¹⁶ and R¹⁷ may be the same or different, and are a hydrogen atom, a C₁₋₆ alkyl group or a halo C₁₋₆ alkyl group, and r is 0 or an integer of 1 to 3; ring C is a C₆₋₁₄ aryl group, a C₇₋₁₅ arylcarbonylamino group, a C₈₋₁₇aralkylcarbonylamino group, a hetero ring residue, a C₃₋₇ cycloalkyl group or a C₇₋₁₆ aralkyl group, or ring C may form, together with R⁷ and R⁸, 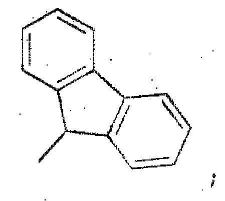 and
   R⁸ and R⁹ may be the same or different, and are a hydrogen atom, a C₁₋₆ alkyl group, a C₆₋₁₄ aryl group which may be substituted, a hydroxy C₁₋₆ alkyl group, -CON(R¹⁸) (R¹⁹), wherein R¹⁸ and R¹⁹ may be the same or different, and are a hydrogen atom, a C₁₋₆ alkyl group, a C₃₋₇ cycloalkyl group, a halo C₁₋₆ alkyl group, a C₂₋₁₂ alkoxyalkyl group or a C₆₋₁₄ aryl group which may be substituted, -COO(R²⁰) or -(CH₂)ₛ-OCO(R²⁰), wherein R²⁰ is a hydrogen atom, a C₁₋₆ alkyl group or a C₃₋₇ cycloalkyl group and s is 0 or an integer of 1 to 3; -N(R²¹) (R²²), wherein R²¹ and R²² may be the same or different, and are a hydrogen atom, a C₁₋₆ alkyl group, a C₁₋₆ acyl group or a C₁₋₆ alkylsulfonyl group or R²¹ and R²² may form, together with the nitrogen atom to which they are bonded, 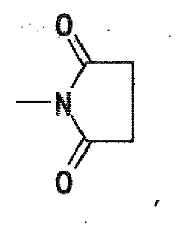
   or R⁸ and R⁹ together may form a C₃₋₇ cycloalkyl group, or a prodrug thereof, or a pharmaceutically acceptable salt thereof.

In addition, the following compounds are described in this patent document as specific compounds.
· 2-(2-{3-methyl-4-[(4^{r}-trifluoromethyl-biphenyl-2-carbonyl)-amino]-phenyl}-acetoxymethyl)-2-phenyl-malonic acid diethyl ester,
· 2-(2-{4-[methyl-(4'-trifluoromethyl-biphenyl-2-carbonyl)- amino]-phenyl}-acetoxymethyl)-2-phenyl-malonic acid diethyl ester,
· 2-phenyl-2-(2-{4-[(4'-trifluoromethyl-biphenyl-2-carbonyl) -amino] -phenyl}-acetoxymethyl) -malonic acid diethyl ester,
· 2-phenyl-2-(2-{4-[(4'-trifluoromethyl-biphenyl-2-carbonyl)-amino]-phenyl}-acetoxymethyl)-malonic acid diisopropylester,
· 2-phenyl-2-(2-{4[(4'-trifluoromethyl-biphenyl-2-carbonyl)-amino]-phenyl}-acetoxymethyl)-malonic acid dimethylester,
· 2-cyclopentyl-2-(2-{4-[(4'-trifluoromethyl-biphenyl-2-carbonyl)-amino]-phenyl}-acetoxymethyl)-malonic acid diethylester,
· 2-phenyl-2-(2-{4-[(4'-trifluoromethyl-biphenyl-2-carbonyl)-amino]-phenyl}-acetoxymethyl)-malonic acid dicyclohexylester,
· 2-benzyl-2-(2-{4-[(4'-trifluoromethyl-biphenyl-2-carbonyl)-amino]-phenyl}-acetoxymethyl)-malonic acid diethyl ester,
· 2-(2-{2-methyl-4-[{4'-trifluoromethyl-biphenyl-2-carbonyl )-amino]-phenyl}-acetoxymethyl)-2-phenyl-malonic aciddiethyl ester,
· 2-cyclohexyl-2-(2-{4-[(4'-trifluoromethyl-biphenyl-2-carbonyl)-amino]-phenyl}-acetoxymethyl)-malonic acid diethylester,
· 2-phenyl-2-(2-{2-trifluoromethyl-4-[(4'-trifluoromethyl-biphenyl-2-carbonyl)-amino]-phenyl}-acetoxymethyl)-malonicacid diethyl ester,
· 2-pyridin-2-yl-2-(2-{4-[(4'-trifluoromethyl-biphenyl-2-carbonyl)-amino]-phenyl}-acetoxymethyl)-malonic acid diethylester,
· 2-pyridin-3-yl-2-(2-{4-[(4'-trifluoromethyl-biphenyl-2-carbonyl)-amino]-phenyl}acetoxymethyl)-malonic acid diethylester,
· 2-phenyl-2-(2-{3-trifluoromethyl-4-[(4'-trifluoromethyl-biphenyl-2-carbonyl)-amino]-phenyl}-acetoxymethyl)-malonicacid diethyl ester,
· 2-(2-{4-[(4'-methyl-biphenyl-2-carbonyl)-amino]-phenyl}-acetoxymethyl)-2-phenyl-malonic acid diethyl ester,
· 2-(2-{4-[(4'-methoxy-biphenyl-2-carbonyl)-amino]-phenyl}-acetoxymethyl)-2-phenyl-malonic acid diethyl ester,
· 2-phenyl-2-(2-{4-[(3'-trifluoromethyl-biphenyl-2-carbonyl) -amino] -phenyl} -acetoxymethyl) -malonic acid diethyl ester,
· 2-(2-{4-[isopropyl-(4'-trifluoromethyl-biphenyl-2-carbonyl)-amino]-phenyl}-acetoxymethyl)-2-phenyl-maloníc acid diethyl ester,
· 2-(2-(4-[cyclohexyl-(4'-trifluoromethyl-biphenyl-2-carbonyl)-amino]-phenyl)-acetoxymethyl)-2-phenyl-malonic acid diethyl ester,
· 2-phenyl-2-(2-{4-[(4'-trifluoromethyl-biphenyl-2-carbonyl)-amino]-phenyl}-acetoxymethyl)-malonic acid dipropylester,
· 2-phenyl-2-(2-{4-[(4'-trifluoromethyl-biphenyl-2-carbonyl)-amino]-phenyl}-acetoxymethyl)-malonic acid diisobutylester,
· 2-(2-{4-[ethyl-(4'-trifluoromethyl-biphenyl-2-carbonyl)-amino]-phenyl}-acetoxymethyl)-2-phenyl-malonic acid diethylester,
· 2-(2-{3-ethyl-4-[(4'-trifluoromethyl-biphenyl-2-carbonyl)-amino]-phenyl}-acetoxymethyl)-2-phenyl-malonic acid diethyl ester,
· 2-(2-{3-isopropyl-4-[(4'-trifluoromethyl-biphenyl-2-carbonyl)-amino]-phenyl}-acetoxymethyl)-2-phenyl-malonic acid diethyl ester,
· 2-(2-{3-isobutyl-4-[(4'-trifluoromethyl-biphenyl-2-carbonyl)-amino]-phenyl}-acetoxymethyl)-2-phenyl-malonic acid diethyl ester,
· 2-(2-{3-chloro-4-[(4'-trifluoromethyl-biphenyl-2-carbonyl)-amino]-phenyl}-acetoxymethyl)-2-phenyl-malonic acid diethyl ester,
· 2-(2-{3-bromo-4-[(4'-trifluoromethyl-biphenyl-2-carbonyl)-amino]-phenyl}-acetoxymethyl)-2-phenyl-malonic-acid diethyl ester,
· 2-(2-{3-dimethylcarbamoyl-4-[(4'-trifluoromethyl-biphenyl-2-carbonyl)-amino]-phenyl}-acetoxymethyl)-2-phenyl-malonic acid diethyl ester,
· 2-(2-{3-diethylcarbamoyl-4-[(4'-trifluoromethyl-biphenyl- 2-carbonyl)-amino]-phenyl}-acetoxymethyl)-2-phenyl-malonic acid diethyl ester,
· 2-(2-{3-diisopropylcarbamoyl-4-[(4'-trifluoromethyl-biphenyl-2-carbonyl)-amino]-phenyl}-acetoxymethyl)-2-phenyl-malonic acid diethyl ester,
· 2-(2-{3-(ethyl-methylcarbamoyl)-4-[(4'-trifluoromethyl-biphenyl-2-carbonyl)-amino]-phenyl}-acetoxymethyl)-2-phenyl-malonic acid diethyl ester,
· {3-(ethyl-methylcarbamoyl)-4-[(4'-trifluoromethyl-biphenyl-2-carbonyl)-amino]-phenyl}-acetic acid2,2-bis-ethylcarbamoyl-2-phenyl-ethyl ester,
· {3-(pyrrolidine-1-carbonyl)-4-[(4'-trifluoromethyl-biphenyl-2-carbonyl)-amino]-phenyl}-acetic acid2,2-bis-ethylcarbamoyl-2-phenyl-ethyl ester,
· 2-phenyl-2-(2-{3-(pyrrolidine-1-carbonyl)-4-[(4'-trifluoromethyl-biphenyl-2-carbonyl)-amino]-phenyl}-acetoxymethyl)-malonic acid diethyl ester,
· 2-phenyl-2-(2-{3-(piperidine-1-carbonyl)-4-[(4'-trifluoromethyl-biphenyl-2-carbonyl)-amino]-phenyl}-acetoxymethyl)-malonic acid diethyl ester,
· 2-[2-(2-{3-dimethylcarbamoyl-4-[(4'-trifluoromethyl-biphenyl-2-carbonyl)-amino]-phenyl}-acetoxy)-ethyl]-2-phenyl-malonic acid diethyl ester,
· 2-(2-{3-dimethylcarbamoyl-4-[(4'-fluoro-biphenyl-2-carbonyl)-amino]-phenyl}-acetoxymethyl)-2-phenyl-malonic acid diethyl ester,
· 2-(2-{4-[(4'-bromo-biphenyl-2-carbonyl)-amino]-3-dimethyl carbamoyl-phenyl}-acetoxymethyl)-2-phenyl-malonic acid diethyl ester,
· 2-(2-{3-dimethylcarbamoyl-4-[(4'-trifluoromethyl-biphenyl-2-carbonyl)-amino]-phenyl}-acetoxymethy)-2-phenyl-malonic acid dimethyl ester,
· 2-cyclopentyl-2-(2-{3-dimethylcarbamoyl-4-[(4'-trifluoromethyl-biphenyl-2-carbonyl)-amino]-phenyl}-acetoxymethyl)-malonic acid diethyl ester,
· 2-cyclohexyl-2-(2-{3-dimethylcarbamoyl-4-[(4'-trifluoromethyl-biphenyl-2-carbonyl)-amino]-phenyl}-acetoxymethyl)-malonic acid diethyl ester,
· 2-(2-{4-[(4'-chloro-biphenyl-2-carbonyl)-amino]-3-dimethy lcarbamoyl-phenyl}-acetoxymethyl)-2-phenyl-malonic acid diethyl ester,
· 2-(2-{4-[(4'-acetyl-biphenyl-2-carbonyl)-amino]-3-dimethylcarbamoyl-phenyl}-acetoxymethyl)-2-phenyl-malonic acid diethyl ester,
· 2-(2-{4-[(4'-cyano-biphenyl-2-carbonyl)-amino]-3-dimethylcarbamoyl-phenyl}-acetoxymethyl)-2-phenyl-malonic acid diethyl ester,
· 2-(2-{3-dimethylcarbamoyl-4-[(4-methyl-4'-trifluoromethyl-biphenyl-2-carbonyl)-amino]-phenyl}-acetoxymethyl)-2-phenyl-malonic acid diethyl ester,
· 2-(2-{3-dimethylcarbamoyl-4-[(5-methyl-4'-trifluoromethyl-biphenyl-2-carbonyl)-amino]-phenyl}-acetoxymethyl)-2-phenyl-malonic acid diethyl ester,
· 2-[3-(2-{3-dimethylcarbamoyl-4-[(4'-trifluoromethyl-biphenyl-2-carbonyl)-amino]-phenyl}-acetoxy)-propyl]-2-phenyl-malonic acid diethyl ester,
· 2-(2-{3-dimethylcarbamoyl-4-[(5-methoxy-4'-trifluoromethyl-biphenyl-2-carbonyl)-amino]-phenyl}-acetoxymethyl)-2-phenyl-malonic acid diethyl ester,
· 2-(2-{4-[(5-chloro-4'-trifluoromethyl-biphenyl-2-carbonyl)-amino]-3-dimethylcarbamoyl-phenyl}-acetoxymethyl)-2-phenyl-malonic acid diethyl ester,
· 2-(2-{3-dimethylcarbamoyl-4-[(6-methyl-4-trifluoromethyl-biphenyl-2-carbonyl)-amino]-phenyl}-acetoxymethyl)-2-phenyl-ma.lonic acid diethyl ester,
· 2-(2-{3-dimethylcarbamoyl-4-[(4'-trifluoromethyl-biphenyl-2-carbonyl)-amino]-phenyl}-acetoxymethyl)-2-phenyl-malonic acid di-2,2,2-trifluoroethyl ester,
· 2-(2-{3-dimethylcarbamoyl-4-[(2'-fluoro-4'-trifluoromethyl-biphenyl-2-carbonyl)-amino]-phenyl}-acetoxymethyl)-2-phenyl-malanic acid diethyl ester,
· 2-(2-{5-dimethylcarbamoyl-2-fluoro-4-[(4'-tri-fluoromethyl-biphenyl-2-carbonyl)-amino]-phenyl}-acetoxymethyl)-2-phenyl-malonic acid diethyl ester,
· 2-(2-{3-bromo-5-dimethylcarbamoyl-4-[(4'-trifluoromethyl-biphenyl-2-carbonyl)-amino]-phenyl}-acetoxymethyl)-2-phenyl-malonic acid diethyl ester,
· 2-(2-{3-chloro-5-dimethylcarbamoyl-4-[{4'-trifluoromethyl -biphenyl-2-carbonyl)-amino]-phenyl}-acetoxymethyl)-2-phenyl-malonic acid diethyl ester,
· 2-(2-{3-dimethylcarbamoyl-4-[(3'-fluoro-4'-trifluoromethyl-biphenyl-2-carbonyl)-amino]-phenyl}-acetoxymethyl)-2-phenyl-malonic acid diethyl ester,
· 2-(2-{4-[(3'-chloro-4'-trifluoromethyl-biphenyl-2-carbonyl)-amino]-3-dimethylcarbamoyl-phenyl}-acetoxymethyl)-2-phenyl-malonic acid diethyl ester,
· 2-(2-{3-dimethylcarbamoyl-4-[(4'-trifluoromethyl-biphenyl-2-carbonyl)-amino]-phenyl}-acetoxymethyl)-2-(5-nitro-pyridin-2-yl)-malonic acid diethyl ester,
· 2-(5-amino-pyridin-2-yl)-2-(2-{3-dimethylcarbamoyl-4-[(4'-trifluoromethyl-biphenyl-2-carbonyl)-amino]-phenyl}-acetoxymethyl)-malonic acid diethyl ester,
· 2-(2-{3-dimethylcarbamoyl-4-[(4'-trifluoromethyl-biphenyl-2-carbonyl)-amino]-phenyl}-acetoxymethyl)-2-pyridin-2-yl-malonic acid diethyl ester,
· 2-(2-{3-chloro-5-dimethylcarbamoyl-2-fluoro-4-[(4'-trifluoromethyl-biphenyl-2-carbortyl)-amino]-phenyl}-acetoxymethyl)-2-phenyl-malonic acid diethyl ester,
· 2-(2-{3-bromo-5-dimethylcarbamoyl-2-fluoro-4-[(4'-trifluoromethyl-biphenyl-2-carbonyl)-amino]-phanyl}-acetoxymethyl)-2-phenyl-malonic acid diethyl ester,
· 2-(2-{3-dimethylcarbamoyl-4-[(4'-trifluoromethyl-biphenyl-2-carbonyl)-amino]-phenyl}-acetoxymethyl)-2-o-tolyl-malonic acid diethyl ester,
· 2-(2-{3-dimethylcarbamoyl-4-[(4'-trifluoromethyl-biphenyl-2-carbonyl)-amino]-phenyl}-acetoxymethyl)-2-m-tolyl-malonic acid diethyl ester,
· 2-(2-{3-dimethylcarbamoyl-4-[(4'-trifluoromethyl-biphenyl-2-carbonyl)-amino]-phenyl}-acetoxymethyl)-2-p-tolyl-malonic acid diethyl ester,
· 2-(2-chloro-phenyl)-2-(2-{3-dimethylcarbamoyl-4-[(4'-trifluoromethyl-biphenyl-2-carbonyl)-amino]-phenyl}-acetoxymethyl)-malonic acid diethyl ester,
· 2-(3-chloro-phenyl)-2-(2-{3-dimethylcarbamoyl-4-[(4'-trifluoromethyl-biphenyl-2-carbonyl)-amino]-phenyl}-acetoxymethyl)-malonic acid diethyl ester,
· 2-(4-chloro-phenyl)-2-(2-{3-dimethylcarbamoyl-4-[4'-trif luoromethyl-biphenyl-2-carbonyl)-amino]-phenyl}-acetoxymethyl)-malonic acid diethyl ester,
· 2-(2-{3-dimethylcarbamoyl-4-[(4'-trifluoromethyl-biphenyl-2-carbonyl)-amino]-phenyl}-acetoxymethyl)-2-phenyl-succinic acid diethyl ester,
· 2-(2-{3-dimethylcarbamoyl-4-[(4'-trifluoromethyl-biphenyl-2-carbonyl)-amino]-phenyl}-acetoxy-methyl)-2-(2-methoxy-phenyl)-malonic acid diethyl ester,
· 2-(2-{3-dimethylcarbamoyl-4-[(4'-trifluoromethyl-biphenyl-2-carbonyl)-amino]-phenyl}-acetoxymethyl)-2-(3-methoxy-phenyl)-malonic acid diethyl ester,
· 2-(2-{3-dimethylcarbamoyl-4-[(4'-trifluoromethyl-biphenyl-2-carbonyl)-amino]-phenyl}-acetoxymethyl)-2-(4-methoxy-phenyl)-malonic acid diethyl ester,
· 2-(2-{4-[(5,4'-bis-trifluoromethyl-biphenyl-2-carbonyl)-amino]-3-dimethylcarbamoyl-phenyl}-acetoxymethyl)-2-phenyl-malonic acid diethyl ester,
· 2-(2-{4-[(6-chloro-4'-trifluoromethyl-biphenyl-2-carbonyl)-amino]-3-dimethylcarbamoyl-phenyl}-acetoxymethyl)-2-phenyl-malonic acid diethyl ester,
· 2-{2-{3-dimethylcarbamoyl-4-[(6-fluoro-4'-trifluoromethyl-biphenyl-2-carbonyl)-amino]-phenyl}-acetoxymethyl)-2-phenyl-malonic acid diethyl ester,
· 2-[2-(2-{3-dimethylcarbamoyl-4-[(5-methyl-4'-trifluoromethyl-biphenyl-2-carbonyl)-amino]-phenyl}-acetoxy)-ethyl]-2-phenyl-malonic acid diethyl ester,
· 2-(2-{3-dimethylcarbamoyl-4-[(5-ethoxy-4'-trifluoromethyl-biphenyl-2-carbonyl)-amino]-phenyl}-acetoxymethyl)-2-phenyl-malonic acid diethyl ester,
· 2-(2-{3-dimethylcarbamoyl-4-[(5-isopropoxy-4'-trifluoromethyl-biphenyl-2-carbonyl)-amino]-phenyl}-acetoxymethyl)-2-phenyl-malonic acid diethyl ester,
· 2-[2-(2-{4-[(5,4'-bis-trifluoromethyl-biphenyl-2-carbonyl)-amino]-3-dimethylcarbamoyl-phenyl}-acetoxy)-ethyl]-2-phenyl-malonic acid diethyl ester,
· 2-(2-{3-dimethylcarbamoyl-4-[-(6-methoxy-4'-trifluoromethyl-biphenyl-2-carbonyl)-amino]-phenyl}-acetoxymethyl)-2-phenyl-malonic acid diethyl ester,
· 2-(2-{3-dimethylcarbamoyl-4-[(3-methyl-4'-trifluoromethyl -biphenyl-2-carbonyl)-amino]-phenyl}-acetoxymethyl)-2-phenyl-malonic acid diethyl ester,
· 2-{2-[4-(2,4-bis-trifluoromethyl-benzoylamino)-3-dimethylcarbamoyl-phenyl]-acetoxymethyl}-2-phenyl-malonic acid diethyl ester,
· 2-(2-{3-dimethylcarbamoyl-4-[(4'-methyl-biphenyl-2-carbonyl)-amino]-phenyl}-acetoxymethyl)-2-phenyl-malonic acid diethyl ester,
· 2-{2-[3-dimethylcarbamoyl-4-(2-ethyl-4-trifluoromethyl-benzoylamino)-phenyl]-acetoxymethyl}-2-phenyl-malonic acid diethyl ester,
· 2-(2-{3-dimethylcarbamoyl-4-[(4'-ethyl-biphenyl-2-carbonyl)-amino]-phenyl}-acetoxymethyl)-2-phenyl-malonic acid diethyl ester,
· 2-(2-{3-dimethylcarbamoyl-4-[(4'-isopropenyl-biphenyl-2-carbonyl)-amino]-phenyl}-acetoxymethyl)-2-phenyl-malonicacid diethyl ester,
· 2-(2-{3-dimethylcarbamcyl-4-[(4'-isopropyl-biphenyl-2-carbonyl)-amino]-phenyl}-acetoxymethyl)-2-phenyl-malonicacid diethyl ester,
· 2-(2-{3-dimethylcarbamoyl-4-[(4'-trifluoromethyl-biphenyl-2-carbonyl)-amino]-phenyl}-acetoxyrnethyl)-2-thiophen-2-y1-malonic acid diethyl ester,
· 2-(2-{3-dimethylcarbamoyl-4-[(4'-trifluoromethyl-biphenyl-2-carbonyl)-amino]-phenyl}-acetoxymethyl)-2-thiophen-3-y1-malonic acid diethyl ester,
· 2-(2-{4-dimethylcarbamdyl-5-[(4'-trifluoromethyl-biphenyl-2-carbonyl)-amino]-pyridin-2-yl}-acetoxymethyl)-2-phenyl-malonic acid diethyl ester,
· 2-(2-{3-dimethylcarbamoyl-4-[(4'-trifluoromethyl-biphenyl-2-carbonyl)-amino]-phenyl}-acetoxymethyl)-2-(3-methyl-thiophen-2-yl)-malonic acid diethyl ester,
· 2-(2-{3-dimethylcarbamoyl-4-[(4'-trifluoromethyl-biphenyl-2-carbonyl)-amino]-phenyl}-acetoxymethyl)-2-(5-methyl-thiophen-2-yl)-malonic acid diethyl ester,
· 2-(2-{3-dimethylcarbamoyl-4-[(4'-trifluoromethyl-biphenyl-2-carbonyl)-amino]-phenyl}-acetoxymethyl)-2-thiazol-2-yl-malonic acid diethyl ester,
· 2-(2-{3-ethoxy-4-[(4'-trifluoromethyl-biphenyl-2-carbonyl )-amino]-phenyl}-acetoxymethyl)-2-phenyl-malonic acid diethyl ester,
· 2-(2-{3-methoxy-4-[(4'-trifluoromethyl-biphenyl-2-carbonyl)-amino]-phenyl}-acetoxymethyl)-2-phenyl-malonic aciddiethyl ester,
· 2-(2-{3-isopropoxy-4-[(4'-trifluoromethyl-biphenyl-2-carbonyl)-amino]-phenyl}-acetoxymethyl)-2-phenyl-malonic acid diethyl ester,
· 2-(2-{3-benzyloxy-4[(4'-trifluoromethyl-biphenyl-2-carbonyl)-amino]-phenyl}-acetoxymethyl)-2-phenyl-malonic acid diethyl ester,
· 2-(2-{3-hydroxy-4-[(4'-trifluoromethyl-biphenyl-2-carbonyl)-amino]-phenyl}-acetoxymethyl)-2-phenyl-malonic acid diethyl ester,
· 2-phenyl-2-(2-{3-piperidin-1-yl-4-1(4'-trifluoromethyl-biphenyl-2-carbonyl)-amino]-phenyl}-acetoxymethyl)-malonicacid diethyl ester,
· 2-phenyl-2-(2-{3-pyrrolidin-1-yl-4-[(4'-trifluoromethyl-biphenyl-2-carbonyl)-amino]-phenyl}-acetoxymethyl)-malonicacid diethyl ester,
· 2-(2-{3-dimethylamino-4-[(4'-trifluoromethyl-biphenyl-2-carbonyl)-amino]-phenyl}-acetoxymethyl)-2-phenyl-malonicacid diethyl ester,
· 2-(2-{3-morpholin-4-yl-4-[(4'-trifluoromethyl-biphenyl-2-carbonyl)-amino]-phenyl}-acetoxymethyl)-2-phenyl-malonicacid diethyl ester,
· 2-(2-{3-diethylamino-4-[(4'-trifluoromethyl-biphenyl-2-carbonyl)-amino]-phenyl}-acetoxymethyl)-2-phenyl-malonicacid diethyl ester,
· 2-(2-{2-chloro-4-[(4'-trifluoromethyl-biphenyl-2-carbonyl)-amino]-bensoyloxy}-ethyl)-2-phenyl-malonic acid diethylester,
· 2-(2-{3-ethoxycarbonyl-4-[(4'-trifluoromethyl-biphehyl-2-carbonyl)-amino]-phenyl}-acetoxymethyl)-2-phenyl-malonicacid diethyl ester,
· 2-(3-{3-dimethylcarbamoyl-4-[(4'-trifluoromethyl-biphenyl-2-carbonyl)-amino]-phenyl}-propionyloxymethyl)-2-phenyl-malonic acid diethyl ester,
· 2-(2-{3-benzyloxycarbonyl-4-[(4'-trifluoromethyl-biphenyl -2-carbonyl)-amino]-phenyl}-acetoxymethyl)-2-phenyl-malonic acid diethyl ester,
· 2-(2-{3-carboxy-4-[(4'-trifluoromethyl-biphenyl-2-carbonyl)-amino]-phenyl}-acetoxymethyl)-2-phenyl-malonic acid diethyl ester,
· 2-(2-{3-isopropoxycarbonyl-4-[(4'-trifluoromethyl-biphenyl-2-carbonyl)-amino]-phenyl}-acetoxymethyl)-2-phenyl-malonic acid diethyl ester,
· 2-(2-{3-methoxycarbonyl-4-[(4'-trifluoromethyl-biphenyl-2-carbonyl)-amino]-phenyl}-acetoxymethyl)-2-phenyl-malonicacid diethyl ester,
· 2-(2-{3-acetylamino-4-[(4'-trifluoromethyl-biphenyl-2-carbonyl)-amino]-phenyl}-acetoxymethyl)-2-phenyl-malonicacid diethyl ester,
· 2-(2-{3-methoxycarbonylamino-4-[(4'-trifluoromethyl-biphenyl-2-carbonyl)-amino]-phenyl}-acetoxymethyl)-2-phenyl-malonic acid diethyl ester,
· 2-(2-{3-(4-methyl-thiazol-2-yl)-4-[(4'-trifluoromethyl-biphenyl-2-carbonyl)-amino]-phenyl}-acetoxymethyl)-2-phenyl-malonic acid diethyl ester,
· 2-phenyl-2-(2-{6-[(4'-trifluoromethyl-biphenyl-2-carbonyl)-amino]-biphenyl-3-yl}-acetoxymethyl)-malonic acid diethyl ester,
· 2-(2-{3-formyl-4-[(4'-trifluoromethyl-biphenyl-2-carbonyl)-amino]-phenyl}-acetoxymethyl)-2-phenyl-malonic acid diethyl ester,
· 2-(2-{3-dimethylaminomethyl-4-[(4'-trifluoromethyl-biphenyl-2-carbonyl)-amino]-phenyl}-acetoxymethyl)-2-phenyl-malonic acid diethyl ester,
· 2-(2-{3-(methoxy-methylcarbamoyl)-4-[(4'-trifluoromethyl-biphenyl-2-carbonyl)-amino]-phenyl}-acetoxymethyl)-2-phenyl-malonic acid diethyl ester,
· 2-(2-{3-isobutylyl-4-[4'-trifluoromethyl-biphenyl-2-carbonyl)-amino]-phenyl}-acetoxymethyl)-2-phenyl-malonic acid diethyl ester and
· 2-(2-{3-(1-hydroxy-2-methyl-propyl)-4-[(4'-trifluoromethyl-biphenyl-2-carbonyl)-amino]-phenyl}-acetoxymethyl)-2-phenyl-malonic acid diethyl ester.

Among the above-mentioned compound group, particularly representative biphenylamide derivatives are as follows.
· 2-phenyl-2-(2-{4-[(4'-trifluoromethyl-biphenyl-2-carbonyl)-amino]-phenyl}-acetoxymethyl)-malonic acid diisobutylester,
· 2-(2-{4-[ethyl-(4'-trifluoromethyl-biphenyl-2-carbonyl)-amino]-phenyl}-acetoxymethyl) -2-phenyl-malonic acid diethylester,
· 2-(2-{3-dimethylcarbamoyl-4-[(4'-trifluoromethyl-biphenyl-2-carbonyl)-amino]-phenyl}-acetoxymethyl)-2-phenyl-malonic acid diethyl ester,
· 2-(2-{3-dimethylcarbamoyl-4-[(4'-trifluoromethyl-biphenyl-2-carbonyl)-amino]-phenyl}-acetoxymethyl)-2-phenyl-malonic acid dimethyl ester,
· 2-cyclopentyl-2-(2-{3-dimethylcarbamoyl-4-[(4'-trifluoromethyl-biphenyl-2-carbonyl)-amino]-phenyl}-acetoxymethyl)-malonic acid diethyl ester,
· 2-cyclohexyl-2-(2-{3-dimethylcarbamoyl-4-[(4'-trifluoromethyl-biphenyl-2-carbonyl)-amino]-phenyl}-acetoxymethyl)-malonic acid diethyl ester,
· 2-(2-{3-dimethylcarbamoyl-4-[(4-methyl-4'-trifluoromethyl -biphenyl-2-carbonyl)-amino]-phenyl}-acetoxymethyl)-2-phenyl-malonic acid diethyl ester,
· 2-(2-{3-dimethylcarbamoyl-4-[(5-methyl-4'-trifluoromethyl-biphenyl-2-carbonyl)-amino]-phenyl}-acetoxymethyl)-2-phenyl-malonic acid diethyl ester,
· 2-[3-(2-{3-dimethylcarbamoyl-4-[(4'-trifluoromethyl-biphenyl-2-carbonyl)-amino]-phenyl}-acetoxy)-propyl]-2-phenyl-malonic acid diethyl ester,
· 2-(2-{3-dimethylcarbamoyl-4-[(5-methoxy-4'-trifluoromethyl-biphenyl-2-carbonyl)-amino]-phenyl}-acetoxymethyl)-2-phenyl-malonic acid diethyl ester,
· 2-(2-{4-[(5-chloro-4'-trifluoromethyl-biphenyl-2-carbonyl)-amino]-3-dimethylcarbamoyl-phenyl}-acetoxymethyl)-2-phenyl-malonic acid diethyl ester,
· 2-(2-{3-dimethylcarbamoyl-4-[(6-methyl-4'-trifluoromethyl-biphenyl-2-carbonyl)-amino]-phenyl}-acetoxymethyl)-2-phenyl-malonic acid diethyl ester,
· 2-(2-{3-dimethylcarbamoyl-4-[(4'-trifluoromethyl-biphenyl-2-carbonyl)-amino]-phenyl}-acetoxymethyl)-2-phenyl-malonic acid di-2,2,2-trifluoroethyl ester,
· 2-(2-{3-dimethylcarbamoyl-4-[(2'-fluoro-4'-trifluoromethyl-biphenyl-2-carbonyl)-amino]-phenyl}-acetoxymethyl)-2-phenyl-malonic acid diethyl ester,
· 2-(2-{5-dimethylcarbamoyl-2-fluoro-4-[(4'-trifluoromethyl-biphenyl-2-carbonyl)-amino]-phenyl}-acetoxymethyl)-2-phenyl-malonic acid diethyl ester,
· 2-(2-{3-bromo-5-dimethylcarbamoyl-4-[(4'-trifluoromethyl-biphenyl-2-carbonyl)-amino]-phenyl}-acetoxymethyl)-2-phenyl-malonic acid diethyl ester,
· 2-(2-{3-chloro-5-dimethylcarbamoyl-4-[(4'-trifluoromethyl-biphenyl-2-carbonyl)-amino]-phenyl}-acetoxymethyl)-2-phenyl-malonic acid diethyl ester,
· 2-(2-{3-dimethylcarbamoyl-4-[(3'-fluoro-4'-trifluoromethyl-biphenyl-2-carbonyl)-amino]-phenyl}-acetoxymethyl)-2-phenyl-malonic acid diethyl ester,
· 2-(2-{4-[(3'-chloro-4'-trifluoromethyl-bipheny-1-2-carbonyl) -amino]-3-dimethylcarbamoyl-phenyl}-acetoxymethyl)-2-phenyl-malonic acid diethyl ester,
· 2-(2-(3-dimethylcarbamoyl-4-[(4'-trifluoromethyl-biphenyl -2-carbonyl)-amino]-phenyl}-acetoxymethyl)-2-(5-nitro-pyridin-2-yl)-malonic acid diethyl ester,
· 2-(5-amino-pyridin-2-yl)-2-(2-{3-dimethylcarbamoyl-4-[(4'-trifluoromethyl-biphenyl-2-carbonyl)-amino]-phenyl}-acetoxymethyl)-malonic acid diethyl ester,
· 2-(2-{3-dimethylcarbamoyl-4-[(4'-trifluoromethyl-biphenyl-2-carbonyl)-amino]-phenyl}-acetoxymethyl)-2-pyridin-2-yl-malonic acid-diethyl ester,
· 2-(2-{3-chloro-5-dimethylcarbamoyl-2-fluoro-4-[(4'-trifluoromethyl-biphenyl-2-carbonyl)-amino]-phenyl}-acetoxymethyl)-2-phenyl-malonic acid diethyl ester,
· 2-(2-{3-bromo-5-dimethylcarbamoyl-2-fluoro-4-[(4'-trifluoromethyl-biphenyl-2-carbonyl)-amino]-phenyl}-acetoxymethyl)-2-phenyl-malonic acid diethyl ester,
· 2-(2-{3-dimethylcarbamoyl-4-[(4'-trifluoromethyl-biphenyl-2-carbonyl)-amino]-phenyl}-acetoxymethyl)-2-o-tolyl-malonic acid diethyl ester,
· 2-(2-{3-dimethylcarbamoyl-4-[(4'-trifluoromethyl-biphenyl-2-carbonyl)-amino]-phenyl}-acetoxymethyl)-2-m-tolyl-malonic acid diethyl ester,
· 2-(2-{3-dimethylcarbamoyl-4-[(4'-trifluoromethyl-biphenyl-2-carbonyl)-amino]-phenyl}-acetoxymethyl)-2-p-tolyl-malonic acid diethyl ester,
· 2-(2-chloro-phenyl)-2-(2-{3-dimethylcarbamoyl-4-[(4'-trifluoromethyl-biphenyl-2-carbonyl)-amino]-phenyl}-acetoxymethyl)-malonic acid diethyl ester,
· 2-(3-ehloro-phenyl)-2-(2-{3-dimethylcarbamoyl-4-[(4'-trifluoromethyl-biphenyl-2-carbonyl)-amino]-phenyl}-acetoxymethyl)-malonic acid diethyl ester,
· 2-(4-chloro-phenyl)-2-(2-{3-dimethylcarbamoyl-4-[(4'-trifluoromethyl-biphenyl-2-carbonyl)-amino]-phenyl}-acetoxymethyl)-malonic acid diethyl ester,
· 2-(2-{3-dimethylcarbamoyl-4-[(4'-trifluoromethyl-biphenyl-2-carbonyl)-amino]-phenyl}-acetoxymethyl)-2-phenyl-succinic acid diethyl ester,
· 2-(2-{3-dimethylcarbamoyl-4-[(4'-trifluoromethyl-biphenyl-2-carbonyl)-amino]-phenyl}-acetoxymethyl)-2-(2-methoxy-phenyl)-malonic acid diethyl ester,
· 2-(2-{3-dimethylcarbamoyl-4-[(4'-trifluoromethyl-biphenyl -2-carbonyl)-amino]-phenyl}-acetoxymethyl)-2-(3-methoxy-phenyl)-malonic acid diethyl ester,
· 2-(2-{-dimethylcarbamoyl-4-[(-4'-trifluaramethyl-biphenyl-2-carbonyl)-amino]-phenyl}-acetoxymethyl)-2-(4-methoxy-phenyl)-malonic acid diethyl ester,
· 2-(2-{4-[(5,4'-bis-trifluoromethyl-biphenyl-2-carbonyl)-amino]-3-dimethylcarbamoyl-phenyl}-acetoxymethyl)-2-phenyl-malonic acid diethyl ester,
· 2-(2-{4-[(6-chloro-4'-trifluoromethyl-biphenyl-2-carbonyl)-amino]-3-dimethylcarbamoyl-phenyl}-acetoxymethyl)-2-phenyl-malonic acid diethyl ester,
· 2-(2-{3-dimethylcarbamoyl-4-[(6-fluoro-4'-triflucromethyl-biphenyl-2-carbonyl)-amino]-phenyl}-acetoxymethyl)-2-phenyl-malonic acid diethyl ester,
· 2-[2-(2-{3-dimethylcarbamoyl-4-[(5-methyl-4'-trifluoromethyl-biphenyl-2-carbonyl)-amino]-phenyl}-acetoxy)-ethyl]-2-phenyl-malonic acid diethyl ester,
· 2-(2-{3-dimethylcarbamoyl-4-[(5-ethoxy-4'-trifluoromethyl-biphenyl-2-carbonyl)-amino]-phenyl}-acetoxymethyl)-2-phenyl-malonic acid diethyl ester,
· 2-(2-{3-dimethylcarbamoyl-4-[(5-isopropoxy-4'-trifluoromethyl-biphenyl-2-carbonyl)-amino]-phenyl}-acetoxymethyl)-2-phenyl-malonic acid diethyl ester,
· 2-[2-(2-{4-[(5,4'-bis-trifluoromethyl-biphenyl-2-carbonyl)-amino]-3-dimethylcarbamoyl-phenyl}-acetoxy)-ethyl]-2-phenyl-malonic acid diethyl ester,
· 2-(2-{3-dimethylcarbamoyl-4-[(6-methoxy-4'-trifluoromethyl-biphenyl-2-carbonyl)-amino]-phenyl}-acetoxymethyl)-2-phexayl-malonic acid, diethyl ester and
· 2-(2-{3-dimethylcarbamoyl-4-[(3-methyl-4-trifluoromethyl-biphenyl-2-carbonyl)-amino]-phenyl}-acetoxymethyl)-2-phenyl-malonic acid diethyl ester.

Patent Document 1: Japanese Patent Laid-Open No. 2000-281561
Patent Document 2: Japanese Patent Laid-Open No. 9-309834
Patent Document 3: Japanese Patent Laid-Open No. 2004-10575
Patent Document 4 : National Publication of International Patent Application No. 11-503238
Patent Document 5: Japanese Patent Laid-Open No. 2003-73261
Patent Document 6: Japanese Patent Publication No. 3-1288
Patent Document 7: Japanese Patent Publication No. 3-28404
Patent Document 8: Japanese Patent Laid-Open No. 11-246404
Patent Document 9: National Publication of International Patent Application No. 2003-531099
Patent Document 10: WO 00/056726
Patent Document 11: Japanese Patent Laid-Open No. 08-208476
Patent Document 12: WO 2002/051385
Patent Document 13: Japanese Patent Laid-Opeh No. 9-59159
Patent Document 14: Japanese Patent Laid-Open Publication No. 11-228569
Patent Document 15: National Publication of International Patent Application No. 2004-514676 (WO 2002/042291)
Patent Document 16: National Publication of International Patent Application No. 2003-535900 (WO 01/097810)
Patent Document 17: National Publication of International Patent Application No. 2003-521484 (WO 01/047898)
Patent Document 18: National Publication of International Patent. Application No. 2003-520270 (WO 01/053260)
Patent Document 19: National Publication of International Patent Application No. 2003-519131 (WO 01/047899)
Patent Document 20: National Publication of International Patent Application No. 2003-509505 (WO 01/021604)
Patent Document 21: National Publication of International Patent Application No. 2003-505373 (WO 01/005762)
Patent Document 22: National Publication of International Patent Application No. 2000-510483 (WO 98/47875)
Patent Document 23: Japanese Patent Laid-Open No. 2001-172180
Patent Document 24: Japanese Patent Laid-Open No. 2000-169395
Patent Document 25: Japanese Patent Laid-Open No. 2002-220345
Patent Document 26: Japanese Patent Laid-Open No. 2003-321424
Non-Patent Document 1: W. L. Chiou and S. Riegelman: pharmaceutical application of solid dispersion systems, J. Pharm. Sci. (vol. 60, 1281-1302, 1971)
Non-parent Document 2: Wetterau and Zilversmit, Chem. Phys. Lipids (38, pp. 205-222, 1985)

### Disclosure of the Invention

As for a pharmaceutical product, sufficient effectiveness is demanded from the original purpose of a pharmaceutical product, and, in addition, high stability is demanded from the viewpoint of safety. With regard to effectiveness or drug efficacy, it is important to design sufficiently high bioavailability in the design of a solid pharmaceutical formulation, in particular an oral formulation. Factors affecting bioavailability include water solubility, drug absorbency in the whole alimentary canal , dosage intensity and first-pass effect as mentioned above, and since it is known that the dissolution rate is the rate-determining step of absorption particularly in pharmaceutical agents poorly soluble in water, solubility in water is the most important and dominant factor in the development of pharmaceutical products.

Unfortunately, however, many chemical substances for pharmaceutical products are poorly soluble in water and only very little of the dosage is transferred and absorbed in the blood and thus they have a defect that the bioavailability is extremely low.

The same holds true with MTP inhibiting substances. Besides, a of the conventional MTP inhibiting substances have defects that they do not selectively act in the intestinum tenue but also act on the liver, which is suspected of causing side effects such as fatty liver.

In addition, with regard to the production method of the solid dispersion, when the solid dispersion method is adopted, the solid dispersion may be in the form a film depending on the production conditions as mentioned above and a step of finely cutting this is necessary, and when the fusion method is adopted, the obtained solid dispersion is in the form of pellets and a further working such as milling is necessary. And when the spray drying method is adopted, a large amount of solvent is necessary and the obtained granules are bulky since the inside thereof is hollow, which leads to unfavorable handling properties and results in the difference in the specific gravity from the other blended agent and therefore the method has defects that uniformity upon punching cannot be maintained and so on.

Therefore, an object of the present invention is to provide a new solid formulation having improved solubility, improved bioavailability and highly excellent stability by applying device in formulation to a chemical substance poorly soluble in water (for example, lipid absorption inhibitor like MTP inhibitor) as a pharmaceutically active ingredient, and an effective production method thereof.

The present inventors have performed intensive studies mainly in an aspect of preparing a formulation in order to provide a pharmaceutical product, for example, an MTP inhibitor, having an excellent, dissolution characteristic, that is a high bioavailability, and also an excellent stability. As a result, the present inventors have found a solid formulation obtained by dissolving a substance poorly soluble in water (for example, MTP inhibitor) as an active ingredient and a water-soluble polymer substance such as polyvinylpyrrolidone or hydroxypropylcellulose in an organic solvent, mixing and stirring this solution and an inorganic porous substance followed by granulation working and drying has an outstanding solubility and an excellent stability and completed the present invention.

Specifically, the present invention is as follows.
1. A solid formulation having improved water-solubility which comprises a chemical substance poorly soluble in water as a pharmaceutically active ingredient, a water-soluble polymer substance and an inorganic porous substance,
   characterized in that
   (1) the chemical substance poorly soluble in water forms a solid dispersion together with the water-soluble polymer substance,
   (2) the water-soluble polymer substance forming the solid dispersion is in the form of fine pieces, and
   (3) the water-soluble polymer substance in the form of fine pieces is maintained to be adsorbed on and/or adhered to the surface of the inorganic porous substance.
2. The solid formulation according to the above-mentioned 1, wherein the water-soluble polymer substance is hydroxypropylcellulose, hydroxypropylmethylcellulose, polyvinylpyrrolidone, polyvinylalcohol, copolyvidone, agar, dextrin, gelatin or a mixture thereof.
3. The solid formulation according to the above-mentioned 2, wherein the water-soluble polymer substance is polyvinylpyrrolidone or hydroxypropylcellulose.
4. The solid formulation according to any one of the above-mentioned 1 to 3, wherein the weight ratio of the water-soluble polymer substance to the chemical substance poorly soluble in water as apharmaceuticallyactive ingredient is 3 to 100.
5. The solid formulation according to the above-mentioned 4, wherein the water-soluble polymer substance is polyvinylpyrrolidone, and the weight ratio of polyvinylpyrrolidone to the chemical substance poorly soluble in water as a pharmaceutically active ingredient is 3 to 50.
6. The solid formulation according to the above-mentioned 4, wherein the water-soluble polymer substance is hydroxypropylcellulose, and the weight ratio of hydroxypropylcellulose to the chemical substance poorly soluble in water as a pharmaceutically active ingredient is 6 to 15.
7. The solid formulation according to any one of the above-mentioned 1 to 6, wherein the inorganic porous substance is calcium silicate, light anhydrous silicic acid, magnesium silicate, magnesium aluminosilicate, magnesium aluminometasilicate, magnesium aluminum silicate, synthetic aluminum silicate, hydrated silicon dioxide or a mixture thereof.
8. The solid formulation according to the above-mentioned 7, wherein the inorganic porous substance is light anhydrous silicic acid.
9. The solid formulation according to any one of the above-mentioned 1 to 8, wherein the weight ratio of the inorganic porous substance to the chemical substance poorly soluble in water as a pharmaceutically active ingredient is 4 to 14.
10. The solid formulation according to any one of the above-mentioned 1 to 9, wherein the chemical substance poorly soluble in water as a pharmaceutically active ingredient is a lipid absorption inhibitor.
11. The solid formulation according to the above-mentioned 10, wherein the lipid absorption inhibitor is an apolipoprotein B (Apo B) secretion inhibitor, a triglyceride (TG) transfer protein inhibitor or a cholesteryl ester transfer protein (CETP) inhibitor.
12. The solid formulation according to the above-mentioned 10, wherein the lipid absorption inhibitor is an MTP inhibitor, a lipase inhibitor or an ACAT inhibitor.
13. The solid formulation according to the above-mentioned 12, wherein the lipid absorption, inhibitor is an MTP inhibitor.
14. The solid formulation according to the above-mentioned 13, wherein the MTP inhibitor is a substituted piperazine derivative or a salt thereof.
15. The solid formulation according to the above-mentioned 13, wherein the MTP inhibitor is a benzamide derivative or a salt thereof.
16. The solid formulation according to the above-mentioned 15, wherein the MTP inhibitor is a benzamide derivative selected from the following or a salt thereof:
   · 4'-trifluoromethyl-biphenyl-2-carboxylicacid-(2-butyl-1,2,3,4-tetrahydroisoquinolin-6-yl)-amide,
   · 4'-trifluoromethyl-biphenyl-2-carboxylicacid-[2-(2-acetylaminoethyl)-1,2,3,4-tetrahydroisoquinolin-6-yl)-amide,
   · 9-[4-[4-(2,3-dihydro-1-oxo-1H-isoindol-2-yl)-1-piperidinyl]butyl]-N-propyl-9H-fluorene-9-carboxamide,
   · 2-[1-(3,3-di-phenylpropyl)-4-piperidinyl]-2,3-dihydro-1H-isoindol-1-one,
   · 9-[4-(4-[2-(4-trifluoromethylphenyl)benzoylamino]piperidin-1-yl)butyl]-N-2,2,2-trifluoroethyl-9H-fluorene-9-carboxamide,
   · 4'-trifluoromethyl-biphenyl-2-carboxylicacid-[2-(1H-[1,2,4]triazol-3-ylmethyl)-1,2,3,4-tetrahydroisoquinolin-6-yl]-amide,
   · 4'-trifluoromethyl-biphenyl-2-carboxylicacid-[2-(2-acetylaminoethyl)-1,2,3,4-tetrahydroisoquinolin-6-yl]-amide,
   · 9H-(4-[4-[4'-trifluoromethyl-biphenyl-2-carbonyl]amino]-piperidin-1-yl}butyl)-9H-fluorene-9-carboxylicacid-(2,2,2-trifluoroethyl)-amide,
   · 9-[4-[4-(2-benzothiazol-2-yl-benzoylamino)piperidin-l-yl]butyl]-9H-fluorene-9-carboxylicacid-(2,2,2-trifluoroethyl)amide,
   · [11a-R]-8-[(4-cyanophenyl)methoxy]-2-cyclopentyl-7-(prop-2-enyl)-2,3,11,11a-tetrahydro-6H-pyrazino[1,2b]isoquinoline-1,4-dione,
   · [11a-R]-2-cyclopentyl-7-(prop-2-enyl)-8-[(pyridin-2-yl)methoxy]-2,3,11,11a-tetrahydro-6H-pyrazino[1,2b]isoquinoline-1,4-dione,
   · 2-cyclopentyl-2-[4-(2,4-dimethylpyrido[2,3-b]indol-9-ylmethyl)phenyl]-N-(2-hydroxy-l-phenylethyl)acetamide and
   · 2-cyclopentyl-N-(2-hydroxy-1-phenylethyl)-2-[4-(quinolin-2-ylmethoxy)-phenyl]acetamide
17. The solid formulation according to the above-mentioned 15, wherein the benzamide derivative is a compound represented by the following formula, wherein X refers to -CON(R¹⁰)-(CH₂)ₙ-, the compound which is an ester compound represented by the general formula (1) : 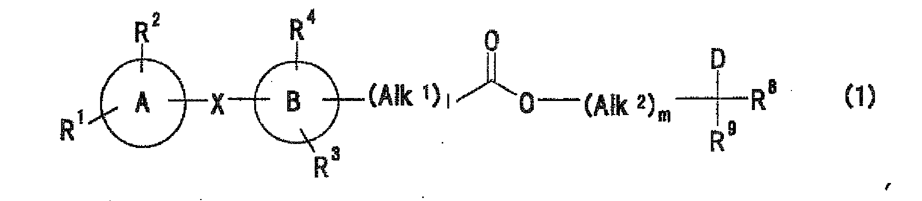 wherein R¹ and R² are a hydrogen atom, a C₁₋₆ alkyl group, a C₃₋₇ cycloalkyl group, a C₁₋₆ alkoxy group, a halo C₁₋₆ alkyl group, a halo C₁₋₆ alkyloxy group, a C₆₋₁₄ aryl group which may be substituted, a C₇₋₁₆ aralkyl group which may be substituted, a C₆₋₁₄ aryloxy group which may be substituted, a C₇₋₁₆ aralkyloxy group which may be substituted, a C₇₋₁₅ arylcarbonyl group which may be substituted, a heterocyclic group which may be substituted, a C₂₋₇ alkoxycarbonyl group, a halogen atom, a C₂₋₆ alkenyl group or -N(R⁴⁰) (R⁴¹), wherein R⁴⁰ and R⁴¹ may be the same or different, and are a hydrogen atom or a C₆₋₁₄ aryl group which may be substituted; ring A is a C₆₋₁₄ aryl group, a heterocyclic group or 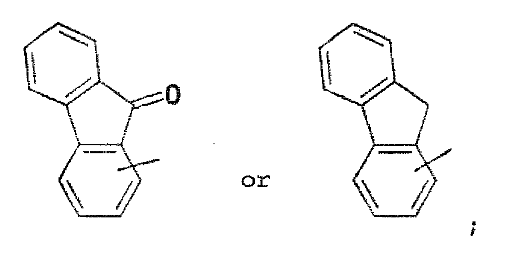
   X is -CON(R¹⁰)-(CH₂)ₙ-, wherein R¹⁰ is a hydrogen atom, a C₁₋₆ alkyl group or a C₃₋₇ cycloalkyl group, and n is 0 or an integer of 1 to 3; R³ and R⁴ may be the same or different, and are a hydrogen atom, a hydroxyl group, a halogen atom, a C₁₋₆ alkyl group which may be substituted, a C₁₋₆ alkoxy group, a halo C₁₋₆ alkyl group, a C₇₋₁₆ aralkyloxy group, a C₁₋₆ acyl group, a heterocyclic group which may be substituted, -CON (R¹¹) (R¹²), wherein R¹¹ and R¹² may be the same or different, and are a hydrogen atom, a C₁₋₆ alkyl group, a C₆₋₁₄ aryl group which may be substituted, a C₇₋₁₆ aralkyl group which may be substituted or a C₁₋₆ alkoxy group, or may form, together with the nitrogen atom to which they are bonded, 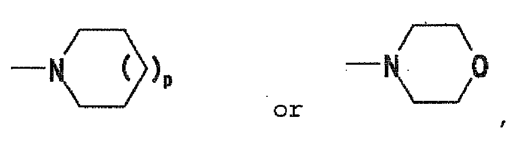 wherein p is 0 or an integer of 1 to 2, -(CH₂)_{q}-N(R¹³) (R¹⁴), wherein R¹³ and R¹⁴ may be the same or different, and are a hydrogen atom, a C₁₋₆ alkyl group, a C₂₋₇ alkoxycarbonyl group or a C₁₋₆ acyl group, or may form, together with the nitrogen atom to which they are bonded, 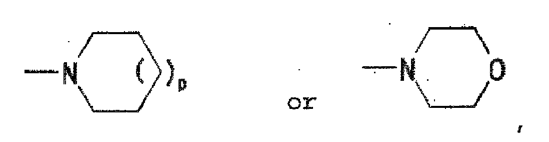 wherein p is the same meaning as above, and q is 0 or an integer of 1 to 3, or -CO(R¹⁵), wherein R¹⁵ is a hydroxyl group, a C₁₋₆ alkoxy group, a C₆₋₁₄ aryloxy group which may be substituted, a C₇₋₁₆ aralkyloxy group which may be substituted or a C₁₋₆ alkyl group; ring B is 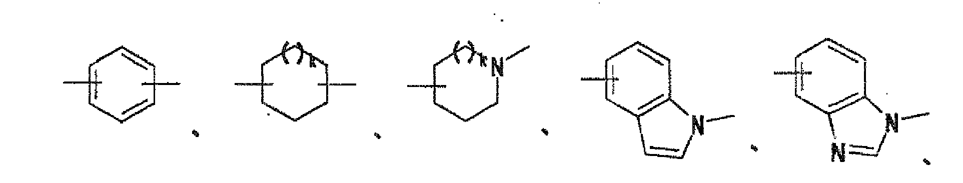 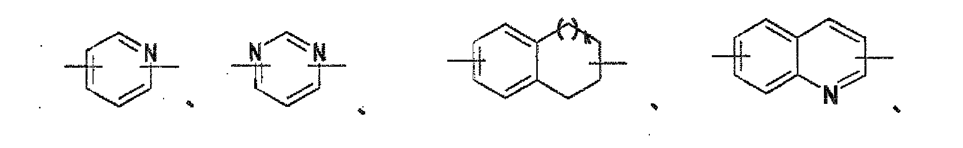 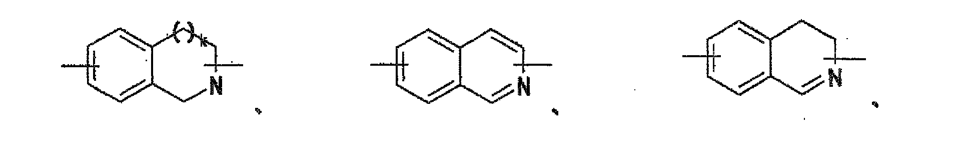 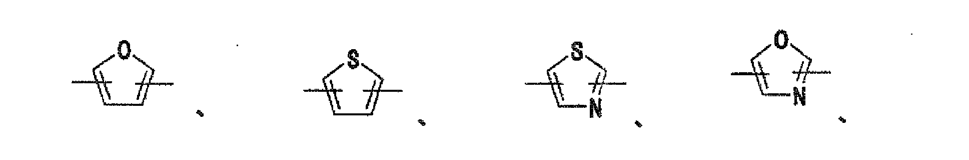 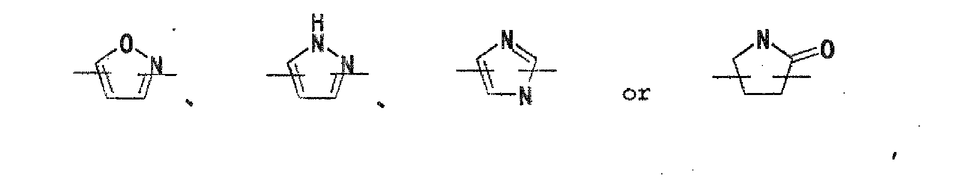 wherein K is 0 or an integer of 1 to 2, or ring B may form, together with R³, R¹⁰ and the nitrogen atom to which R¹⁰ is bonded, 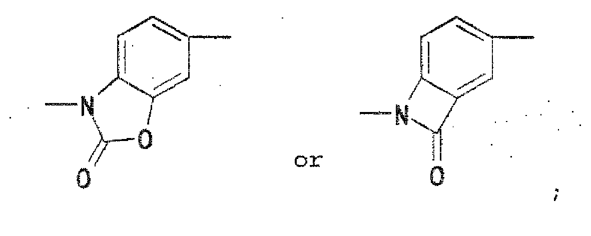
   Alkl¹ is alkandiyl or alkendiyl; Alkl² is alkandiyl or alkendiyl; 1 is 0 or an integer of 1 to 3; m is 0 or an integer of 1 to 3; D is a C₁₋₆ alkyl group, a C₂₋₆ alkenyl group, a C₂₋₇ alkoxycarbonyl group, -N(R⁴²) -CO(R⁴³), wherein R⁴² is a hydrogen atom or a C₁₋₆ alkyl group, and R⁴³ is a C₆₋₁₄ aryl group or a C₇₋₁₆ aralkyl group, or the following formula: 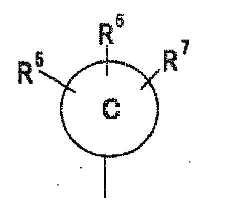 wherein R⁵, R⁶ and R⁷ may be the same or different, and are a hydrogen atom, a C₁₋₆ alkyl group, a C₁₋₆ alkoxy group, a C₂₋₇ alkoxycarbonyl group, a carboxyl group a halogen atom, a cyano group, a nitro group, a halo C₁₋₆ alkyl group, a C₁₋₆ acyl group, a hydroxyl group, an amino group, a C₆₋₁₄ aryl group which may be substituted or - (CH₂)ᵣ-CON(R¹⁶) (R¹⁷) , wherein R¹⁶ and R¹⁷ may be the same or different, and are a hydrogen atom, a C₁₋₆ alkyl group or a halo C₁₋₆ alkyl group, and r is 0 or an integer of 1 to 3; ring C is a C₆₋₁₄ aryl group, a C₇₋₁₅ arylcarbonylamino group, a C₈₋₁₇aralkylcarbonylamino group, a hetero ring residue, a C₃₋₇ cycloalkyl group or a C₇₋₁₆ aralkyl group, or ring C may form, together with R⁷ and R⁸, 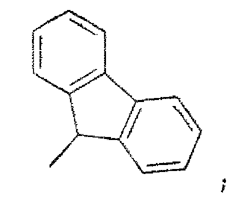 and
   R⁸ and R⁹ may be the same or different, and are a hydrogen atom, a C₁₋₆ alkyl group, a C₆₋₁₄ aryl group which may be substituted, a hydroxy C₁₋₆ alkyl group, -CON(R¹⁸) (R¹⁹), wherein R¹⁸ and R¹⁹ may be the same or different, and are a hydrogen atom, a C₁₋₆ alkyl group, a C₃₋₇ cycloalkyl group, a halo C₁₋₆ alkyl group, a C₂₋₁₂ alkoxyalkyl group or a C₆₋₁₄ aryl group whichmay be substituted, -COO(R²⁰) or -(CH₂)ₛ-OCO(R²⁰), wherein R²⁰ is a hydrogen atom, a C₁₋₆ alkyl group or a C₃₋₇ cycloalkyl group, and s is 0 or an integer of 1 to 3, or -N(R²¹) (R²²), wherein R²¹ and R²² may be the same or different, and are a hydrogen atom, a C₁₋₆ alkyl group, a C₁₋₆ acyl group or a C₁₋₆ alkylsulfonyl group, or R²¹ and R²² may form, together with the nitrogen atom to which they are bonded, 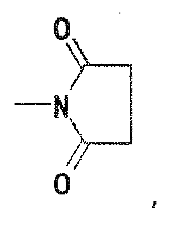
   or R⁸ and R⁹ together may form a C₃₋₇ cycloalkyl group, or a prodrug thereof, or a pharmaceutically acceptable salt thereof.
18. The solid formulation according to the above-mentioned 15, wherein the derivative is a compound selected from the following:
   · 2-phenyl-2-(2-{4-[(4'-trifluoromethyl-biphenyl-2-carbonyl)-amino]-phenyl}-acetoxymethyl)-malonic acid diisobutylester,
   · 2-(2-{4-[ethyl-(4'-trifluoromethyl-biphenyl-2-carbonyl)-amino]-phenyl}-acetoxymethyl)-2-pheny-malonic acid diethylester,
   · 2-(2-{3-dimethylcarbamoyl-4-[(4'-trifluoromethyl-biphenyl-2-carbonyl)-amino]-phenyl}-acetoxymethyl)-2-phenyl-malonic acid diethyl ester,
   · 2-(2-{3-dimethylcarbamoyl-4-[(4'-trifluoromethyl-biphenyl-2-carbonyl)-amino]-phenyl}-acetoxymethyl)-2-phenyl-malonic acid dimethyl ester,
   · 2-cyclopentyl-2-(2-{3-dimethylcarbamoyl-4-[(4'-trifluoromethyl-biphenyl-2-carbonyl) -amino]-phenyl}-acetoxymethyl)-malonic acid diethyl ester,
   · 2-cyclohexyl-2-(2-{3-dimethylcarbamoyl-4-[(4'-trifluoromethyl-biphenyl-2-carbonyl)-amino]-phenyl}-acetoxymethyl)-malonic acid diethyl ester,
   · 2-(2-{3-dimethylcarbamoyl-4-[(4-methyl-4'-trifluoromethyl-biphenyl-2-carbonyl)-amino]-pheny}-acetoxymethyl)-2-phenyl-malonic acid diethyl ester,
   · 2-(2-{3-dimethylcarbamoyl-4-[(5-methyl-4'-trifluoromethyl -biphenyl-2-carbonyl)-amino]-phenyl}-acetoxymethyl)-2-phenyl-malonic acid diethyl ester,
   · 2-[3-(2-{3-dimethylcarbamoyl-4-[(4'-trifluoromethyl-biphenyl-2-carbonyl)-amino]-phenyl}-acetoxy)-propyl]-2-phenyl-malonic acid diethyl ester,
   · 2-(2-{3-dimethylcarbamoyl-4-[(5-methoxy-4'-trifluoromethyl-biphenyl-2-carbonyl)-amino]-phenyl}-acetoxymethyl)-2-phenyl-malonic acid diethyl ester,
   · 2-(2-[4-[(5-chloro-4'-trifluoromethyl-biphenyl-2-carbonyl)-amino]-3-dimethylcarbamoyl-phenyl}-acetoxymethyl)-2-phenyl-malonic acid diethyl ester,
   · 2-(2-{3-dimethylcarbamoyl-4-[(6-methyl-4'-trifluoromethyl-biphenyl-2-carbonyl)-amino]-phenyl}-acetoxymethyl)-2-phenyl-malonic acid diethyl ester,
   · 2-(2-{3-dimethylcarbamoyl-4-[(4'-trifluoromethyl-biphenyl-2-carbonyl)-amino]-phenyl}-acetoxymethyl)-2-phenyl-malonic acid di-2,2,2-trifluoroethyl ester,
   · 2-(2-{3-dimethylcarbamoyl-4-[(2'-fluoro-4'-trifluoromethyl-biphenyl-2-carbonyl)-amino]-phenyl}-acetoxymethyl)-2-phenyl-malonic acid diethyl ester,
   · 2-(2-(5-dimethylcarbamoyl-2-fluoro-4-[(4'-trifluoromethyl-biphenyl-2-carbonyl)-amino]-phenyl}-acetoxymethyl)-2-phenyl-malonic acid diethyl ester,
   · 2-(2-{3-bromo-5-dimethylcarbamoyl-4-[(4'-trifluoromethyl-biphenyl-2-carbonyl)-amino]-phenyl}-acetoxymethyl)-2-phenyl-malonic acid diethyl ester,
   · 2-(2-{3-chloro-5-dimethylcarbamoyl-4-[(4'-trifluoromethyl-biphenyl-2-carbonyl)-amino]-phenyl}-acetoxymethyl)-2-phenyl-malonic acid diethyl ester,
   · 2-(2-{3-dimethylcarbamoyl-4-[(3'-fluoro-4'-trifluoromethyl-biphenyl-2-carbonyl)-amino]-phenyl}-acetoxymethyl)-2-pHenyl-malonic acid diethyl ester,
   · 2-(2-{4-[(3'-chloro-4'-trifluoromethyl-biphenyl-2-carbonyl)-amino]-3-dimethylcarbamoyl-phenyl}-acetoxymethyl)-2-phenyl-malonic acid diethyl ester,
   · 2-(2-{3-dimethylcarbamoyl-4-[(4'-trifluoromethyl-biphenyl-2-carbonyl)-amino]-phenyl}-acetoxymethyl)-2-(5-nitro-pyridin-2-yl)-malonic acid diethyl ester,
   · 2-(5-amino-pyridin-2-yl)-2- (2-{3-dimethylcarbamoyl-4-[(4' -trifluoromethyl-biphenyl-2-carbonyl)-amino]-phenyl}-acetoxymethyl)-malonic acid diethyl ester,
   · 2-(2-{3-dimethylcarbamoyl-4-[(4'-trifluoromethyl-biphenyl-2-carbonyl)-amino]-phenyl}-acetoxymethyl)-2-pyridin-2-yl-malonic acid diethyl ester,
   · 2-(2-{3-chloro-5-dimethylcarbamoyl-2-fluoro-4-[(4'-trifluoromethyl-bipheny-2-carbonyl)-amino]-phenyl}-acetoxymethyl)-2-phenyl-malonic acid diethyl ester,
   · 2-(2-{3-bromo-5-dimethylcarbamoyl-2-fluoro-4-[(4'-trifluoromethyl-biphenyl-2-carbonyl)-amino]-phenyl}-acetoxymethyl)-2-phenyl-malonic acid diethyl ester,
   · 2-(2-{3-dimethylcarbamoyl-4-[(4'-trifluoromethyl-biphenyl-2-carbonyl)-amino]-phenyl}-acetoxymethyl)-2-o-tolyl-malonic acid diethyl ester,
   · 2-(2-{3-dimethylcarbamoyl-4-[(4'-trifluoromethyl-biphenyl-2-carbonyl)-amino]-phenyl}-acetoxymethyl)-2-m-tolyl-malonic acid diethyl ester,
   · 2-(2-{3-dimethylcarbamoyl-4-[(4'-trifluoromethyl-biphenyl-2-carbonyl)-amino]-phenyl}-acetoxymethyl)-2-p-tolyl-malonic acid diethyl ester,
   · 2-(2-chloro-phenyl)-2-(2-{3-dimethylcarbamoyl-4-[(4'-trifluoromethyl-biphenyl-2-carbonyl)-amino]-phenyl}-acetoxymethyl)-malonic acid diethyl ester,
   · 2-(3-chloro-phenyl)-2-(2-{3-dimethylcarbamoyl-4-[(4'-triflucromethyl-biphenyl-2-carbanyl)-amino]-phenyl}-acetoxymethyl)-malonic acid diethyl ester,
   · 2-(4-chloro-phenyl)-2-(2-{3-dimethylearbamoyl-4-[(4'-trifluoromethyl-biphenyl-2-earbonyl)-amino]-phenyl}-acetoxymethyl)-malonic acid diethyl ester,
   · 2-(2-{3-dimethylcarbamoyl-4-[(4'-trifluoromethyl-biphenyl-2-carbonyl)-amino]-phenyl}-acetoxymethyl)-2-phenyl-succinic acid diethyl ester,
   · 2-(2-{3-dimethylcarbamoyl-4-[(4'-trifluoromethyl-biphenyl-2-carbonyl)-amino]-phenyl}-acetoxymethyl)-2-(2-methoxy-phenyl)-malonic acid diethyl ester,
   · 2-(2-{3-dimethylcarbamoyl-4-[(4'-trifluoromethyl-biphenyl-2-carbonyl)-amino]-phenyl}-acetoxymethyl)-2-(3-methoxy-phenyl)-malonic acid diethyl ester,
   · 2-(2-{3-dimethylcarbamoyl-4-[(4'-trifluoromethyl-biphenyl -2-carbonyl)-amino]-phenyl}-acetoxymethyl)-2-(4-methoxy-phenyl)-malonic acid diethyl ester,
   · 2-(2-{4-[(5,4'-bis-trifluoromethyl-biphenyl-2-carbonyl)-amino]-3-dimethylcarbamoyl-phenyl}-acetoxymethyl)-2-phenyl-malonic acid diethyl ester,
   · 2-{2-{4{-[(6-chloro-4'-trifluoromethyl-biphenyl-2-carbonyl)-amino]-3-dimethylcarbamoyl-phenyl}-acetoxymethyl)-2-phenyl-malonic acid diethyl ester,
   · 2-(2-{3-dimethylcarbamoyl-4-[(6-fluoro-4'-trifluoromethyl-biphenyl-2-carbonyl)-amino]-phenyl}-acetoxymethyl)-2-phenyl-malonic acid diethyl ester,
   · 2-[2-(2-{3-dimethylcarbamoyl-4-[(5-methyl-4'-trifluoromethyl-biphenyl-2-carbonyl)-amino]-phenyl}-acetoxy)-ethyl]-2-phenyl-malonic acid diethyl ester,
   · 2-(2-{3-dimethylcarbamoyl-4-f(5-ethoxy-4'-trifluoromethyl-biphenyl-2-carbonyl)-amino]-phenyl}-acetoxymethyl)-2-phenyl-malonic acid diethyl ester,
   · 2-(2-{3-dimethylcarbamoyl-4-[(5-isopropoxy-4'-trifluoromethyl-biphenyl-2-carbonyl)-amino]-phenyl}-acetoxymethyl)-2-phenyl-malonic acid diethyl ester,
   · 2-[2-(2-{4-[(5,4'-bis-trifluoromethyl-biphenyl-2-carbonyl)-amino]-3-dimethylcarbamoyl-phenyl}-acetoxy)-ethyl]-2-phenyl-malonic acid diethyl ester,
   · 2-(2-{3-dimethylcarbamoyl-4-[(6-methoxy-4'-trifluoromethyl-biphenyl-2-carbonyl)-amino]-phenyl}-acetoxymethyl)-2-phenyl-malonic acid diethyl ester and
   · 2-(2-{3-dimethylcarbamoyl-4-[(3-methyl-4'-trifluoromethyl-biphenyl-2-carbonyl)-amino]-phenyl}-acetoxymethyl)-2-phenyl-malonic acid diethyl ester.
19. A production method of a solid formulation having improved water-solubility which comprises a chemical substance poorly soluble in water as a pharmaceutically active ingredient, a water-soluble polymer substance and an inorganic porous substance characterised in that
   (1) the chemical substance poorly soluble in water forms a solid dispersion together with the water-soluble polymer substance,
   (2) the water-soluble polymer substance forming the solid dispersion is in the form of fine pieces, and
   (3) the water-soluble polymer substance in the form of fine pieces is maintained to be adsorbed on and/or adhered to the surface of the inorganic porous substance, the method
   characterised by comprising mixing and stirring a solution obtained by dissolving the substance poorly soluble in water as an active ingredient and the water-soluble polymer substance in an organic solvent with the inorganic porous substance and then by granulating and drying the mixture.
20. The production method of a solid formulation according to the above-mentioned 19, wherein the water-soluble polymer substance is hydroxypropylcellulose, hydroxypropylmethylcellulose, polyvinylpyrrolidone, polyvinyl alcohol, copolyvidone, agar, dextrin, gelatin or a mixture thereof.
21. The production method of a solid formulation according to the above-mentioned 20, wherein the water-soluble polymer substance is polyvinylpyrrolidone or hydroxypropylcellulose.
22. The production method of a solid formulation according to any one of the above-mentioned 19 to 21, wherein the weight ratio of the water-soluble polymer substance to the chemical substance poorly soluble in water as a pharmaceutically active ingredient is 3 to 100.
23. The production method of a solid formulation according to the above-mentioned 22, wherein the water-soluble polymer substance is polyvinylpyrrolidone, and the weight ratio of polyvinylpyrrolidone to the chemical substance poorly soluble in water as a pharmaceutically active ingredient is 3 to 50.
24. The production method of a solid formulation according to the above-mentioned 22, wherein the water-soluble polymer substance is hydroxypropylcellulose, and the weight ratio of hydroxypropylcellulose to the chemical substance poorly soluble in water as a pharmaceutically active ingredient is 6 to 15.
25. The production method of a solid formulation according to any one of the above-mentioned 19 to 24, wherein the inorganic porous substance is calcium silicate, light anhydrous silicic acid, magnesium silicate, magnesium aluminosilicate, magnesium aluminometasilicate, magnesium aluminum silicate, synthetic aluminum silicate, hydrated silicon dioxide or a mixture thereof.
26. The production method of a solid formulation according to the above-mentioned 25, wherein the inorganic porous substance is light anhydrous silicic acid.
27. The production method of a solid formulation according to any one of the above-mentioned 19 to 26, wherein the weight ratio of the inorganic porous substance to the chemical substance poorly soluble in water as a pharmaceutically active ingredient is 4 to 14.
28. The production method of a solid formulation according to any one of the above-mentioned 19 to 27, wherein the weight ratio of the organic solvent to the water-soluble polymer substance is 1 to 2.
29. The production method of a solid formulation according to any one or the above-mentioned 19 to 28, wherein the chemical substance poorly soluble in water as a pharmaceutically active ingredient is a lipid absorption inhibitor.
30. The production method of a solid formulation according to the above-mentioned 29, wherein the lipid absorption inhibitor is an apolipoprotein B (Apo B) secretion inhibitor, a triglyceride (TG) transfer protein inhibitor or a cholesteryl ester transfer protein (CETP) inhibitor.
31. The production method of a solid formulation according to the above-mentioned 29, wherein the lipid absorption inhibitor is an MTP inhibitor, a lipase inhibitor or an ACAT inhibitor.
32. The production method of a solid formulation according to the above-mentioned 31, wherein the lipid absorption inhibitor is an MTP inhibitor.

The solid formulation of the present invention having outstanding solubility has markedly improved bioavailability and can sufficiently and effectively exhibit the function which the pharmaceutical product inherently has with an extremely little dosage and, besides it is excellent in stability. Furthermore, improvement in the solubility brought about by the present invention is not considered to be derived from chemical properties but from physical effects, and therefore application to a wide variety of pharmaceutical products can be expected.

For example, when the technique of the present invention is applied to MTP inhibiting substances and/or Apo B secretion inhibiting substances, since they can be dissolved in the body promptly and completely after administration, the dosage thereof can be reduced, and in addition, a little amount of the drug acts on the intestinum tenue immediately, from which it can be expected that danger of side effects such as fatty liver and unexpected side effects caused by an excessive amount of MTP inhibiting substances arriving at the liver will be reduced. Accordingly, the MTP inhibitor applied with the technique of the present invention can be an unprecedentedly excellent therapeutic or prophylactic agent of hyperlipemia, arterial sclerosis, coronary artery disease, corpulence, diabetes mellitus or hypertension.

It is presumed that a chemical substance as an active ingredient forms a solid dispersion of fine pieces with a water- soluble polymer substance in these solid pharmaceutical formulations and further the water-soluble polymer substance in the form of fine pieces are maintained to be adsorbed on and/or adhered to the surface of the above-mentioned inorganic porous substance surface, thereby contributes to the improvement of solubility.

Furthermore, the solid dispersion obtained by the production method of the present invention is in the form fine pieces and bulky and dense (with a high density) and therefore it does not need to be cut or milled unlike the solid dispersion obtained by the solvent; method or fusion method, and the granules do not adhere to the container nor disperse around during drying operation and they are excellent in handling properties and easier to secure content homogeneity as compared with the granules obtained, by the spray drying method and can be filled into hard capsules and tableted as they are.

### Brief Description of the Drawings

Figure 1 is a graph showing the results of the dissolution test (Example 2) for the solid formulation obtained in Example 1; and
Figure 2 is a graph showing the powder X-ray diffraction pattern (Example 5) of the amorphous solid formulation obtained in Example 3-2.

### Best Mode for Carrying Out the Invention

Meanings of each terms in the present invention are as follows.

The term "solid dispersion" means a solid mixture in which a chemical substance as an active ingredient is dispersed in a polymer substance in single molecular or amorphous state (existence of some crystals is permitted).

The term. "poorly soluble in water" means a state in which a drug is poorly soluble in water and cannot be used as a pharmaceutical product by a usual formulation method and it is specifically expressed as the amount of water required for dissolving 1 g of a drug at about 20°C for not more than 30 minutes.

Generally, if the amount of water required for dissolving 1g of a drug is 100 mL or more and less than 1,000 mL, it is referred to as "hard to dissolve", and if the amount of water required therefor is 1,000 mL or more and less than 10,000 mL, it is referred to as "very hard to dissolve", and if it is 10,000 mL or more, it is referred to as "almost insoluble". Here, the term "poorly soluble in water" refers to the above-mentioned "hard to dissolve", "very hard to dissolve" and "almost insoluble state, and "very hard to dissolve" and "almost insoluble" state in a narrow sense.

The term "having improved water solubility" means that such a hardly soluble drug as mentioned above can be used as a pharmaceutical product by a usual formulation method by the improvement of the solubility in water. Specifically, it refers to the state that the solubility in water at about 20°C is improved to about 5 times, preferably about 10 times, and more preferably 100 times or more.

The term "amorphous" is a term against "crystal", and it means a state that a chemical substance as an active ingredient in a solid dispersion, for example, an MTP inhibiting substance is uniformly dispersed in a carrier matrix consisting of a water-soluble polymer substance in a molecular size or a single molecular state (existence of some crystals is permitted).

Whether it is amorphous or not can be confirmed by powder X-ray diffraction or differential scan calorimetry (hereinbelow, DSC) which are conventional analysis means.

The term "solid formulation" is a term against a liquid formulation or injectable solution and means a solid preparation. As long as it is a solid preparation, there is not restriction in particular and, therefore, it can be used by converting it various kinds of dosage forms such as powder, fine granule, granule, pill and tablet following conventional methods.

The "water-soluble polymer substance" is a polymer substance to form a solid dispersion with a chemical substance as an active ingredient, for example, an MTP inhibiting substance and/or Apo B secretion inhibiting substance. As a water-soluble polymer substance, there is particular no limitation as long as it can form a solid dispersion with these chemical substances. Specific examples include hydroxypropylcellulose, hydroxypropylmethylcellulose, polyvinylpyrrolidone, polyvinyl alcohol, copolyvidone, agar, dextrin, gelatin or a mixture thereof. Preferably it is polyvinylpyrrolidone or hydroxypropylcellulose, and particularly preferably it is polyvinylpyrrolidone. Povidone K30 is preferable in consideration of grade.

These water-soluble polymer substances, particularly polyvinylpyrrolidone, are known as a binder and a stabilizing agent, and it is considered that they do not only form a solid dispersion but also contribute to the stabilization of the chemical substance which is present in the solid dispersion as an amorphous molecule in the solid dispersion.

In order to effectively achieve the purpose of the present invention, these water-soluble polymer substances are preferably 3 to 100 times, particularly preferably 3 to 50 times in amount, by weight to the chemical substance poorly soluble in water (for example, an MTP inhibiting substance, Apo B secretion inhibiting substance). When polyvinylpyrrolidone is used as a water-soluble polymer substance, preferably 3 to 50 times, particularly preferably 3 to 20 times, more preferably 8 to 12 times in amount by weight can be used. Sufficient effect as a formulation, for example, suitable dosage or suitable dissolving properties cannot be obtained out of this range.

When hydroxypropylcellulose is used as a water-soluble polymer substance, preferably it can be used in a range of 6 to 15 times by weight. This is because the stability is inferior to some extent when the amount is less than 6 times.

The "organic solvent" for producing a solid dispersion is not particularly limited as long as it can dissolve both of the chemical substance as an active ingredient (for example, an MTP inhibiting substance, Apo B secretion inhibiting substance) and a water-soluble polymer substance such as PVP, but it is desirable to use a solvent with a relatively low boiling point. For example, alcohols such as methanol, ethanol, propanol, isopropyl alcohol, butanol, monomethoxyethanol, ethylene glycol monomethyl ether; ethers such as diethylether, dibutyl ether diisobutyl ether, dioxane, tetrahydrofuran, ethylene glycol; aliphatic hydrocarbons such as n-hexane, cyclohexane, n-heptane; aromatic hydrocarbons such as benzene, toluene, dimethylbenzene; nitrites such as acetonitrile, organic acids such as acetic acid, propionic acid; esters such as ethyl acetate, aliphatic halogenated hydrocarbons such as dichloromethane, dichloroethane, chloroform; ketones such as acetone, methyl ketone; amides such as dimethylformamide, dimethylacetamide, or a mixture of these in a suitable ratio can be used. Among these, solvents with a relatively low boiling point such as ketones such as acetone and alcohols such as absolute ethanol are preferable and particularly acetone and absolute ethanol are preferable.

The "excipient" is a pharmaceutical additive suitably added to a solid formulation such as powders, granules and tablet suitably shaped thereof and it is used to increase the bulk of the powder formulation or tablet, or expecting an action to accelerate or suppress the compressibility and solubility.

The "inorganic porous substance" is an inorganic material having a lot of inorganic pores, and although it is not particularly limited examples thereof include calcium silicate, light anhydrous silicic acid, magnesium silicate, magnesium aluminosilicate, magnesium aluminometasilicate, magnesium aluminum silicate, synthetic aluminum silicate, hydrated silicon dioxide or a mixture thereof. Calcium silicate or light anhydrous silicic acid is preferred, and light anhydrous silicic acid is particularly desirable. The type of calcium silicate and light anhydrous silicic acid is not particularly limited, as for calcium silicate, those described in Japanese Pharmaceutical Excipients (1998) are desirable and as for light anhydrous silicic acid, those described in Japanese Pharmacopeia (14th revision) are desirable. As for the particle size, those in which 70% or more, preferably 80% or more fall within the range from 0.01 µ to 100 µ are preferable, and those in which 70% or more, preferably 80% or more fall within the range from 0.1 µ to 10 µ are optimally preferable.

The weight ratio of the inorganic porous substance and the chemical substance as an active ingredient (for example, an MTP inhibiting substance, Apo B secretion inhibiting substance) is not limited. However, in order to provide a sufficiently effective excipient, the blending ratio of the inorganic porous substance in the solid dispersion of the present invention is 4 to 100 times is preferable, further 6 to 50 times is preferable, and particularly 7 to 20 times is preferable in amount by weight ratio to the chemical substance as an active ingredient. When the amount of the inorganic porous substance is less than 4 assuming the amount of the chemical substance, an active ingredient, as 1, desirable effective solid dispersion cannot be obtained and thus it is undesirable. When the amount of the inorganic porous substance is more than 100 assuming the amount of the chemical substance, an active ingredient, as 1, even though a solid dispersion can be obtained, the amount to be used of the solid dispersion inevitably increases for obtaining an amount of the MTP inhibiting substance and/or Apo B secretion inhibiting substance therapeutically needed and the dosage per dose to be administered to the patient increases and thus it is undesirable.

The term "in the form of fine piece" means almost flat fine piece, in other words, minute flakes. However, those particulate or filamentous are not excluded and it suffices that 70% or more, preferably 80% or more are the pieces of the above-mentioned flakes. In addition, it is desirable that the size is preferably on the scale of 0.01 times to 100 times, particularly 0.1 times to 10 times of the particle size of the inorganic porous substance and, those in which 70% or more, preferably 80% or more are in the range of 0.01 to 100 µ, particularly 0.1 to 10 µ are desirable.

The term "maintained to be adsorbed on and/or adhered to" does not mean a state where both of the water-soluble polymer substance in the form of fine pieces and the inorganic porous substance forming a water-soluble polymer substance are merely mixed with each other but a state where they are maintained to be adsorbed on and/or adhered to each other.

The formation of the fine piece and the state of absorption/adhesion mentioned above can be attained by stirring mixing a solution having dissolved an active ingredient and a water-soluble polymer substance with an inorganic porous substance, and, followed by granulating and drying.

The term "poorly soluble in water" in the "chemical substance poorly soluble, in water as a pharmaceutically active ingredient" is as defined above. The "chemical substance as a pharmaceutically active ingredient" is a chemical substance constituting a main ingredient for exhibiting an action as a pharmaceutical product. The chemical substance as used herein includes a substance obtained by chemical synthesis as well as a natural substance.

As for the "solid pharmaceutical formulation" of the present invention, the definition thereof is as stated above but other pharmaceutical additives such as "excipient", "lubricant", "binder" "disintegrating agents", "detergent", "antiseptic", "antioxidant", "colorant", "sweetener" can be used if necessary. As examples of the other "excipient", for example, lactose, saccharose, D-mannitol, starch, crystalline cellulose, starch, mannitol, calcium silicate (trade name: Flowlight RE), magnesium aluminometasilicate (trade name: Neucillin), saccharose/starch spherical granules (article name: Nonpareil), crystalline cellulose carboxymethylcellulose (trade name: Avicel RC), hydroxypropyl starch, etc. can be used. Preferable examples of the "lubricant" include crystalline cellulose, magnesium stearate, calcium stearate, talc, colloidal silica, corn starch, magnesium oxide. Preferable examples of the other "binder" include crystalline cellulose, saccharose, D-mannitol, dextrin, hydroxypropylcellulose hydroxypropylmethylcellulose, polyvinylpyrrolidone. As examples of the other "disintegrating agents", for example, starch, carboxymethylcellulose, carboxymethylcellulose calcium, croscarmellose sodium, carboxymethyl starch sodium, methylcellulose (trade name: metolose SM), croscarmellose sodium, carmellose calcium, low substituted hydroxypropylcellulose, starch sodium glycolate, partial alphatized starch can be used. Particularly preferable it is carboxymethylcellulose calcium. As the "detergent", for example, polyoxyethylene polyoxypropylene glycol (trade name: Pluronic), glycerine fatty acid ester, sucrose fatty acid ester, polyoxyethylene consolidation castor oil, polysorbate 80, cetanol, etc. can be used. As preferable examples of the other "antiseptic", paraoxybenzoic acid, chlorobutanol, benzyl alcohol, phenethyl alcohol, dehydroacetic acid, sorbic acid are used. Preferable examples of the "antioxidant" include sulfite and ascorbic acid. These additives may be used alone or two of more of them may be mixed.

The "lipid absorption inhibitor" in the present invention is not limited as long as it is an agent which inhibits or suppresses the absorption of lipid regardless of the action mechanism (chemical substance as an active ingredient:, which may be a natural substance.). Specifically, lipid absorption inhibitors selected from the group consisting of apolipoprotein B (Apo B) secretion inhibitor, triglyceride (TG) transfer protein inhibitor and cholesteryl ester transfer protein (CETP) inhibitor and lipid absorption inhibitors selected from the group consisting of MTP inhibitor, lipase inhibitor and ACAT inhibitor are included.

The "MTP inhibiting substance (MTP inhibitor)", "Apo B secretion inhibiting substance (Apo B secretion inhibitor)" in the present invention respectively mean substances, in particular, chemically synthesized substances, having MTP inhibiting action or Apo B secretion inhibiting action, hydrates or solvates thereof , prodrugs thereof or salts thereof , and they are not particularly limited, and mean any MTP inhibitor, Apo B secretion inhibitor.

The "MTP inhibiting substance is 3-piperidyl-4-oxoquinazoline derivative or a salt thereof" in the present invention means the compounds defined by the above-mentioned and/or following (A) and the salts thereof.

The "MTP inhibiting substance is a piperazine derivative or a salt" in the present invention means the compounds defined by the above-mentioned and/or following (D), (F) and/or (G) and the salts thereof.

The "MTP inhibiting substance is a benzamide derivative or a salt" in the present invention means the benzamide derivatives defined by the above-mentioned and/or following (B), (C), (E), (H), (I), (J), (K) and/or (L) and the salts thereof.

"MTP. inhibiting substances" of these (A) to (L) are specifically as follows.
(A) 3-piperidyl-4-oxoquinazoline derivative defined and described in Japanese Patent Laid-Open Publication No. 11-228569 (see the above-mentioned (A) and Patent Document 14).
(B) Compound group having a piperidine skeleton defined and described in Japanese Parent Laid-Open Publication No. 2004-514676 (WO 2002/042291) (see the above-mentioned (B) and Patent Document 15).
(C) Benzamide derivatives defined and described in National Publication of International Patent Application No. 2003-535900 (WO 01/097810) (see the above-mentioned (C) and Patent Document 16).
(D) Substituted piperazine derivatives defined and described in National Publication of International Patent Application No. 2003-521484 (WO 01/047898) (see the above-mentioned (D) and Patent Document 17).
(E) Benzamide derivatives defined and described in National Publication of International Patent Application No. 2003-520270 (WO 01/053260) (see the above-mentioned (E) and Patent Document 18).
(F) Substituted piperazine derivatives defined and described in National Publication of International Patent Application No. 2003-519131 (WO 01/047899) (see the above-mentioned (F) and Patent Document 19).
(G) Substituted piperazine derivatives defined and described in National Publication of International Patent Application No.2003-509505, WO 01/021604 (see the above-mentioned. (G) and Patent Document 20).
(H) Benzamide derivatives (biphenylamide derivatives) defined and described in National Publication of International Parent Application No. 2003-505373 (WO 01/005762) (see the above-mentioned (H) and Patent Document 21).
(I) Benzamide derivatives defined and described in National Publication of International Patent Application No. 2000-510483, WO 98/47875, for example, 4¹-trifluoromethyl-biphenyl-2-Carboxylic acid (1,2,3,4-tetrahydro-isoquinolin-6-yl)-amide (see the above-mentioned (I) and Patent Document 22).
(J) Benzamide derivatives (biphenylamide derivatives) defined and described in (i) (ii), (iii) and (iv) of Japanese Patent Laid-Open No. 2001-172180 (see the above-mentioned (J) and Patent Document 23).
(K) Benzamide derivatives defined and described in Japanese Patent Laid-Open No. 2000-169395 (see the above-mentioned (K) and Patent Document 24).
(L) Benzamide derivatives defined and described in Japanese Patent Laid-Open No. 2003-321424 (see the above-mentioned (L) and Patent Document 26).

In addition, the following specific compounds are also included in the present invention.

The following compounds described in Japanese Patent Laid-Open. No. 2001-172180 (see Patent Document 23).
· 4'-trifluoromethyl-biphenyl-2-carboxylicacid-(2-butyl-1,2,3,4-tetrahydroisoquinolin-6-yl)-amide,
· 4'-trifluoromethyl-biphenyl-2-carboxylicacid-[2-(2-acetylaminoethyl)-1,2,3,4-tetrahydroisoquinolin-6-yl)-amide,
· 9-[4-[4-(2,3-dihydro-1-oxo-1H-isoindol-2-yl)-1-piperidinyl]butyl]-N-propyl-9H-fluorene-9-carboxamide,
· 2-[1-(3,3-diphenylpropyl)-4-piperidinyl]-2,3-dihydro-1H-isoindol-1-one and
· 9-[4-(4-[2-(4-trifluoromethylphenyl)benzoylamino]piperidin-1-yl)butyl]-N-2,2,2-trifluoroethyl-9H-fluorene-9-carboxamide.

The following compounds described in Japanese Patent Laid-Open No. 2001-172180 (see Patent Document 23).
· 4'-trifluoromethyl-biphenyl-2-carboxylicacid-[2-(1H-[1,2,4]triazol-3-ylmethyl)-1,2,3,4-tetrahydroisoquinolin-6-yl]-amide,
· 4'-trifluoromethyl-biphenyl-2-carboxylicacid-[2-(2-acetylaminoethyl)-1,2,3,4-tetrahydroisoquinolin-6-yl]-amide,
· 9-(4-[4-[4'-trifluoromethyl-biphenyl-2-carbonyl)amino]-piperidin-1-yl}butyl)-9H-fluorene-9-carboxylicacid-(2,2,2-trifluoroethyl)-amide,
· 9-[4-[4-(2-benzothiazol-2-yl-benzoylamino)piperidin-1-yl]butyl]-9H-fluorene-9-carboxylicacid-(2,2,2-trifluoroethyl)amide,
· [11a-R]-8-[(4-cyanophenyl)methoxy}-2-cyclopentyl-7-(prop-2-enyl)-2,3,11,11a-tetrahydro-6H-pyrazino[1,2b]isoquinoline-1,4-dione,
· [11a-R]-2-cyclopentyl-7-(prop-2-enyl)-B-[(pyridin-2-yl)methoxy]-2,3,11,11a-tetrahydro-6H-pyrazino[1,2b]isoquinoline-1,4-dione,
· 2-cyclopentyl-2-[4-(2,4-dimethylpyrido[2,3-b]indol-9-ylmethyl)phenyl]-N-(2-hydroxy-1-phenylethyl)acetamide and
· 2-cyclopentyl-N-(2-hydroxy-l-phenylethyl)-2-[4-(quinolin-2-ylmethoxy)-phenyl]acetamide.

Besides the above, BAY13-9952, CP-10447, R 1037570 or WM-1159 is also included in the present invention.

Particularly favorable "MTP inhibiting substances" include compounds described in the above-mentioned item (L) wherein X is CON(R¹⁰) (CH₂)ₙ- in particular.

To give the compounds described in these (L) by the names of more specific compound, the following can be listed.
· 2-phenyl-2-(2-{4-[(4'-trifluoromethyl-biphenyl-2-carbonyl)-amino]-phenyl}-acetoxy-methyl)-malonic acid diisobutylester,
· 2-(2-{4-[ethyl-(4'-trifluoromethyl-biphenyl-2-carbonyl)-amino]-phenyl}-acetoxymethyl)-2-phenyl-malonic acid diethylester,
· 2-(2-{3-dimethylcarbamoyl-4-[(4'-trifluoromethyl-biphenyl -2-carbonyl)-amino]-phenyl}-acetoxymethyl)-2-phenyl-malonic acid diethyl ester,
· 2-(2-{3-dimethylcarbamoyl-4-[(4'-trifluoromethyl-biphenyl-2-carbonyl)-amino]-phenyl}-acetoxymethyl)-2-phenyl-malonic acid dimethyl ester,
· 2-cyclopentyl-2-(2-{3-dimethylcarbamoyl-4-[(4'-trifluoromethyl-biphenyl-2-carbonyl)-amino]-phenyl}-acetoxymethyl)-malonic acid diethyl ester,
· 2-cyclohexyl-2-(2-{3-dimethylcarbamoyl-4-[(4'-trifluoromethyl-biphenyl-2-carbonyl)-amino]-phenyl}-acetoxymethyl)-malonic acid diethyl ester,
· 2-(2-{3-dimethylcarbamoyl-4-[(4-methyl-4'-trifluoromethyl-biphenyl-2-carbonyl)-amino]-phenyl}-acetoxymethyl)-2-phenyl-malonic acid diethyl ester,
· 2-(2-{3-dimethylcarbamoyl-4-[(5-methyl-4'-trifluoromethyl-biphenyl-2-carbonyl)-amino]-phenyl}-acetoxymethyl)-2-phenyl-malonic acid diethyl ester,
· 2-[3-(2-{3-dimethylcarbamoyl-4-[(4'-trifluoromethyl-biphenyl-2-carbonyl)-amino]-phenyl}-acetoxy)propyl]-2-phenyl-malonic acid diethyl ester,
· 2-(2-{3-dimethylcarbamoyl-4-[(5-methoxy-4'-trifluoromethyl-biphenyl-2-carbonyl)-amino]-phenyl}-acetoxymethyl)-2-phenyl-malonic acid diethyl ester,
· 2-(2-{4-[(5-chloro-4'-trifluoromethyl-biphenyl-2-carbonyl)-amino]-3-dimethylcarbamoyl-phenyl}-acetoxymethyl)-2-phenyl-malonic acid diethyl ester,
· 2-(2-{3-dimethylcarbamoyl-4-((6-methyl-4'-trifluoromethyl-biphenyl-2-carbonyl)-amino]-phenyl}-acetoxymethyl)-2-phenyl-malonic acid diethyl ester,
· 2-(2-{3-dimethylcarbamoyl-4-[(4'-trifluoromethyl-biphenyl-2-carbonyl)-amino]-phenyl)-acetoxymethyl)-2-phenyl-malonic acid di-2,2,2-trifluoroethyl ester,
· 2-(2-{3-dimethylcarbamoyl-4-[(2'-fluoro-4'-trifluoromethyl-biphenyl-2-carbonyl)-amino]-phenyl}-acetoxymethyl)-2-phenyl-malonic acid diethyl ester,
· 2-(2-{5-dimethylcarbamoyl-2-fluoro-4-[(4'-trifluoromethyl-biphenyl-2-carbonyl)-amino]-phenyl}-acetoxymethyl)-2-phenyl-malonic acid diethyl ester,
· 2-(2-{3-bromo-5-dimehylcarbamoyl-4-[(4'-trifluoromethyl- biphenyl-2-carbonyl)-amino]-phenyl}-acetoxymethyl)-2-phenyl-malonic acid diethyl ester,
· 2-(2-{3-chloro-5-dimethylcarbamoyl-4-[(4'-trifluoromethyl-biphenyl-2-carbonyl)-amino]-phenyl}-acetoxymethyl)-2-phenyl-malonic acid diethyl ester,
· 2-(2-{3-dimethylcarbamoyl-4-[(3'-fluoro-4'-trifluoromethyl-biphenyl-2-carbonyl)-amino]-phenyl}-acetoxymethyl)-2-phenyl-malonic acid diethyl ester,
· 2-(2-{4-[(3'-chloro-4'-trifluoromethyl-biphenyl-2-carbonyl)-amino]-3-dimethylcarbamoyl-phenyl}-acetoxymethyl)-2-phenyl-malonic acid diethyl ester,
· 2-(2-{3-dimethylcarbamoyl-4-[(4'-trifluoromethyl-biphenyl-2-carbonyl)-amino]-phenyl}-acetoxylethyl)-2-(5-nitro-pyridin-2-yl)-malonic acid diethyl ester,
· 2-(5-amino-pyridin-2-yl)-2-(2-{3-dimethylcarbony-4-[(4'-trifluoromethyl-biphenyl-2-carbonyl)-amino]-phenyl}-acetoxymethyl)-malonic acid diethyl ester,
· 2-(2-{3-dimethylcarbamoyl-4-[(4'-trifluoromethyl-biphenyl-2-carbonyl)-amino]-phenyl}-acetoxymethyl)-2-pyridin-2-yl-malonic acid diethyl ester,
· 2-(2-{3-chloro-5-dimethylcarbamoyl-2-fluoro-4-[(4'-trifluoromethyl-biphenyl-2-carbonyl)-amino]-phenyl}-acetoxymethyl)-2-phenyl-malonic acid diethyl ester,
· 2-(2-{3-bromo-5-dimethylcarbamoyl-2-fluoro-4-[(4'-trifluoromethyl-biphenyl-2-carbonyl)-amino]-phenyl}-acetoxymethyl)-2-phenyl-malonic acid diethyl ester,
· 2-(2-{3-dimethylcarbamoyl-4-[(4'-trifluoromethyl-biphenyl-2-carbonyl)-amino]-phenyl}-acetoxymethyl)-2-o-tolyl-malonic acid diethyl ester,
· 2-(2-{3-dimethylcarbamoyl-4-[(4'-trifluoromethyl-biphenyl-2-carbonyl) -amino] -phenyl}-acetoxymethyl) -2-m-tolyl-malonic acid diethyl ester,
· 2-(2-{3-dimethylcarbamoyl-4-[(4'-trifluoromethyl-biphenyl-2-carbonyl)-amino]-phenyl}-acetoxymethyl)-2-p-tolyl-malonic acid diethyl ester,
· 2-(2-chloro-phenyl)-2-(2-{3-dimethylcarbamoyl-4-[(4'-trifluoromethyl-biphenyl-2-carbonyl)-amino]-phenyl}-acetoxymethyl)-malonic acid diethyl ester,
· 2-(3-chloro-phenyl)-2-(2-{3-dimethylcarbamoyl-4-[(4'-trif luoromethyl-biphenyl-2-carbonyl)-amino]-phenyl}-acetoxymethyl)-malonic acid diethyl ester,
· 2-(4-chloro-phenyl)-2-(2-{3-dimethylcarbamoyl-4-[(4'-trifluoromethyl-biphenyl-2-carbonyl)-amino]-phenyl}-acetoxymethyl)-malonic acid diethyl ester,
· 2-(2-{3-dimethylcarbamoyl-4-[(4'-trifluoromethyl-biphenyl-2-carbonyl)-amino]-phenyl}-acetoxymethyl)-2-phenyl-succinic acid diethyl ester,
· 2-(2-{3-dimethylcarbamoyl-4-[(4'-trifluoromethyl-biphenyl-2-carbonyl)-amino]-phenyl}-acetoxymethyl)-2-(2-methoxy-phenyl)-malonic acid diethyl ester,
· 2-(2-{3-dimethylcarbamoyl-4-[(4'-trifluoromethyl-biphenyl-2-carbonyl)-amino]-phenyl}-acetoxymethyl)-2-(3-methoxy-phenyl)-malonic acid diethyl ester,
· 2-(2-{3-dimethylcarbamoyl-4-[(4'-trifluopromethyl-biphenyl-2-carbonyl)-amino]-phenyl}-acetoxymethyl)-2-(4-methoxy-phenyl)-malonic acid diethyl ester,
· 2-(2-{4-[(5,4'-bis-trifluoromethyl-biphenyl-2-carbonyl)-amino]-3-dimethylcarbamoyl-phenyl}-acetoxymethyl-2-phenyl-malonic acid diethyl ester,
· 2-(2-{4-[(6-chloro-4'-trifluoromethyl-biphenyl-2-carbonyl)-amino]-3-dimethylcarbamoyl-phenyl}-acetoxymethyl)-2-phenyl-malonic acid diethyl ester,
· 2-(2-{3-dimethylcarbamoyl-4-[(6-fluoro-4'-trifluoromethyl-biphenyl-2-carbonyl)-amino]-phenyl}-acetoxymethyl)-2-phenyl-malonic acid diethyl ester,
· 2-[2-(2-{3-dimethylcarbamoyl-4-[(5-methyl-4'-trifluoromethyl-biphenyl-2-carbonyl)-amino]-phenyl}-acetoxy)-ethyl]-2-phenyl-malonic acid diethyl ester,
· 2-(2-{3-dimethylcarbamoyl-4-[(5-ethoxy-4'-trifluoromethyl-biphenyl-2-carbonyl)-amino]-phenyl}-acetoxymethyl)-2-phenyl-malonic acid diethyl ester,
· 2-(2-{3-dimethylcabamoyl-4-[(5-isopropoxy-4'-trifluoromethyl-biphenyl-2-carbonyl)-amino]-phenyl}-acetoxymethyl)-2-phenyl-malonic acid diethyl ester,
· 2-[2-(2-{4-[(5,4'-bis-trifluoromethyl-biphenyl-2-carbonyl)-amino]-3-dimethylcarbamoyl-phenyl}-acetoxy)-ethyl]-2-phenyl-malonic acid diethyl ester,
· 2-(2-{3-dimethylcarbamoyl-4-[(6-methoxy-4'-trifluoromethy 1-biphenyl-2-carbonyl)-amino]-phenyl}-acetoxymethyl)-2-phenyl-malonic acid diethyl ester and
· 2-(2-{3-dimethylcarbamoyl-4-[(3-methyl-4'-trifluoromethyl-biphenyl-2-carbonyl)-amino]-phenyl}-acetoxymethyl)-2-phenyl-malonic acid diethyl ester.

The salts of the compound constituting MTP inhibiting substances, Apo B secretion inhibiting substances are preferably pharmaceutically acceptable salts, and, for example, include salts with inorganic bases, salts with organic bases, salts with inorganic acids, salts with organic acids, and salts with basic or acidic amino acids. Preferable examples of the salts with inorganic bases include alkali metal salts such as sodium salt, potassium salt; alkaline earth metal salts such as calcium sodium, magnesium salt, aluminum salt; ammonium salts. Preferable examples of the salts with organic bases include, salts with trimethylamine, triethylamine, pyridine, picoline, ethanolamine, diethanolamine, triethanolamine, cyclohexylamine, N,N'-dibenzylethylenediamine. Preferable examples of the salts with inorganic acids include salts with hydrochloric acid, hydrobromic acid, nitric acid, sulfuric acid, phosphoric acid. Preferable examples of the salts with organic acids include, salts with formic acid, acetic acid, trifluoroacetic acid, fumaric acid, oxalic acid, tartaric acid, maleic acid, citric acid, *s*uccinic acid, malic acid, methanesulfonic acid, benzenesulfonic acid, p-toluenesulfonic acid. Preferable examples of the salts with basic amino acids include salts with arginine, lysine and orni thine, and preferable examples of the salts with acidic amino acids include, salts with aspartic acid and glutamic acid.

The process of the solid pharmaceutical formulation of the present invention is as follows.

### 1. A granulating process

(1) An appropriate amount of a chemical substance poorly soluble in water as an active ingredient, for example, an MTP inhibiting substance is dissolved in a suitable organic solvent such as acetone, absolute ethanol or a mixed solvent thereof. The organic solvent should be in an amount sufficient to dissolve a polymer substance to be added at the next step. However, too much solvent unnecessarily used causes the following steps cumbersome and further adversely affects the formulation, and accordingly it is appropriate to use the solvent in an amount of 1 to 2 times by weight of the polymer substance. Stirring dissolution can be performed, for example, by using a propeller mixer.
(2) A water-soluble polymer substance is added in an organic solvent having dissolved an appropriate amount of the chemical substance and further mixing and stirring are performed to sufficiently dissolve both of the chemical substance and the water-soluble polymer substance. The solvent may be heated if needed at this time to promote the dissolution.
(3) Separately from this, an inorganic porous substance such as light anhydrous silicic acid as an excipient and a disintegrating agent such as carboxymethylcellulose calcium are mixed and stirred as required by a blending stirrer and the like.
(4) Next, a mixture comprising the inorganic porous substance prepared in the above-mentioned (3) and a disintegrating agent and a solution having dissolved both of the chemical substance and the polymer substance obtained in (2) are mixed and granulated by a conventional method.
   It is preferable from an operability point of view that the solution of (2) is added and mixed to the mixture obtained at (3), but this is not particularly limited and the mixture of (3) may be added and mixed to the solution of (2).
(5) Next, this is granulated and subsequently subjected to vacuum drying and the like thereby the organic solvent is evaporated under reduced pressure or ordinary pressure.
(6) Finally, the dried, granules are sized, with a sieve, etc.

The organic solvent to be used at the above-mentioned operation (1) is not particularly limited as long as it can dissolve the chemical substance poorly soluble or insoluble in water as an active ingredient (for example, an MTP inhibiting substance and/or Apo B secretion inhibiting substance) and the polymer substance, and for example, alcohols such as methanol, ethanol, propanol, isopropyl alcohol, butanol, monomethoxyethanol, ethylene glycol monomethyl ether; ketones such as acetone, methylketone or a mixture thereof can be used. Solvent of low boiling point such as ketones such as acetone, alcohols such as absolute ethanol is preferable, and particularly acetone, absolute ethanol or a mixture thereof is preferable. Water may be added if necessary.

### 2. Punching step

If a tablet is desired, a disintegrating agent such as carboxymethylcellulose calcium, cross-linked polyvinylpovidone and a lubricant such as magnesium stearate are added to and uniformly mixed with the sized granular substance obtained at the above-mentioned granulating step and punched by a conventional method to obtain a tablet.

The amount of the chemical substance to be contained in the solid formulation as an active ingredient may be different depending on drug form, administration method, carrier, or age, body weight, etc. of the subject to be administered and can be determined in accordance with each of the condition and environment, and it is not particularly limited. In the case of MTP inhibiting substance, usually it is 0.1 to 20% (w/w), preferably 1 to 8% (w/w) to the total amount of the formulation. When it is 0.1% (w/w) or less, the effect as a drug cannot be expected while when it is 20% (w/w) or more, the effect as a solid dispersion cannot sufficiently be exhibited.

In order to attain a sufficient effect as a solid dispersion, the blending ratio of the water-soluble polymer substance in the solid formulation of the present invention is 3 to 100 times, preferably 3 to 50 times, more preferably 3 to 20 times in amount by weight ratio to the chemical substance as an active ingredient (for example, an MTP inhibiting substance, Apo B secretion inhibiting substance), and it is 3 to 20 times amount, more preferably 8 to 12 times amount by weight ratio in the case of polyvinylpyrrolidone and, in the case of hydroxypropylcellulose, particularly preferably 6 to 15 times amount.

In order to attain a sufficient effect as a solid dispersion of the present invention, the inorganic porous substance is preferably 4 to 14 times amount, more preferably 6 to 13 times amount, and particularly preferably 7 to 11 times amount by weight ratio to the chemical substance as an active ingredient (for example, an MTP inhibiting substance, Apo B secretion inhibiting substance).

In order to attain a sufficient effect as a solid dispersion of the present invention, the disintegrating agent is preferably 1 to 10 times amount, and particularly preferably 3 to 7 times amount by weight ratio to the chemical substance as an active ingredient (for example, an MTP inhibiting substance, Apo B secretion inhibiting substance). Preferred disintegrating agent is a cross-linked polyvinyl pyrrolidone (crosspovidone).

The thus obtained "solid dispersion" is dense (with high density) and it can be used as a tablet as well as a pharmaceutical formulation for an oral administration. In addition, it may be formed into a pharmaceutical formulation such as a fine grain agent, fine granules, granules, capsules, liquid drug. When a pharmaceutical formulation such as powders, granules or capsules is desired, such a formulation can be easily obtained by adding a suitable excipient, disintegrating agent, lubricant, detergent, etc. as required to the sized drug particles obtained by the above-mentioned method and performing preparation by a conventional method. Production methods of these formulation are known to those skilled in the art and a suitable method can be adopted in accordance with the formulation.

Further, if a sugarcoated tablet or the like is desired, the surface of the formulation may be coated with a suitable coating composition.

Usually, chemical, substances as a pharmaceutically active ingredient (for example, an MTP inhibiting substance, Apo B secretion inhibiting substance) are poorly soluble or insoluble in water and when it is orally administered, the ratio of the dosage which is really absorbed to the blood is small and thus has a defect that bioavailability is low. The solid pharmaceutical formulation of the present invention however, has excellent solubility, oral absorbency and absorbency to the blood, and therefore, bioavailability of the chemical substance poorly soluble in water has been drastically improved.

Furthermore, the solid formulation of the present invention has not only improved solubility but also maintained outstanding stability.

The solid formulation of the present invention can be used in combination with other pharmaceutical formulations. For example, the solid formulation of the present invention containing as an active ingredient, anMTP inhibiting substance, Apo B secretion inhibiting substance, can be used in combination with another drug for treating hyperlipemia. In this case, the solid formulation of the MTP inhibiting substance or Apo B secretion inhibiting substance can be taken separately from the other drug for treating hyperlipemia, or alternatively the both can be incorporated into a combinational drug.

### Example 1

Hereinbelow, the present invention is described in detail by way of Examples and Test Examples, but the present invention is not limited to these.

An MTP inhibiting substance comprising a benzamide derivative, diethyl 2-(2-{3-dimethylcarbamoyl-4-[(4'-trifluoromethyl-biphenyl -2-carbonyl)-amino]-phenyl}-acetoxymethyl)-2-phenyl-malon ate therein below, simply referred to as "MTP inhibition compound") was used as a chemical substance poorly soluble in water serving as an active ingredient in the following Examples.
1. Granulating,
   (1) Dissolution of chemical substance as active ingredient
      418.5 g of acetone and 46.5 g of absolute ethanol (ratio by weight%; 9: 1) were injected into a propeller mixer and 30.0 g of the above-mentioned MTP inhibiting compound as a chemical substance poorly soluble in water was added to and mixed with the resulting acetone/absolute ethanol mixture and fully dissolved.
   (2) Dissolution of polymer substance
      300.0 g of polyvinylpyrrolidone (Povidone K30) as a water-soluble polymer substance was gradually added to the above-mentioned solvent having dissolved an MTP inhibiting compound, subjected to further mixing and stirring and thereby dissolved.
   (3) Preparation of excipient,
      Separately from this, 239.1 g of light anhydrous silicic acid as an excipient and 150.0 g of carboxymethylcellulose calcium as a disintegrating agent were mixed using a blending stirrer and uniformly mixed and stirred with a blending and stirring granulator.
   (4) Mixing of excipient and polymer substance solution
      The organic solvent mixture, prepared at step (2) having dissolved an MTP inhibiting compound and polyvinylpyrrolidone was gradually added to the light anhydrous silicic acid mixture prepared at step (3) until the mixture became homogeneous.
   (5) Rough granulation
      The mixture obtained in the above-mentioned (4) was granulated by a conventional method and subsequently dried this with a vacuum dryer.
   (6) Sizing
      Furthermore, sizing operation was performed by a conventional method using a sieve having a mesh size of 600 µm to make the grain size uniform.
2. Tablet punching
   The above-mentioned granulating process was repeated twice and 150.0 g of a cross-linked polyvinylpyrrolidone (crosspovidone) as a disintegrating agent and 7.8 g of magnesium stearate as a lubricant were uniformly mixed with 1,438.2 g of the thus obtained granules using a W-type mixer and then 1, 200 tablets containing about 133 mg per tablet (about. 5 mg/tablet MTP inhibiting compound as an active ingredient) were produced by tablet punching using a rotary tablet machine.

### Example 2

### Dissolution test

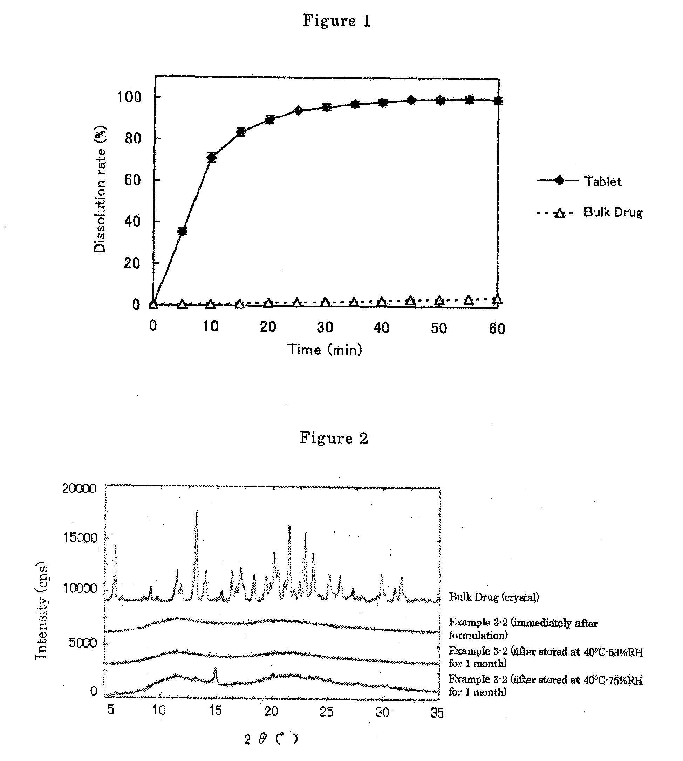

Dissolution test was performed following the second method of Japanese Pharmacopoeia dissolution test method (paddle method, 50 rpm) using a 0.1 w/v% deaerated sodium lauryl sulfate solution (900 mL, 37°C). The tablets (133.00 mg/tablet; containing 5 mg of bulk drug) produced in Example 1 were put into the test solution as they were. For referential control, the bulk drug consisting crystal was put into the test solution as it was. The temperature was adjusted to 37±0.1°C. The results are shown in Figure 1.

As shown in FIG 1, 95.45% of the solid formulation of the present invention dissolved in 15 minutes while the referential control compound (crystal) did not dissolve, even after 60 minutes.

Considering that it is said that the food entering the mouth will arrive at intestinum tenue for about 15 minutes to 30 minutes, the solid formulation of the present invention will be completely dissolved at a time point where the present invention formulation arrives at intestinum tenue and will be absorbed extremely efficiently by intestinum tenue, and it may be said that this is ideal for a drug, in particular, targeting the intestinum tenue.

In addition, since it has an excellent solubility, the dosage can be reduced, that is, minimum administration, is enabled and since it has an improved solubility, the amount of the drug migrated to the blood increases effectively. This prevents unnecessary drug which is not decomposed in the blood from being transferred to the liver and as a result can prevent a risk derived from the drug (for example, possibility of fat liver by the MTP inhibiting substance) and sometimes an unexpected risk beforehand.

In addition, the effect or the improvement in solubility is supposed to be derived not from the chemical properties of the active ingredient substance but from the mechanism in which a chemical substance as an active ingredient forms a solid dispersion in the form of fine pieces with a water-soluble polymer substance and the water-soluble polymer substance in the form of fine pieces is maintained to be adsorbed on and/or adhered to the surface of the above-mentioned inorganic porous substance and thereby contributes to the improvement in solubility.

Therefore, the formulation technique of the present invention can be applied to a wide variety of the drug compounds without being affected by the chemical properties of the drug as the active ingredient.

### Example 3

Formulation for stability test and confirmation test of amorphousness

Test formulations were produced in the following method in order to confirm the stability and amorphousness of the present solid formulation.

In the meantime, it was considered that main cause which affected the stability and crystallinity depended on the combination of the water-soluble polymer substance and the chemical substance poorly soluble in water, solid dispersions consisting of both of these substances were used as test formulations. The above-described MTP inhibiting compound was used as a chemical substance poorly soluble in water.

The test formulation was prepared by adding an appropriate amount of the MTP inhibiting compound to an appropriate amount of a suitable solvent as shown below and further adding a polymer substance to be tested gradually, dissolving it enough, and performing spray drying of this.

### Example 3-1

A powder formulation was obtained by dissolving 5 g of the MTP inhibiting compound and 15 g of polyvinylpyrrolidone (Colidone 30, product of BASF) in a mixed solvent of 108 g of acetone and 72 g of water and spray drying.

### Example 3-2

A powder formulation was obtained by dissolving 5 g of the MTP inhibiting compound and 50 g of polyvinylpyrrolidone (Colidone 30, product of BASF) in a mixed solvent of 270 g of acetone and 180 g of water and spray drying.

### Example 3-3

A powder formulation was obtained by dissolving 5 g of the MTP inhibiting compound and 15 g of hydroxypropylcellulose (HPC-SSL, product of Nippon Soda) in 180 g of acetone and spray drying.

### Example 3-4

A powder formulation was obtained by dissolving 5 g of the MTP inhibiting compound and 50 g of hydroxypropylcellulose (HFC-SSL, product of Nippon Soda) in 450 g of acetone and spray drying.

### Example 3-5

A powder formulation was obtained by dissolving 5 g of the MTP inhibiting compound and 15 g of hydroxypropylmethylcellulose (TC-5EW, a product made in Shin-Etsu Chemical) in a mixed solvent of 135 g of acetone and 60 g of water and spray drying.

### Example 3-6

A powder formulation was obtained by dissolving 5 g of the MTP inhibiting compound and 50 g of hydroxypropylmethylcellulose (TC-5EW, a product made in Shin-Etsu Chemical) in a mixed solvent of 375 g of acetone and 150 g of water and spray drying.

### Example 4

### Stability test under heating condition

Each of the powder formulations obtained in Example 3 was tested under heating condition at 80°C. The purity was determined by high performance liquid chromatography (measured wave length; 242 nm). The state of crystallization was estimated by powder X-ray diffraction. The results were as shown in the following Table 1.

Here, the term "Max" in Table 1 means the percentage of the impurity which showed the highest peak among one or more one impurities, and "Total" means total percentage of all the impurities.

**Table 1: Stability of MTP Inhibiting Compound Powder Obtained by Spray Drying Method**

| Polymer Substance | Mixing Ratio MTP Inhibiting Compound/Polymer Substance | Immediately after Formulation | | | 80°C, Open. Condition, After 3 days | | |
|---|---|---|---|---|---|---|---|
| | | Impurities | | State of crystal | Impurities | | State of crystal |
| | | Max (%) | Total (%) | | Max (%) | Total (%) | |
| MTP Inhibiting Compound | | 0.28 | 0.40 | | 0.08 | 0.24 | |
| PVP (Colidone 30) | 1/3 | 0.28 | 0.70 | Amorphous | 0.34 | 1.35 | Amorphous |
| | 1/10 | 0.12 | 0.53 | Amorphous | 0.19 | 1.09 | Amorphous |
| HPC (HPC-SSL) | 1/3 | 0.08 | 0.43 | Amorphous | 0.25 | 0.69 | Non-amorphous |
| | 1/10 | 0.08 | 0.46 | Amorphous | 0.36 | 0.68 | Amorphous |
| HPMC (TC-5EW) | 1/3 | 0.12 | 0.71 | Amorphous | 0.41 | 0.93 | Amorphous |
| | 1/10 | 0.23 | 0.79 | Amorphous | 0.99 | 1.62 | Amorphous |

| | | | | | | | |
|---|---|---|---|---|---|---|---|
| PVP; Polyvinylpyrrolidone (Povidone K30) HPC; Hydroxypropylcellulose HPMC; cellulose 2910 | | | | | | | |

As is apparent from the above Table 1, the formulations of the present invention maintained the amorphous state not to mention the initial state after formulation but under heated condition at 80°C and therefore retained stability.

### Example 5

### Confirmation of amorphousness

In order to observe amorphous state of the formulations obtained in Example 3-2, they were measured by powder X-ray diffraction method immediately after formulation and conditions at 40°C, inone month, and at various humidity (11%, 32%, 53%, 75%). The test was performed by placing an appropriate, amount of the sample in an aluminum cell having a depth of 0.2 mm and by measuring powder X-ray diffraction pattern with a scan speed of 5° per minute. The results are shown in Figure 2.

As is apparent from Figure 2, the formulations of the present invention maintained their good amorphous state under these conditions and therefore have an excellent stability.

### Example 6 '

### Stability test under humid condition

Each of the powder formulations obtained in Example 3 was tested under heating condition at 40°C under humid condition. The results were as shown in the following Table 2.

**Table 2 : Stability of MTP Inhibiting Compound Powder Obtained by Spray Drying Method**

| Solid Dispersion Polymer Substance MTP Inhibiting Compound / Polymer Substance | | Immediately after Formulation | 40°C 11% RH 1 month | 40°C 32% RH 1 month | 40°C 53% RH 1 month |
|---|---|---|---|---|---|
| MTP Inhibiting Compound | A | 0.11 | | | |
| | B | 0.40 | | | |
| PVP 1/3 | A | 0.11 | 0.10 | 0.09 | 0.09 |
| | B | 0.55 | 0.52 | 0.50 | 0.54 |
| | C | Amorphous | Amorphous | Amorphous | Amorphous |
| PVP 1/10 | A | 0.08 | 0.09 | 0.09 | 0.09 |
| | B | 0.50 | 0.51 | 0.52 | 0.53 |
| | C | Amorphous | Amorphous | Amorphous | Amorphous |
| HPC 1/3 | A | 0.08 | 0.08 | 0.10 | 0.09 |
| | B | 0.43 | 0.45 | 0.50 | 0.5 0 |
| | C | Amorphous | Amorphous | Amorphous | Non- amorphous |
| HPC 1/10 | A | 0.08 | 0.08 | 0.22 | 0.21 |
| | B | 0.46 | 0.48 | 0.67 | 0.64 |
| | C | Amorphous | Amorphous | Amorphous | Amorphous |
| HPMC2910 1/3 | A | 0.12 | 0.18 | 0.65 | 1.19 |
| | B | 0.71 | 0.18 | 0.65 | 1.19 |
| | C | Amorphous | Amorphous | Amorphous | Amorphous |
| HPMC2910 1/10 | A | 0.23 | 0.39 | 2.36 | 4.20 |
| | B | 0.79 | 1.04 | 3.05 | 4.56 |
| | C | Amorphous. | Amorphous | Amorphous | Amorphous |

| | | | | | |
|---|---|---|---|---|---|
| A; Impurities Max (%) B; impurities Total (%) C; State of crystal. RH; Relative humidity | | | | | |

As is apparent from the above Table 2, polyvinylpyrrolidone and hydroxypropylcellulose, in particular polyvinylpyrrolidone, showed excellent stability under various humidity conditions.

### Industrial Applicability

As is apparent from the results of the above-mentioned stability test, solid formulations of the present invention, particularly solid formulations using polyvinylpyrrolidone or hydroxypropylcellulose, particularly preferably polyvinylpyrrolidone and further using these polymer substances for a chemical substance as an active ingredient (for example, an MTP inhibiting substance, Apo B secretion inhibiting substance) in about 3 to 100 weight times, and preferably, about 3 to 2 0 weight times, particularly preferably about 8 to 12 weight times in the case or PVP, and about 6 to 15 weight times in the case of HPC have outstanding water dissolution characteristics, and, besides have an excellent stability.

The solid formulation of the present invention has an outstanding water dissolution characteristic as above, and enables high bioavailability. This fact has three important significances. The first is that practical use as a drug of a number of poorly soluble chemical substances as active ingredients, for example, MTP inhibiting substance and/or Apo B secretion inhibiting substance has been enabled due to the realization of high bioavailability; the second is that due to its prompt dissolution, it can be promptly absorbed by the intestinum tenue which is a drug absorption site, and it selectively acts on the intestinum tenue particularly in the case of a drug targeting the intestinum tenue; and the third is that due to its outstandingly improved solubility the dosage has been markedly reduced as compared with conventional formulations, and burdens to the liver, and possibility of side effect such as fatty liver or the side effect which has not been expected are reduced.

In addition, the production method in itself is extremely simple, and besides, it is effective.

In this way, the solid formulation of the present invent ion has surprising dissolution characteristic and is excellent in stability and besides the production method is simple and effective and therefore, it can be widely applied to the drugs which are poorly soluble in water and have been difficult to practical application.

Therefore, when the technique of the solid formulation of the present invention is applied to, for example, lipid absorption inhibiting substances such as MTP inhibiting substances, Apo B secretion inhibiting substances, they can be therapeutic or prophylactic agents for hyperlipemia, arterial sclerosis, coronary artery disease, adiposis, diabetes mellitus or hypertension hypertension superior to any of those and free of the fears of fatty liver and unexpected side effects.

## Claims

1. A solid formulation having improved water-solubility which comprises a chemical substance poorly soluble in water as a pharmaceutically active ingredient, a water-soluble polymer substance and an inorganic porous substance,
**characterized in that**
(1) the chemical substance poorly soluble in water forms a solid dispersion together with the water-soluble polymer substance,
(2) the water-soluble polymer substance forming the solid dispersion is in the form of fine pieces, and
(3) the water-soluble polymer substance in the form of fine pieces is maintained to be adsorbed on and/or adhered to the surface of the inorganic porous substance.

2. The solid formulation according to claim 1, wherein the water-soluble polymer substance is hydroxypropylcellulose, hydroxypropylmethylcellulose, polyvinylpyrrolidone, polyvinyl alcohol, copolyvidone, agar, dextrin, gelatin or a mixture thereof.

3. The solid formulation according to claim 2, wherein the water-soluble polymer substance is polyvinylpyrrolidone or hydroxypropylcellulose.

4. The solid formulation according to any one of claims 1 to 3, wherein the weight ratio of the water-soluble polymer substance to the chemical substance poorly soluble in water as a pharmaceutically active ingredient is 3 to 100.

5. The solid formulation according to claim 4, wherein the water-soluble polymer substance is polyvinylpyrrolidone , and the weight ratio of polyvinylpyrrolidone to the chemical substance poorly soluble in water as a pharmaceutically active ingredient is 3 to 50.

6. The solid formulation according to claim 4, wherein the water-soluble polymer substance is hydroxypropylcellulose, and the weight ratio of hydroxypropylcellulose to the chemical substance poorly soluble in water as a pharmaceutically active ingredient is 6 to 15.

7. The solid formulation according to any one of claims 1 to 6, wherein the inorganic porous substance is calcium silicate, light anhydrous silicic acid, magnesium silicate, magnesium aluminosilicate, magnesium aluminometasilicate, magnesium aluminum silicate, synthetic aluminum silicate, hydrated silicon dioxide or a mixture thereof,

8. The solid formulation according to claim 7, wherein the inorganic porous substance is light anhydrous silicic acid.

9. The solid formulation according to any one of claims 1 to 8, wherein the weight ratio of the inorganic porous substance to the chemical substance poorly soluble in water as a pharmaceutically active ingredient is 4 to 100.

10. The solid formulation according to anyone of claims 1 to 9, wherein the chemical substance poorly soluble in water as a pharmaceutically active ingredient is a lipid absorption inhibitor.

11. The solid formulation according to claim 10, wherein the lipid absorption inhibitor is an apolipoprotein B (Apo B) secretion inhibitor, a triglyceride (TG) transfer protein inhibitor or a cholesteryl ester transfer protein (CETP) inhibitor.

12. The solid formulation according to claim 10, wherein the lipid absorption inhibitor is an MTP inhibitor, a lipase inhibitor or an ACAT inhibitor.

13. The solid formulation according to claim 12, wherein the lipid absorption inhibitor is an MTP inhibitor.

14. The solid formulation according to claim 13, wherein the MTP inhibitor is a substituted piperazine derivative or a salt thereof.

15. The solid formulation according to claim 13, wherein the MTP inhibitor is a benzamide derivative or a salt thereof.

16. The solid formulation according to claim 15, wherein the MTP inhibitor is a benzamide derivative selected from the following or a salt thereof:
· 4'-trifluoromethyl-biphenyl-2-carboxylicacid-(2-butyl-1,2,3,4-tetrahydroisoquinolin-6-yl)-amide,
· 4'-trifluoromethyl-biphenyl-2-carboxylicacid-[2-(2-acetylaminoethyl)-1,2,3,4-tetrahydroisoquinolin-6-yl)-amide,
· 9-[4-[4-(2,3-dihydro-1-oxo-1H-isoindol-2-yl)-1-piperidinyl]butyl]-N-propyl-9H-fluorene-9-carboxamide,
· 2-[1-(3,3-diphenylpropyl)-4-piperidinyl]-2,3-dihydro-1H-isoindol-1-one,
· 9-[4-(4-[2-(4-trifluoromethylphenyl)benzoylamino]piperidin-1-yl)butyl]-N-2,2,2-trifluoroethyl-9H-fluorene-9-carboxamide,
· 4'-trifluoromethyl-biphenyl-2-carboxylicacid-[2-(1H-[1,2,4]triazol-3-ylmethyl)-1,2,3,4-tetrahydroisoquinolin-6-yl]-amide,
· 4'-trifluoromethyl-biphenyl-2-carboxylicacid-[2-(2-acetylaminoethyl)-1,2,3,4-tetrahydroisoquinolin-6-yl]-amide,
· 9H-(4-[4-[4'-trifluoromethyl-biphenyl-2-carbonyl]amino]-piperidin-1-yl}butyl)-9H-fluorene-9-carboxylicacid-(2,2,2-trifluoroethyl)-amide,
· 9-[4-[4-(2-benzothiazol-2-yl-benzoylamino)piperidin-1-yl]butyl] -9H-fluorene-9-carboxylicacid-(2,2,2-trifluoroethyl)amide,
· [11a-R]-8-[(4-cyanophenyl)methoxy]-2-cyclopentyl-7-(prop-2-enyl)-2,3,11,11a-tetrahydro-6H-pyrazino[1,2b]isoquinoline-1,4-dione,
· [11a-R]-2-cyclopentyl-7-(prop-2-enyl)-8-[(pyridin-2-yl)me thoxy]-2,3,11,11a-tetrahydro-6H-pyrazino[1,2b]isoquinoline-1,4-dione,
· 2-cyclopentyl-2-[4-(2,4-dimethylpyrido[2,3-b]indol-9-ylmethyl)phenyl]-N-(2-hydroxy-1-phenylethyl)acetamide and
· 2-cyclopentyl-N-(2-hydroxy-1-phenylethyl)-2-[4-(quinolin-2-ylmethoxy)-phenyl]acetamide.

17. The solid formulation according to claim 15, wherein the benzamide derivative is a compound represented by the following formula, wherein X refers to -CON(R¹⁰)-(CH₂)ₙ-, the compound which is an ester compound represented by the general formula (1) : 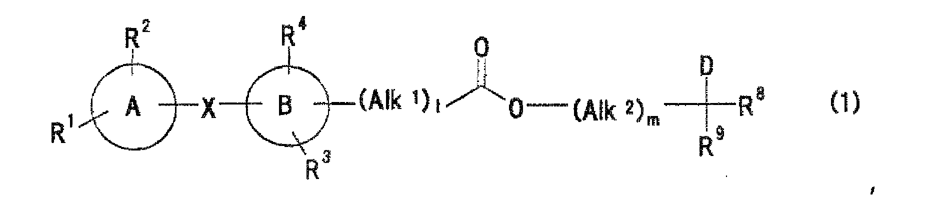 wherein R¹ and R² are a hydrogen atom, a C₁₋₆ alkyl group, a C₃₋₇ cycloalkyl group, a C₁₋₆ alkoxy group, a halo C₁₋₆ alkyl group, a halo C₁₋₆ alkyloxy group, a C₆₋₁₄ aryl group which may be substituted, a C₇₋₁₆ aralkyl group which may be substituted, a C₆₋₁₄ aryloxy group which may be substituted, a C₇₋₁₆aralkyloxy group which may be substituted, a C₇₋₁₅ arylcarbonyl group which may be substituted, a heterocyclic group which may be substituted, a C₂₋₇ alkoxycarbonyl group, a halogen atom, a C₂₋₆ alkenyl group or -N(R⁴⁰) (R⁴¹) wherein R⁴⁰ and R⁴¹ may be the same or different, and are a hydrogen, atom or a C₆₋₁₄ aryl group which may be substituted; ring A is a C₆₋₁₄ aryl group, a heterocyclic group or 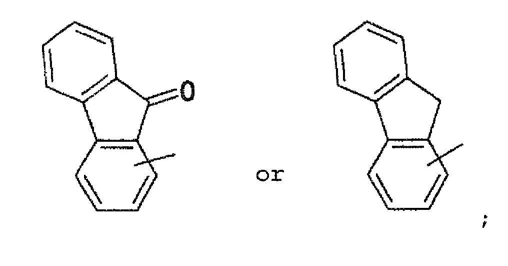
X is -CON(R¹⁰)-(CH₂)ₙ-, wherein R¹⁰ is a hydrogen atom, a C₁₋₆ alkyl group or a C₃₋₇ cycloalkyl group, and n is 0 or an integer of 1 to 3; R³ and R⁴ may be the same or different, and are a hydrogen atom, a hydroxyl group, a halogen atom, a C₁₋₆ alkyl group which may be substituted, a C₁₋₆ alkoxy group, a halo C₁₋₆ alkyl group, a C₇₋₁₆ aralkyloxy group, a C₁₋₆ acyl group, a heterocyclic group which may be substituted, -CON(R¹¹) (R¹²), wherein R¹¹ and R¹² may be the same or different, and are a hydrogen atom, a C₁₋₆ alkyl group, a C₆₋₁₄ aryl group which may be substituted, a C₇₋₁₆ aralkyl group which may be substituted or a C₁₋₆ alkoxy group, or may form, together with the nitrogen atom to which they are bonded, 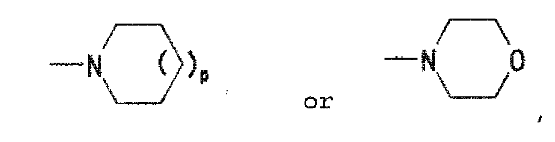 wherein p is 0 or an integer of 1 to 2, -(CH₂)_{q}-N(R¹³)(R¹⁴), wherein R¹³ and R¹⁴ may be the same or different, and are a hydrogen atom, a C₁₋₆ alkyl group, a C₂₋₇ alkoxycarbonyl group
or a C₁₋₆ acyl group, or may form, together with the nitrogen atom to which they are bonded, 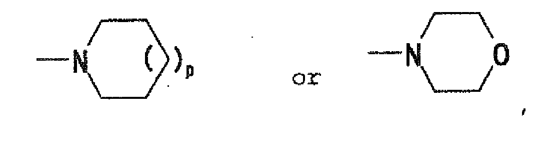 wherein p is the same meaning as above, and q is 0 or an integer of 1 to 3, or -CO(R¹⁵), wherein R¹⁵ is a hydroxyl group, a C₁₋₆ alkoxy group, a C₆₋₁₄ aryloxy group which may be substituted, a C₇₋₁₆ aralkyloxy group which may be substituted or a C₁₋₆ alkyl group; ring B is 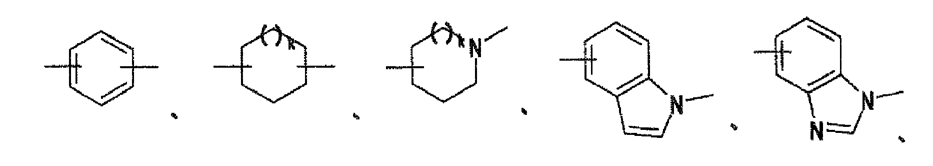 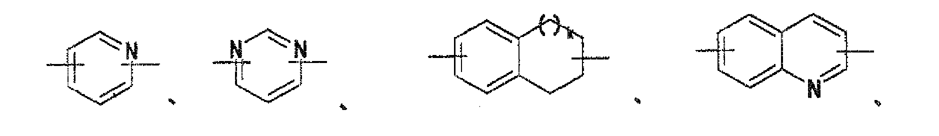 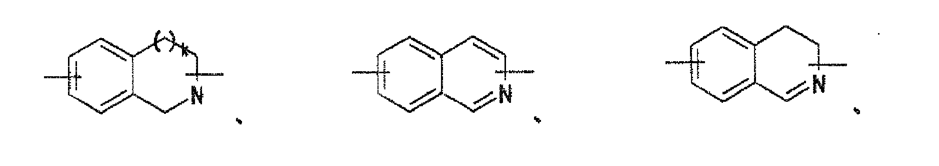 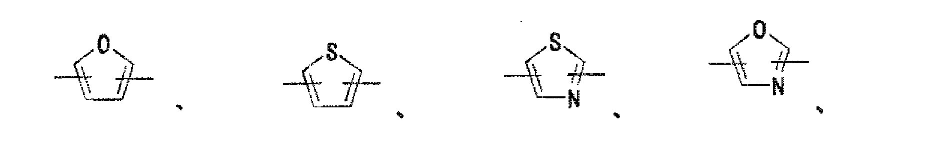 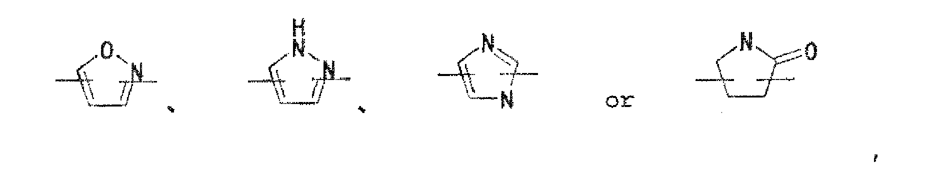 wherein K is 0 or an integer of 1 to 2, or ring B may form, together with R³, R¹⁰ and the nitrogen atom to which R¹⁰ is bonded, 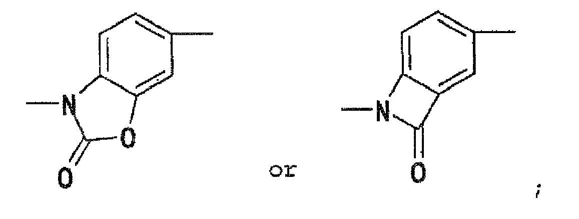
Alkl¹ is alkandiyl or alkendiyl; Alkl² is alkandiyl or alkendiyl; 1 is 0 or an integer of 1 to 3; m is 0 or an integer of 1 to 3; D is a C₁₋₆ alkyl group, a C₂₋₆ alkenyl group, a C₂₋₇ alkoxycarbonyl group, -N(R⁴²) -CO(R⁴³), wherein R⁴² is a hydrogen atom or a C₁₋₆ alkyl group, and R⁴³ is a C₆₋₁₄ aryl group or a C₇₋₁₆ aralkyl group, or the following formula; 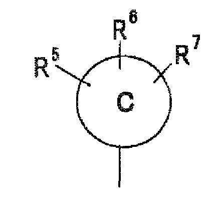 wherein R⁵, R⁶ and R⁷ may be the same or different, and are a hydrogen atom, a C₁₋₆ alkyl group, a C₁₋₆ alkoxy group, a. C₂₋₇ alkoxycarbonyl group, a carboxyl group, a halogen atom, a cyano group, a nitro group, a halo C₁₋₆ alkyl group, a C₁₋₆ acyl group, a hydroxyl group, an amino group, a C₆₋₁₄ aryl group which may be substituted or - (CH₂)ᵣ-CON (R¹⁶) (R¹⁷), wherein R¹⁶ and R¹⁷ may be the same or different, and are a hydrogen atom, a C₁₋₆ alkyl group, and or a halo C₁₋₆ alkyl group, and r is 0 or an integer of 1 to 3; ring C is a C₆₋₁₄ aryl group, a C₇₋₁₅ arylcarbonylamino group, a C₈₋₁₇aralkylcarbonylamino group, a hetero ring residue, a C₃₋₇ cycloalkyl group or a C₇₋₁₆ aralkyl group, or ring C may form, together with R⁷ and R⁸, 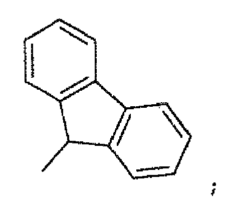 and
R⁸ and R⁹ may be the same or different, and are a hydrogen atom, a C₁₋₆ alkyl group, a C₆₋₁₄ aryl group which may be substituted, a hydroxy C₁₋₆ alkyl group, -CON(R¹⁸) (R¹⁹), wherein R¹⁸ and R¹⁹ may be the same or different, and are a hydrogen atom, a C₁₋₆ alkyl group, a C₃₋₇ cycloalkyl group, a halo C₁₋₆ alkyl group, a C₂₋₁₂ alkoxyalkyl group or a C₆₋₁₄ aryl group which may be substituted, -COO(R²⁰) or- (CH₂)ₛ-OCO(R²⁰), wherein R²⁰ is a hydrogen atom, a C₁₋₆ alkyl group or a C₃₋₇ cycloalkyl group, and s is 0 or an integer of 1 to 3, or -N(R²¹) (R²²), wherein R²¹ and R²² may be the same or different, and are a hydrogen atom, a C₁₋₆ alkyl group, a C₁₋₆ acyl group or a C₁₋₆ alkylsulfonyl group, or R²¹ and R²² may form, together with the nitrogen atom to which they are bonded, 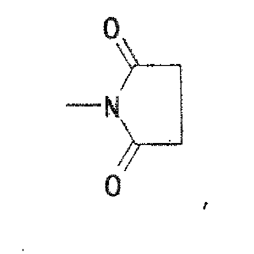
or R⁸ and R⁹ together may form a C₃₋₇ cycloalkyl group, or a prodrug thereof, or a pharmaceutically acceptable salt thereof.

18. The solid formulation according to claim 15, wherein the benzamide derivative is a compound selected from the following:
· 2-phenyl-2-(2-{4-[(4'-trifluoromethyl-biphenyl-2-carbonyl)-amino]-phenyl}-acetoxymethyl)-malonic acid diisobutylester,
· 2-(2-{4-[ethyl-(4'-trifluoromethyl-biphenyl-2-carbonyl)-amino]-phenyl}-acetoxymethyl)-2-phenyl-malonic acid diethylester,
· 2-(2-{3-dimethylcarbamoyl-4-[(4-trifluoromethyl-biphenyl-2-carbonyl)-amino]-phenyl}-acetoxymethyl)-2-phenyl-malonic acid diethyl ester,
· 2-(2-{3-dimethylcarbamoyl-4-[(4'-trifluoromethyl-biphenyl-2-carbonyl)-amino]-phenyl}-acetoxymethyl)-2-phenyl-malonic acid dimethyl ester,
· 2-cyclopentyl-2-(2-{3-dimethylcarbamoyl-4-[(4'-trifluoromethyl-biphenyl-2-carbonyl)-amino]-phenyl}-acetoxymethyl)-malonic acid diethyl ester,
· 2-cyclohexyl-2-(2-{3-dimethylcarbamoyl-4-[(4'-trifluorome thyl-biphenyl-2-carbonyl)-amino]-phenyl}-acetoxymethyl)-malonic acid diethyl ester,
· 2-(2-{3-dimethylcarbamoyl-4-[(4-methyl-4'-trifluoromethyl-biphenyl-2-carbonyl)-amino]-phenyl}-acetoxymethyl)-2-phenyl-malonic acid diethyl ester,
· 2-(2-{3-dimethylcarbamoyl-4-[(5-methyl-4'-trifluoromethyl-biphenyl-2-carbonyl)-amino]-phenyl}-acetoxymethyl)-2-phenyl-mal.onic acid diethyl ester,
· 2-[3-(2-{3-dimethylcarbamoyl-4-[(4'-trifluoromethyl-biphenyl-2-carbonyl)-amino]-phenyl}-acetoxy)-propyl]-2-phenyl-malonic acid diethyl ester,
· 2-(2-{3-dimethylcarbamoyl-4-[(5-methoxy-4'-trifluoromethyl-biphenyl-2-carbonyl)-amino]-phenyl}-acetoxymethyl)-2-phenyl-malonic acid diethyl ester,
· 2-(2-{4-[(5-chloro-4'-trifluoromethyl-biphenyl-2-carbonyl)-amino]-3-dimethylcarbamoyl-phenyl}-acetoxymethyl}-2-phenyl-malonic acid diethyl ester,
· 2-(2-{3-dimethylcarbamoyl-4-[(6-methyl-4'-trifluoromethyl-biphenyl-2-carbonyl)-amino]-phenyl}-acetoxymethyl)-2-phenyl-malonic acid diethyl ester,
· 2-(2-{3-dimethylcarbamoyl-4-[(4'-trifluoromethyl-biphenyl-2-carbonyl)-amino]-phenyl}-acetoxymethyl) -2-phenyl-malonic acid di-2,2,2-trifluoroethyl ester,
· 2-(2-{3-dimethylcarbamoyl-4-[(2'-fluoro-4'-trifluoromethyl-biphenyl-2-carbonyl)-amino]-phenyl}-acetoxymethyl)-2-phenyl-malonic acid diethyl ester,
· 2-(2-{5-dimethylcarbamoyl-2-fluoro-4-[(4'-trifluoromethyl-biphenyl-2-carbonyl)-amino]-phenyl}-acetoxymethyl)-2-phenyl-malonic acid diethyl ester,
· 2-(2-{3-bromo-5-dimethylcarbamoyl-4-[(4'-trifluoromethyl-biphenyl-2-carbonyl)-amino]-phenyl}-acetoxymethyl)-2-phenyl-malonic acid diethyl ester,
· 2-(2-{3-chloro-5-dimethylcarbamoyl-4-[(4'-trifluoromethyl-biphenyl-2-carbonyl)-amino]-phenyl}-acetoxymethyl)-2-phenyl-malonic acid diethyl ester,
· 2-(2-{3-dimethylcarbamoyl-4-[(3'-fluoro-4'-trifluoromethyl-biphenyl-2-carbonyl)-amino]-phenyl}-acetoxymethyl)-2-phenyl-malonic acid diethyl ester,
· 2-(2-{4-[(3'-chloro-4'-trifluoromethyl-biphenyl-2-carbony 1)-amino]-3-dimethylcarbamoyl-phenyl}-acetoxymethyl)-2-phenyl-malanic acid diethyl ester,
· 2-(2-{3-dimethylcarbamoyl-4-[(4'-trifluoromethyl-biphenyl-2-carbonyl) -amino] -phenyl}-acetoxymethyl)-2-(5-nitro-pyridin-2-yl)-malonic acid diethyl ester,
· 2-(5-amino-pyridin-2-yl)-2-(2-{3-dimethylcarbamoyl-4-[(4'-trifluoromethyl-biphenyl-2-carbonyl)-amino]-phenyl}-acetoxymethyl)-malonic acid diethyl ester,
· 2-(2-{3-dimethylcarbamoyl-4-[(4'-trifluoromethyl-biphenyl-2-carbonyl)-amino]-phenyl}-acetoxymethyl)-2-pyridin-2-yl-malonic acid diethyl ester,
· 2-(2-{3-chloro-5-dimethylcarbamoyl-2-fluoro-4-[(4'-trifluoromethyl-biphenyl-2-carbonyl)-amino]-phenyl}-acetoxymethyl)-2-phenyl-malonic acid diethyl ester,
· 2-(2-{3-bromo-5-dimethylcarbamoyl-2-fluoro-4-[(4'-trifluoromethyl-biphenyl-2-carbonyl)-amino]-phenyl}-acetoxymethyl)-2-phenyl-malonic acid diethyl ester,
· 2-(2-{3-dimethylcarbamoyl-4-[(4'-trifluoromethyl-biphenyl-2-carbonyl)-amino]-phenyl}-acetoxymethyl)-2-o-tolyl-malonic acid diethyl ester,
· 2-(2-{3-dimethylcarbamoyl-4- 1(4'-trifluoromethyl-biphenyl-2-carbonyl)-amino]-phenyl}-acetoxymethyl)-2-m-tolyl-malonic acid diethyl ester,
· 2-(2-{3-dimethylcarbamoyl-4-[(4'-trifluoromethyl-biphenyl-2-carbonyl)-amino]-phenyl}-acetoxymethyl)-2-p-tolyl-malonic acid diethyl ester,
· 2-(2-chloro-phenyl)-2-(2-{3-dimethylcarbamoyl-4-[(4'-trifluoromethyl-biphenyl-2-carbonyl)-amino]-phenyl}-acetoxymethyl)-malonic acid diethyl ester,
· 2-(3-chloro-phenyl)-2-(2-{3-dimethylcarbamoyl-4-[(4'-trifluoromethyl-biphenyl-2-carbonyl)-amino]-phenyl}-acetoxymethyl)-malonic acid diethyl ester,
· 2-(4-chloro-phenyl)-2-(2-{3-dimethylcarbamoyl-4-[(4'-trifluoromethyl-biphenyl-2-carbonyl)-amino]-phenyl}-acetoxymethyl)-malonic acid diethyl ester,
· 2-(2-{3-dimethylcarbamoyl-4-[(4'-trifluoromethyl-biphenyl-2-carbonyl)-amino]-phenyl}-acetoxymethyl)-2 -phenyl-succinic acid diethyl ester,
· 2-(2-{3-dimethylcarbamoyl-4-[(4'-trifluoromethyl-biphenyl -2-carbonyl)-amino]-phenyl}-acetoxymethyl)-2-(2-methoxy-phenyl)-malonic acid diethyl ester,
· 2-(2-{3-dimethylcarbamoyl-4- [(4'-trifluoromethyl-biphenyl-2-carbonyl)-amino]-phenyl}-acetoxymethyl)-2-(3-methoxy-phenyl)-malonic acid diethyl ester,
· 2-(2-{3-dimethylcarbamoyl-4-[(4'-trifluoromethyl-biphenyl-2-carbonyl)-amino]-phenyl}-acetoxymethyl)-2-(4-methoxy-phenyl)-malonic acid diethyl ester,
· 2-(2-{4-[(5,4'-bis-trifluoromethyl-biphenyl-2-carbonyl)-amino]-3-dimethylcarbamoyl-phenyl}-acetoxymethyl)-2-phenyl-malonic acid diethyl ester,
· 2-(2-{4-[(6-chloro-4'-trifluoromethyl-biphenyl-2-carbonyl)-amino]-3-dimethylcarbamoyl-phenyl}-acetoxymethyl)-2-phenyl-malonic acid diethyl ester,
· 2-(2-{3-dimethylcarbamoyl-4-[(6-fluoro-4'-trifluoromethyl-biphenyl-2-carbonyl)-amino]-phenyl}-acetoxymethyl)-2-phenyl-malonic acid diethyl ester,
· 2-[2-(2-{3-dimethylcarbamoyl-4-[(5-methyl-4'-trifluoromethyl-biphenyl-2-carbonyl)-amino]-phenyl}-acetoxy)-ethyl]-2-phenyl-malonic acid diethyl ester,
· 2-(2-{3-dimethylcarbamoyl-4-[(5-ethoxy-4'-trifluoromethyl-biphenyl-2-carbonyl)-amino]-phenyl}-acetoxymethyl)-2-phenyl-malonic acid diethyl ester,
· 2-(2-{3-dimethylcarbamoyl-4-[(5-isopropoxy-4'-trifluoromethyl-biphenyl-2-carbonyl)-amino]-phenyl}-acetoxymethyl)-2-phenyl-malonic acid diethyl ester,
· 2-[2-(2-{4-[(5,4'-bis-trifluoromethyl-biphenyl-2-carbonyl)-amino]-3-dimethylcarbamoyl-phenyl}-acetoxy)-ethyl]-2-phenyl-malonic acid diethyl ester,
· 2-(2-{3-dimethylcarbamoyl-4-[(6-methoxy-4'-trifluoromethyl-biphenyl-2-carbonyl)-amino]-phenyl}-acetoxymethyl)-2-phenyl-malonic acid diethyl ester and
· 2-(2-{3-dimethylcarbamoyl-4-[(3-methyl-4'-trifluoromethyl-biphenyl-2-carbonyl)-amino]-phenyl}-acetoxymethyl)-2-phenyl-malonic acid diethyl ester.

19. A production method of a solid formulation having improved water-solubility which comprises a chemical substance poorly soluble in water as a pharmaceutically active ingredient, a water-soluble polymer substance and an inorganic porous substance, **characterized in that**
(1) the chemical substance poorly soluble in water forms a solid dispersion together with the water-soluble polymer substance,
(2) the water-soluble polymer substance forming the solid dispersion is in the form of fine pieces, and
(3) the water-soluble polymer substance in the form of fine pieces is maintained to be adsorbed on and/or adhered to the surface of the inorganic porous substance, the method
**characterized by** comprising mixing and stirring a solution obtained by dissolving the substance poorly soluble in water as an active ingredient and the water-soluble polymer substance in an organic solvent with the inorganic porous substance and then granulating and drying the mixture.

20. The production method of a solid formulation according to claim 19, wherein the water-soluble polymer substance is hydroxypropylcellulose, hydroxypropylmethylcellulose, polyvinylpyrrolidone, polyvinyl alcohol, copolyvidone, agar, dextrin, gelatin or a mixture thereof.

21. The production method of a solid formulation according to claim 20, wherein the water-soluble polymer substance is polyvinylpyrrolidone or hydroxypropylcellulose.

22. The production method of a solid formulation according to any one of claims 19 to 21, wherein, the weight ratio of the water-soluble polymer substance to the chemical substance poorly soluble in water as a pharmaceutically active ingredient is 3 to 100.

23. The production method of a solid formulation according to claim 22, wherein the water-soluble polymer substance is polyvinylpyrrolidone, and the weight ratio of polyvinylpyrrolidone to the chemical substance poorly soluble in water as a pharmaceutically active ingredient is 3 to 50.

24. The production method of a solid formulation according to claim 22, wherein the water-soluble polymer substance is hydroxypropylcellulose, and the weight ratio of hydroxypropylcellulose to the chemical substance poorly soluble in water as a pharmaceutically active ingredient is 6 to 15.

25. The production method of a solid formulation according to any one of claims 19 to 24, wherein the inorganic porous substance is calcium silicate, light anhydrous silicic acid, magnesium silicate, magnesium aluminosilicate, magnesium aluminometasilicate, magnesium aluminum silicate, synthetic aluminum silicate, hydrated silicon dioxide or a mixture thereof.

26. The production method of a solid formulation according to claim 25, wherein the inorganic porous substance is light anhydrous silicic acid.

27. The production method of a solid formulation according to any one of claims 19 to 26, wherein the weight ratio of the inorganic porous substance to the chemical substance poorly soluble, in water as a pharmaceutically active ingredient is 4 to 100.

28. The production method of a solid formulation according to any one of claims 19 to 27, wherein the weight ratio of the organic solvent to the water-soluble polymer substance is 1 to 2.

29. The production method of a solid formulation according to any one of claims 19 to 28, wherein the chemical substance poorly soluble inwater as a pharmaceutically active ingredient is a lipid absorption inhibitor.

30. The production method of a solid formulation according to claim 29, wherein the lipid absorption inhibitor is an apolipoprotein B (Apo B) secretion inhibitor, a triglyceride (TG) transfer protein inhibitor or a cholesteryl ester transfer protein (CETP) inhibitor.

31. The production method of a solid formulation according to claim 29, wherein the lipid absorption inhibitor is an MTP inhibitor, a lipase inhibitor or an ACAT inhibitor.

32. The production method of a solid formulation according to claim 31, wherein the lipid absorption inhibitor is an MTP inhibitor.
